# EUROPEAN PATENT APPLICATION

(11) **EP 4 810 485 A1**
(43) Date of publication of application: **23.09.2026**
(21) Application number: 25315083.3
(22) Date of filing: 17.03.2025
(51) Int. Cl.: C07D 231/12, C07D 401/04, C07D 417/04, C07D 417/14, C07D 487/04, C07D 491/052, A61P 35/00, A61K 31/4155

(54) **NEW ACYLSULFONAMIDE DERIVATIVES AND THEIR USE FOR THE TREATMENT OF CANCER**

(71) Applicant: Qubit Pharmaceuticals, 75014 Paris (FR)
(72) Inventor: El Hage, Krystel, 75014 Paris (FR); Nicolaï, Eric, 75014 Paris (FR)
(74) Representative: A.P.I. Conseil

(57) **Abstract**

The present disclosure describes acylsulfonamide compounds of Formula (I), and pharmaceutically acceptable salts thereof, compositions, methods, and uses thereof. Such compounds are believed to be therapeutically useful as inhibitors of KAT6A with improved selectivity particularly in the treatment and/or prevention of diseases and disorders mediated by KAT6A in a subject.

## Description

### FIELD OF THE INVENTION

The present invention relates to the field of pharmaceuticals and concerns compounds capable of modulating KAT6 activity and/or KAT6-signaling, including their synthesis methods and therapeutic applications. In addition, the invention relates to incorporation of these compounds into a pharmaceutical formulation, and its application as a KAT6 inhibitor in the development of therapeutic agents for the treatment and/or prevention of cancer.

### BACKGROUND OF THE INVENTION

KAT6A, also designated as MYST3 or MOZ, is a member of the MYST family of histone acetyltransferases (HATs) that play a role in the regulation of gene expression and chromatin modification. KAT6A functions as an epigenetic regulator by acetylating histone H3 at lysine 9 (H3K9) and especially on lysine 24 (H3K24), post-translational modifications associated with transcriptionally active chromatin. Through these modifications, KAT6A modulates chromatin structure and accessibility, thereby influencing the transcription of genes involved in key cellular processes such as differentiation, proliferation, and survival (Lv, D. *et al.,* 2017).

Beyond its role in normal cellular function, KAT6A has been implicated in oncogenesis. Alterations in KAT6A activity, including mutations, chromosomal translocations, and aberrant expression, are associated with the initiation and progression of various cancers. Notably, chromosomal translocations involving KAT6A, such as t(8;16)(p11;p13), result in fusion proteins like KAT6A-CREBBP, which are linked to acute myeloid leukemia (AML) (Coenen, E.A. *et al.,* 2013). Additionally, overexpression of KAT6A has been observed in solid tumors, including breast and colon cancers, where it contributes to increased cellular proliferation and survival (Hu, Z. *et al.,* 2019).

The oncogenic potential of KAT6A is mediated through its capacity to modify the transcriptional landscape in a manner that supports tumor progression. KAT6A, as a histone acetyltransferase, acetylates histone H3 at lysine residues K9, K14, and K23, thereby facilitating the expression of genes involved in cellular proliferation and survival while repressing those associated with differentiation and apoptosis. Additionally, KAT6A seems to interact with other epigenetic regulators, including the NuA4 complex and the p300/CBP coactivator, to modulate the expression of oncogenes and tumor suppressor genes. These interactions collectively contribute to the establishment of a transcriptional environment that promotes oncogenic processes. Thus, targeting KAT6A presents a viable therapeutic strategy for cancers characterized by KAT6A dysregulation. Inhibition of KAT6A's acetyltransferase activity has the potential to disrupt the transcriptional programs that drive cancer progression (Weber, L.M. *et al.,* 2023).

Cancers driven by KAT6A dysregulation, including AML, breast cancer, and colon cancer, frequently present aggressive phenotypes and exhibit resistance to conventional treatments like chemotherapy and radiation. Compounds such as WM-1119 demonstrate moderate efficacy *in vitro* but lack sufficient potency *in vivo* to achieve robust therapeutic outcomes due to issues such as low bioavailability, or poor pharmacokinetic properties (Sharma S. *et al.,* 2023). A primary challenge in developing KAT6A inhibitors is achieving selectivity due to structural homology with other MYST family members, including KAT6B (MORF), KAT5 (Tip60), KAT7 (HBO1), and KAT8 (MYST1). This similarity, particularly within the acetyl-CoA binding pocket, complicates the design of inhibitors that specifically target KAT6A without affecting other HATs, which are essential for normal cellular functions. Indeed, non-selective inhibition may lead to off-target effects and toxicity, thereby narrowing the therapeutic window. The development of novel KAT6A inhibitors with enhanced selectivity and potency could provide new therapeutic options for these cancers, particularly those resistant to existing treatments. Additionally, such inhibitors could be integrated into combination therapies to improve treatment efficacy and mitigate drug resistance.

Recent advancements include the discovery of CTx-648 (PF-9363), a highly potent, and orally bioavailable inhibitor of KAT6A/B histone acetyltransferases. CTx-648 has demonstrated antitumor activity in breast cancer models with high KAT6A expression and estrogen receptor positivity (ER+). By specifically inhibiting KAT6A/B, CTx-648 reduces the acetylation of histone H3 at lysine 23 (H3K23Ac), thereby suppressing tumor growth. The efficacy of CTx-648 in preclinical trials highlights the potential of targeted KAT6A inhibition as a therapeutic approach for cancers with KAT6A dysregulation. However, in the phase 1 clinical trial of PF-07248144 for ER+HER2- metastatic breast cancer, neutropenia and anemia were among the most frequently reported treatment-related adverse events. These occurred in 59.8% and 48.6% of patients, respectively, with 35.5% and 13.1% experiencing grade 3 or 4 severity (Mukohara T. et al., 2024). This high incidence stresses the critical role of the KAT families in hematopoiesis and in particular KAT6 and KAT7. KAT6A/B enzymes are involved in the regulation of gene expression for the proliferation and differentiation of hematopoietic stem cells (HSC) and show a high degree of cooperation in the regulation of hematopoiesis. Like KAT6A/B and KAT7 also have a critical role in maintaining HSC function as it confers differentiation down the erythroid or alternative lineages. However, KAT6A/B, but not KAT7, regulates B cell development. The inhibition of KAT6B and KAT7 alongside KAT6A disrupts normal blood cell development, leading to adverse effects such as anemia and neutropenia (Bergamasco M.I. et al., 2024). The occurrence of neutropenia and anemia emphasizes the necessity for developing compounds with greater specificity to minimize adverse effects while maintaining therapeutic efficacy.

In summary, KAT6A is involved in normal gene regulation and cancer pathogenesis through its histone acetyltransferase activity. While existing inhibitors have laid important groundwork, there remains a pressing need for more selective and potent inhibitors that target KAT6A-driven cancers.

### SUMMARY OF THE INVENTION

The invention aims to address these drawbacks and in particular aims at developing compounds with a better therapeutic index. The following provides a simplified summary of selected aspects, embodiments, and examples of the present invention to offer a basic understanding of it. However, this summary is not intended to provide a comprehensive overview of all aspects, embodiments, and examples of the invention. Its sole purpose is to present selected aspects, embodiments, and examples in a concise manner, serving as an introduction to the more detailed description of the aspects, embodiments, and examples that follow this summary.

The present disclosure aims to provide a compound of general formula (I): 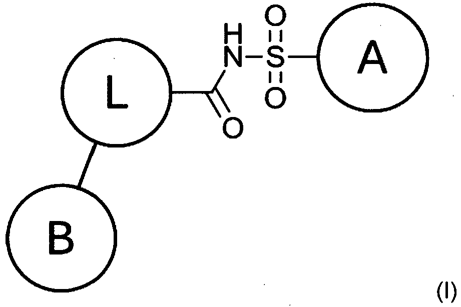 or a pharmaceutically acceptable salt thereof, wherein:
**A represents** an aryl group or a heteroaryl group, such as a benzo-fused alicyclic group, preferably a benzo-fused heterocyclic group;
wherein A is optionally substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms or a hydroxy group;
   - cycloalkyloxy groups, optionally substituted by one or more alkyl groups and/or halogens; and
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogens; and
**L represents** a fused multicyclic ring system comprising at least two rings;
   wherein:
   - the fused multicyclic ring system is, as a whole, aromatic or partially saturated;
   - the rings of the fused multicyclic ring system optionally includes one or more heteroatoms; preferably a partially saturated ring of the fused multicyclic ring system can include a heteroatom selected from nitrogen, oxygen, and sulfur; preferably a partially saturated ring or an aromatic ring of the fused multicyclic ring system can include a nitrogen;
   - a carbon atom of one ring within the system is connected to group A via a N-acylsulfonamide function and a different ring is connected to group B; and
**B represents** a five-or six-membered aromatic heterocyclic ring preferably selected from pyrazole, thiazole, and pyridine, said ring being optionally substituted with one or more substituents; preferably, said ring is substituted with one or more substituents selected from alkyl groups such as methyl, halogenoalkyl groups such as fluoromethyl or difluoromethyl, and/or cycloalkyl groups.

As it is shown in the examples, the compounds according to the invention are effective in modulating or inhibiting the KAT6 signaling pathway. Moreover, the compounds of the invention show selective inhibition of KAT6A in preference to KAT6B. Moreover, the compound of formula (I) shows a better selectivity against KAT6A compared to compounds from the prior art.

According to other optional features of the compound of the invention, it may optionally include one or more of the following features, either individually or in combination:
- the compound is of formula (II): 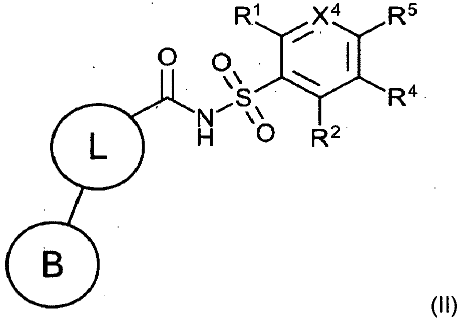 or a pharmaceutically acceptable salt thereof, wherein:
   X⁴ is selected from -C-R³ or nitrogen;
   R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)2); Alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens.
- the compound is of formula (III): 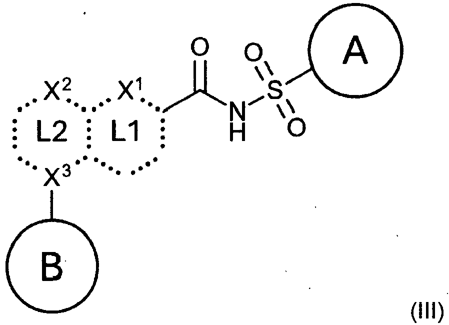 or a pharmaceutically acceptable salt thereof, wherein:
   L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
   X¹ represents a nitrogen atom or a carbon atom, each further bonded to a sufficient number of hydrogen atoms to satisfy its valence;
   X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C1-C4 alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens;-and
   X³ represents a nitrogen atom or a carbon atom further bonded to a sufficient number of hydrogen atoms to satisfy its valence.
- the compound is of formula (IVa): 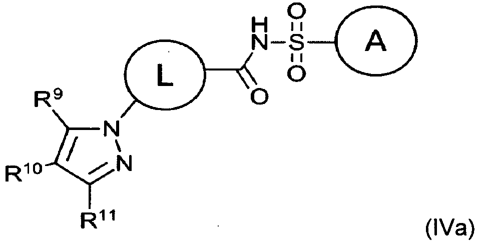 or a pharmaceutically acceptable salt thereof, wherein R⁹, R¹⁰ and R¹¹ each independently represent H; or an alkyl group, such as a linear or branched C₁-C₄ alkyl group, optionally substituted by 1 to 3 fluorine atoms; or a cycloalkyl group.
- the compound is of formula (IVb): 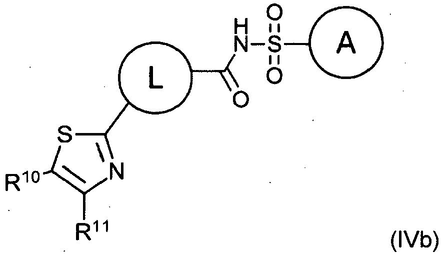 or a pharmaceutically acceptable salt thereof, wherein R¹⁰ and R¹¹ each independently represent H; or an alkyl group, such as a linear or branched C₁-C₄ alkyl group, optionally substituted by 1 to 3 fluorine atoms; or a cycloalkyl group.
- the compound is of formula (Va): 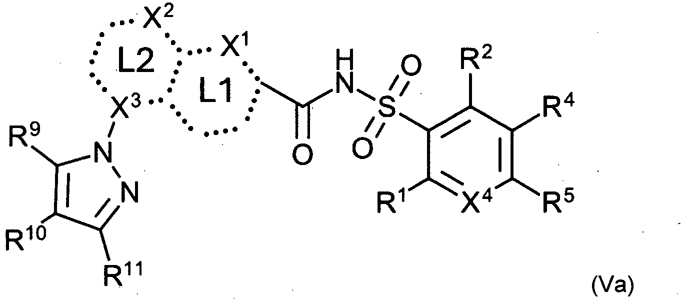 or a pharmaceutically acceptable salt thereof, wherein:
   X⁴ is selected from -C-R³ or nitrogen;
   R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)2); Alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens.
   L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
   X¹ represents a nitrogen atom or a carbon atom, each further bonded to a sufficient number of hydrogen atoms to satisfy its valence;
   X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and X³ represents a carbon atom further bonded to a sufficient number of hydrogen atoms to satisfy its valence;
   R⁹, R¹⁰ and R¹¹ each independently represent H; or an alkyl group, such as a linear or branched C₁-C₄ alkyl group, optionally substituted by 1 to 3 fluorine atoms; or a cycloalkyl group.
- the compound is of formula (Vb): 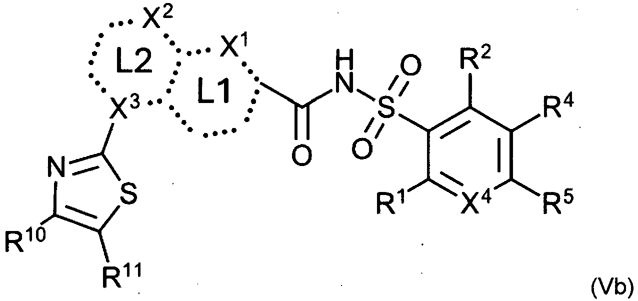 or a pharmaceutically acceptable salt thereof, wherein:
   X⁴ is selected from -C-R³ or nitrogen;
   R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)2); Alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens;
   L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
   X¹ represents a nitrogen atom or a carbon atom, each further bonded to a sufficient number of hydrogen atoms to satisfy its valence;
   X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and X³ represents a nitrogen atom or a carbon atom further bonded to a sufficient number of hydrogen atoms to satisfy its valence; and
   R¹⁰ and R¹¹ each independently represent H; or an alkyl group, such as a linear or branched C₁-C₄ alkyl group, optionally substituted by 1 to 3 fluorine atoms; or a cycloalkyl group.
- the **L group** comprises an aromatic bicyclic system or wherein L1 and L2 together consist in an aromatic bicyclic system, the rings of the aromatic bicyclic system optionally includes one or more nitrogen atoms.
- L1 is an aromatic ring or heteroaromatic ring; preferably, L1 and L2 are both independently an aromatic ring or a heteroaromatic ring.
- the compound has a structure selected from: 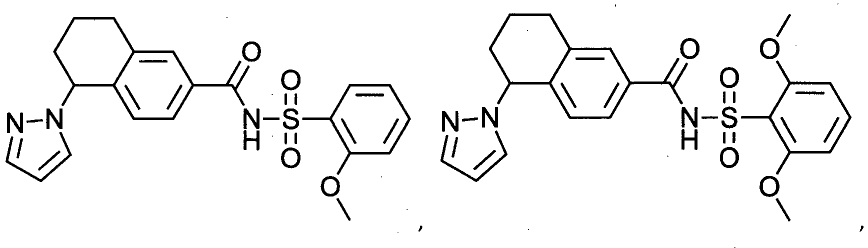 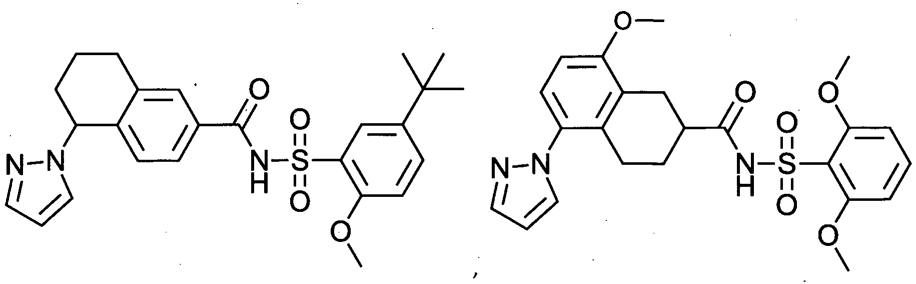 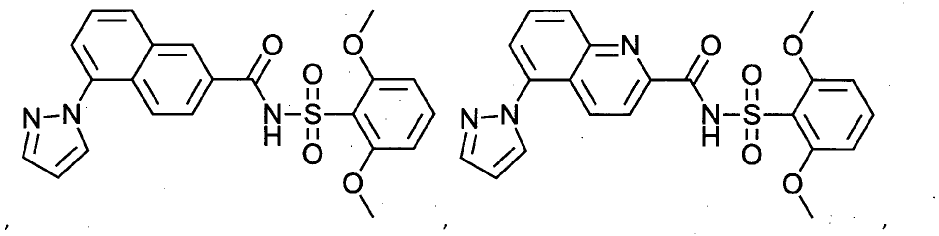 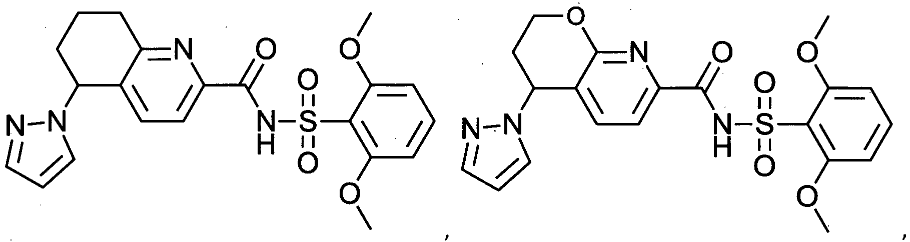 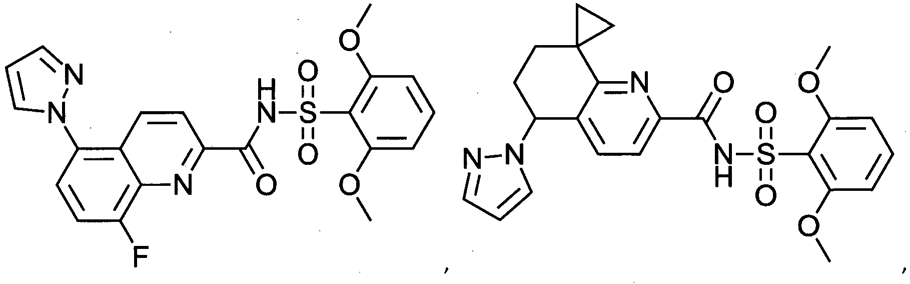 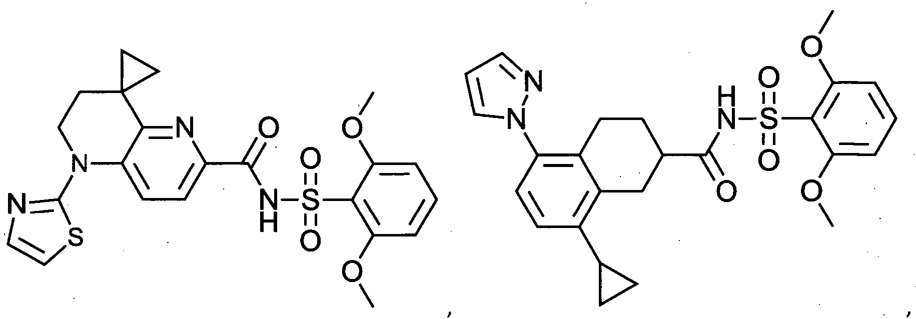 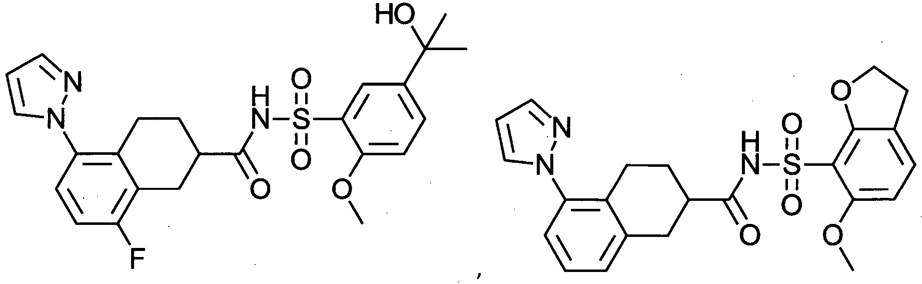 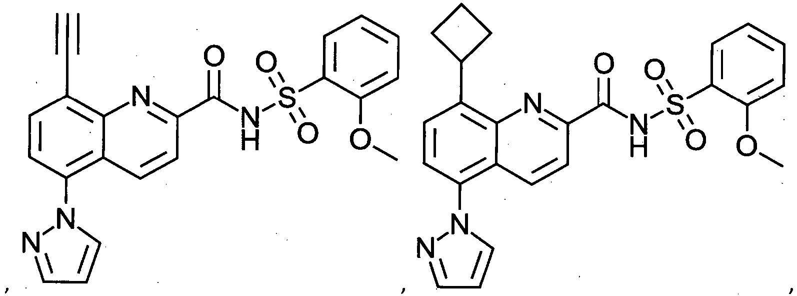 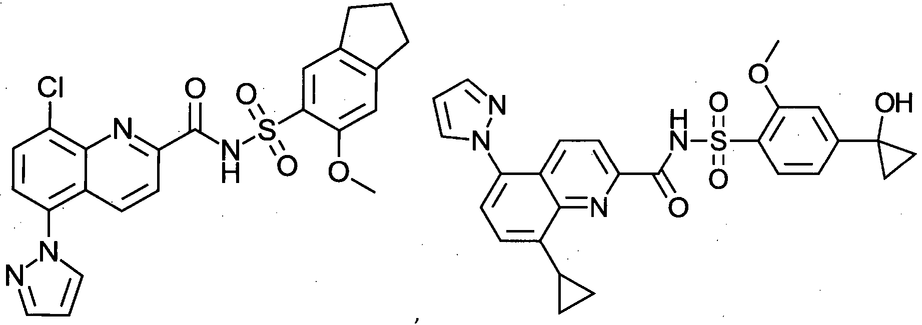 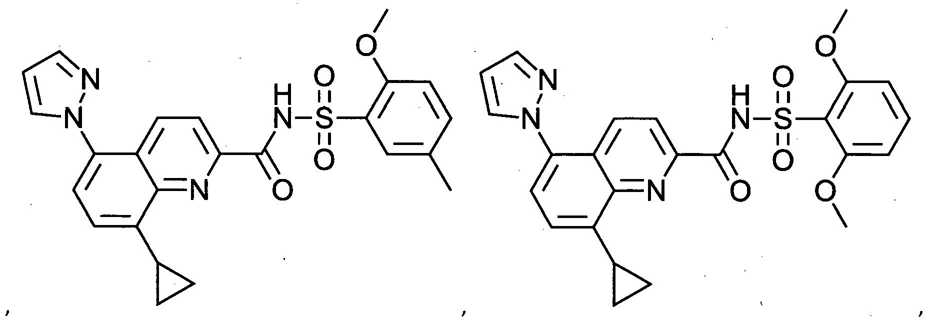 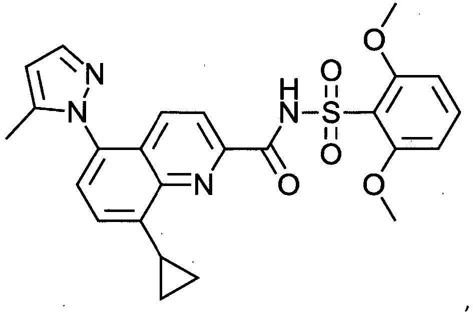 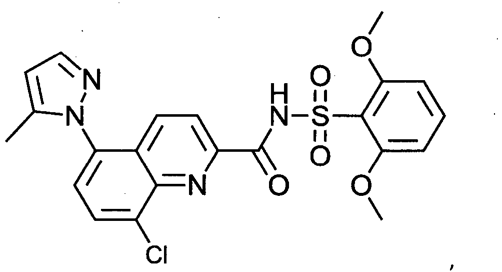 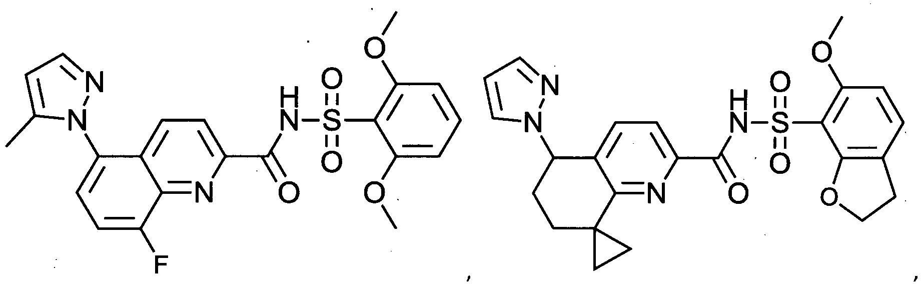 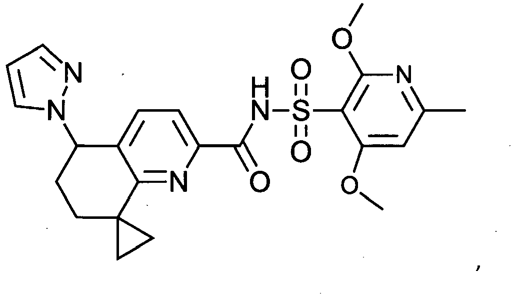 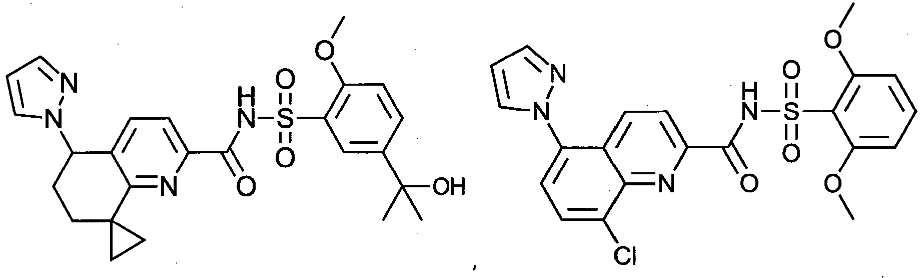 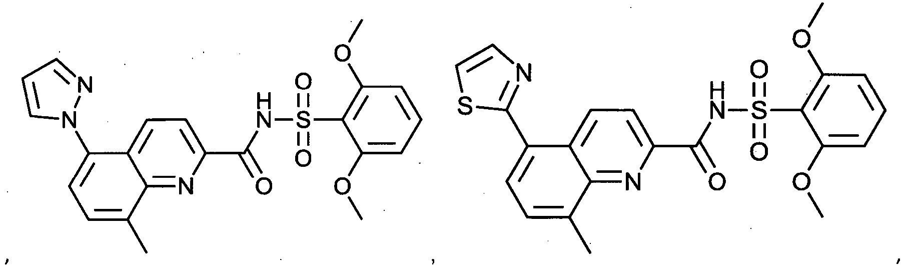 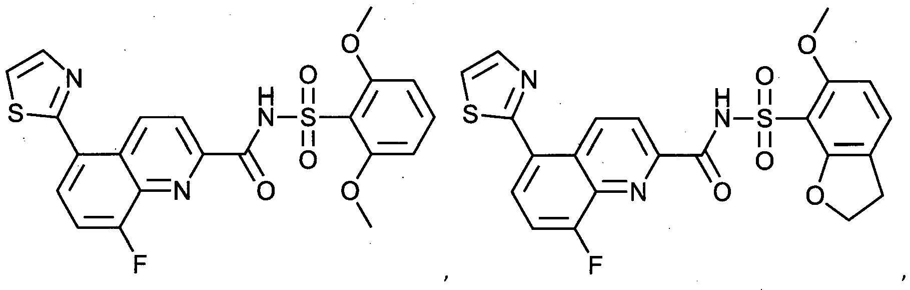 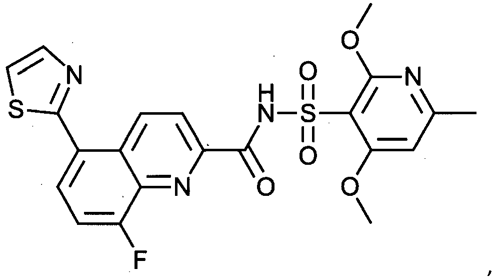 or

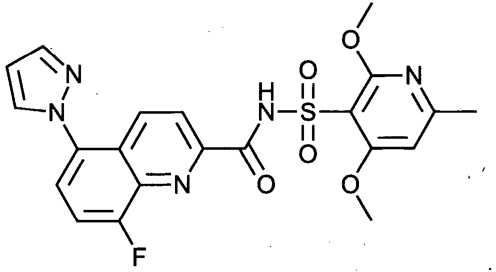

Preferably, the compound has a structure selected from: 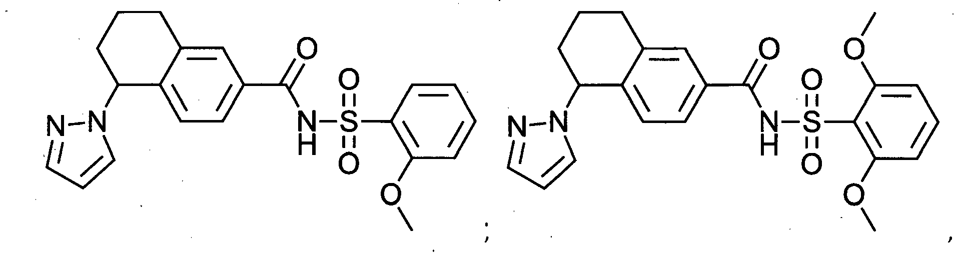 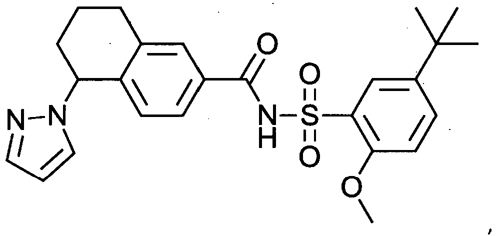 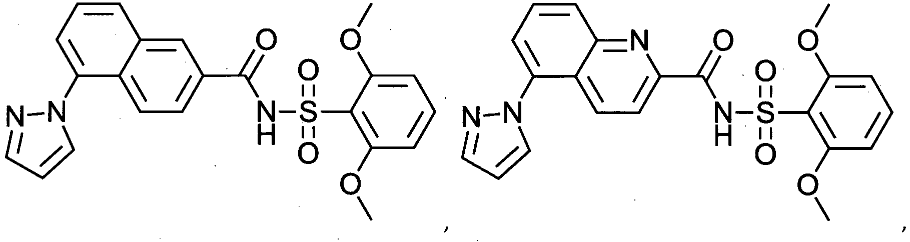 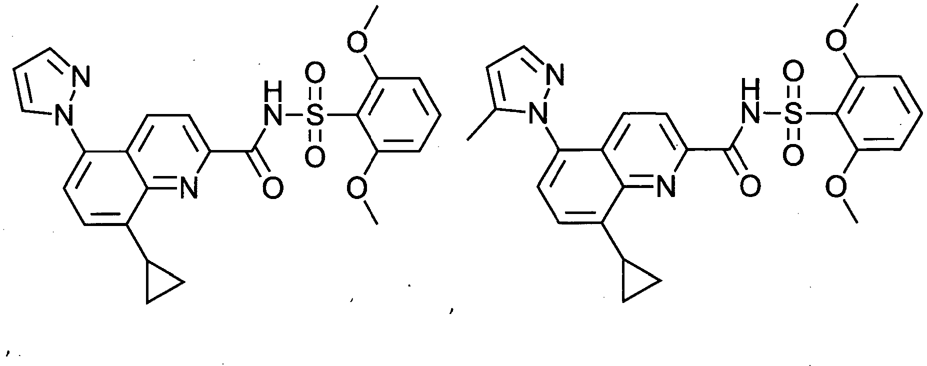 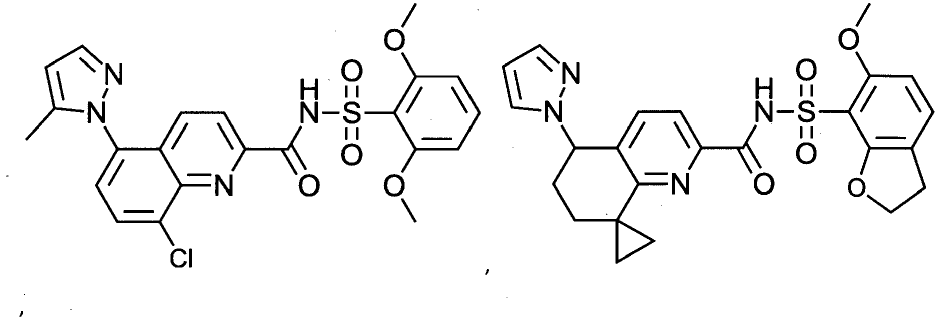 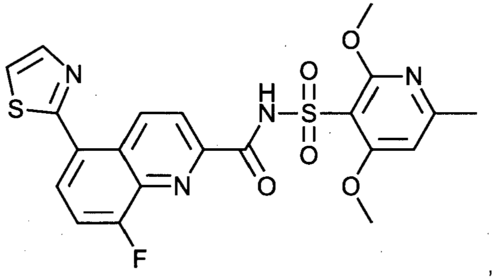 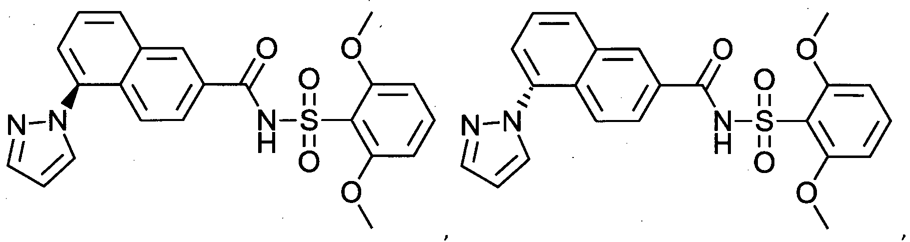 or

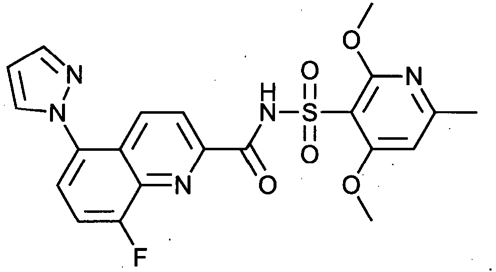

The invention also relates to all the tautomeric forms, stereoisomeric forms, pharmaceutically acceptable salts thereof, racemic mixtures, substantially pure stereoisomeric forms, and optically pure stereoisomeric forms of the compounds described in the present invention.

According to **another aspect,** the invention relates to a **pharmaceutical composition** comprising a compound according to the invention, or a pharmaceutically acceptable salt thereof, and one or more pharmaceutically acceptable excipients.

According to **another aspect,** the invention relates to a compound according to the invention or a pharmaceutically acceptable salt thereof, or a pharmaceutical composition according to the invention, for use in the treatment or prophylaxis of a disease, preferably wherein the disease is a hyperproliferative disorder.

The compounds of the invention are particularly effective for use in the treatment or prophylaxis of a disease at modulating or inhibiting the KAT6 activity or KAT6 signalling pathway, particularly KAT6A.

The invention can in particular relate to the compound, the pharmaceutically acceptable salt or the pharmaceutical composition for use according to the invention, wherein it is for use in combination with a selective estrogen receptor degrader (SERD) such as fulvestrant or oral alternatives like camizestrant; immune checkpoint inhibitors targeting PD-1, PD-L1, or CTLA-4 and/or a cyclin-dependent kinase (CDK) inhibitor, including CDK2, CDK4, CDK6 or dual CDK2/4 or CDK4/6 inhibitors, such as palbociclib, ribociclib, or abemaciclib, for enhanced therapeutic efficacy in estrogen receptor-positive cancer. The compounds of the invention are particularly effective for use in combination with a selective estrogen receptor degrader (SERD) inhibitor; immune checkpoint inhibitors targeting PD-1, PD-L1, or CTLA-4 inhibitor and/or a cyclin-dependent kinase (CDK) inhibitor.

According to **another aspect,** the invention relates to a method for treating or prophylaxis of a disease, preferably wherein the disease is a hyperproliferative disorder, comprising administering to a patient in need thereof a compound of general formula (I), or a pharmaceutically acceptable salt thereof, according to the invention, or a pharmaceutically acceptable composition according to the invention.

The method of the invention is particularly effective for use in the treatment or prophylaxis of a disease at modulating or inhibiting the KAT6 activity or KAT6 signalling pathway.

The invention can in particular relate to the method for treating or prophylaxis of a disease according to the invention, wherein it further comprises administering to the patient in need thereof a Selective Estrogen Receptor Degrader (SERD) such as fulvestrant or oral alternatives like camizestrant; immune checkpoint inhibitors targeting PD-1, PD-L1, or CTLA-4 and/or a cyclin-dependent kinase (CDK) inhibitor, including CDK2, CDK4, CDK6 or dual CDK2/4 or, CDK4/6 inhibitors, such as palbociclib, ribociclib, or abemaciclib.

The method of the invention is particularly effective for use in combination with a selective estrogen receptor degrader (SERD) inhibitor and/or a cyclin-dependent kinase (CDK) inhibitor.

### DETAILED DESCRIPTION AND ADDITIONAL EMBODIMENTS

Below, we provide a summary of the invention and its associated terminology, followed by a discussion of the drawbacks of the prior art. Finally, we will detail how the invention effectively addresses these issues.

Unless otherwise stated, a reference to a physical measurement value in the form of an interval shall be understood to include the limits.

As used herein, the singular form **"a", "an",** and **"the"** include plural references unless indicated otherwise. For example, **"a"** substituent includes one or more substituents.

The expression **"hyperproliferative disorders"** within the meaning of the invention can refer to any diseases characterized by excessive and uncontrolled cell proliferation in humans or animals. This includes a wide range of conditions where abnormal cell growth leads to the formation of tumors or other tissue anomalies. Examples encompass various forms of cancer such as carcinomas, sarcomas, leukemias, lymphomas, and melanomas.

The term **"cancer"** within the meaning of the invention, can refer to a diverse group of diseases or disorders characterized by the uncontrolled growth and spread of abnormal cells. Cancer can manifest in various tissues and organs, presenting as solid tumors or affecting blood-forming tissues, such as in leukemia. This term encompasses a wide range of malignancies, including but not limited to carcinomas, sarcomas, leukemias, lymphomas, and melanomas. Specifically, it includes primary cancers as well as metastatic diseases, wherein cancer cells spread from the original (primary) site to other parts of the body. Examples of cancers covered by this term include lung, breast, colorectal, prostate, pancreatic, liver, stomach, esophageal, ovarian, cervical, bladder, and skin carcinomas; osteosarcoma, chondrosarcoma, liposarcoma, and rhabdomyosarcoma sarcomas; acute and chronic lymphoblastic and myeloid leukemias; Hodgkin and non-Hodgkin lymphomas; central nervous system cancers such as glioblastoma and medulloblastoma; melanomas; germ cell tumors including testicular and ovarian cancers; neuroendocrine tumors; multiple myeloma; thyroid cancers; mesotheliomas; head and neck cancers; gastrointestinal stromal tumors (GIST); Kaposi's sarcoma; and vulvar, vaginal, and penile cancers. Additionally, the term includes rare and mixed types of cancers, as well as metastatic cancers. Furthermore, it encompasses KATs overexpressing cancers, particularly those exhibiting elevated levels of KAT6, which contribute to the malignancy's progression and resistance to conventional therapies.

The term **"tumor"** within the meaning of the invention, can refer to an abnormal mass of tissue that arises from excessive and uncontrolled cell proliferation. Tumors can be classified as either benign or malignant. A benign tumor is non-cancerous and does not invade surrounding tissues or metastasize to other parts of the body. In contrast, a malignant tumor, also known as a cancerous tumor, possesses the potential to invade nearby tissues and spread (metastasize) to distant sites within the body. Hence, the term "tumor" preferably refers to solid masses that may develop in various organs or tissues, as well as neoplastic growths affecting any part of the body, including soft tissues, bones, and blood-forming organs.

The expression **"KATs overexpressing cancer"** within the meaning of the invention, can refer to cancers in which one or more lysine acetyltransferases (KATs) are produced at abnormally high levels compared to normal tissue counterparts. Specifically, this includes cancers that exhibit overexpression of KAT6, a member of the KAT family involved in chromatin remodeling and the regulation of gene expression. Overexpression of KAT6 and other KATs can drive oncogenic processes such as enhanced cell proliferation, resistance to apoptosis, metastasis, and the maintenance of stem cell-like properties in cancer cells. Examples of KATs overexpressing cancers include certain subtypes of leukemia, such as acute myeloid leukemia (AML) with KAT6A or KAT6B rearrangements, as well as solid tumors like specific forms of breast cancer, colorectal cancer, and pancreatic cancer that display elevated KAT6 expression. The expression **"KAT6A overexpressing cancer"** within the meaning of the invention, can refer to cancers in which KAT6A is produced at higher levels compared to normal tissue counterparts. In breast cancer, *KAT6A* was found to be amplified in 12-15% of tumors as part of the 8p11-12 amplicon, and is associated with poor outcomes for primary breast cancer patients, mostly of luminal subtype (Turner-Ivey B. *et al.* 2017). Additionally, neuroendocrine tumors and other malignancies where KAT6 overexpression contributes to tumor progression and therapy resistance are encompassed within this definition.

The terms **"patient"** or **"subject"** within the meaning of the invention, can refer to any individual, whether human or non-human, who is undergoing treatment, diagnosis, or observation related to a medical condition or therapeutic intervention. This includes individuals currently diagnosed with a disease, disorder, or condition, as well as those who are at risk of developing such conditions. Additionally, it encompasses individuals participating in clinical trials or research studies, those receiving preventive care, and those undergoing monitoring for potential health issues. Both symptomatic and asymptomatic individuals fall within this definition.

The expressions **"patient in need thereof"** or **"subject in need thereof"** within the meaning of the invention, can refer to any individual, whether human or non-human, who requires therapeutic intervention, diagnosis, or monitoring for a specific medical condition or disease addressed by the invention. This includes individuals who are diagnosed with the targeted disease or condition and require treatment, as well as those who are at elevated risk of developing the disease and thus require preventive measures. Additionally, it encompasses individuals undergoing therapy or intervention aimed at managing, mitigating, or curing the targeted disease or condition, as well as those participating in diagnostic or monitoring procedures related to the disease. Furthermore, it includes individuals who may benefit from improved therapeutic outcomes, enhanced disease management, or reduced side effects through the use of the invention.

The terms **"treatment"** or **"treating"** within the meaning of the invention, can refer to any medical intervention aimed at managing, alleviating, reducing, or eliminating symptoms of a disease, disorder, or condition, as well as preventing its progression or recurrence. This may include the administration of pharmaceutical compounds, therapeutic agents, medical procedures, or other interventions that directly or indirectly modify the course of the condition. The terms may encompass both curative approaches, aimed at eradicating the condition, and palliative approaches, intended to relieve symptoms without necessarily curing the underlying disease. Treatment can also involve preventive measures to inhibit the onset of a disease in individuals at risk.

The terms **"prophylaxis"** within the meaning of the invention, can refer to the use of a compound that, in a statistical sample, reduces the occurrence of the disorder or condition in the treated sample relative to an untreated control sample, or delays the onset or reduces the severity of one or more symptoms of the disorder or condition relative to the untreated control sample, when administered prior to the onset of the disorder or condition.

The expression **"pharmaceutical composition"** within the meaning of the invention, can refer to a formulation that contains one or more active therapeutic agents selected from the compounds described herein, their pharmaceutically acceptable salts, prodrugs, or derivatives thereof, in combination with pharmaceutically acceptable excipients, carriers, adjuvants, or other chemical components. These compositions are prepared in a form suitable for administration to a patient and are designed to deliver the active ingredient effectively while ensuring stability, bioavailability, and eventually controlled release. The pharmaceutical composition encompasses a variety of dosage forms, including but not limited to solid forms such as tablets, capsules, granules, and powders; liquid forms such as solutions, suspensions, emulsions, and syrups; parenteral forms including injectable solutions or suspensions; topical forms like creams, ointments, gels, and lotions; and inhalable forms such as aerosols and dry powders. Additionally, these compositions are formulated for multiple routes of administration, including oral, intravenous (IV), intramuscular (IM), subcutaneous (SC), transdermal, and inhalation.

The expression **"pharmaceutically acceptable"** within the meaning of the invention, can refer to substances, compounds, or compositions that are suitable for use in humans or animals without producing significant adverse effects, toxicity, or undesirable reactions. These acceptable components are compatible with the active pharmaceutical ingredients and other formulation components, ensuring safety and efficacy in therapeutic applications. Characteristics of pharmaceutically acceptable substances include being non-toxic and non-irritating at the concentrations used, chemically and physically compatible with active ingredients and other excipients, and not adversely affecting the stability of the pharmaceutical composition.

The expression **"pharmaceutically acceptable salt"** within the meaning of the invention, can refer to a salt form of an active pharmaceutical compound that is suitable for therapeutic use in humans or animals. These salts are derived from the reaction of the active compound with pharmaceutically acceptable inorganic or organic acids or bases, enhancing properties such as solubility, stability, or bioavailability without introducing significant toxicity. Examples of pharmaceutically acceptable salts include those formed by reacting the active compound with inorganic acids like hydrochloric acid or sulfuric acid, organic acids such as citric acid or acetic acid, inorganic bases like sodium hydroxide or potassium hydroxide, and organic bases including ammonia or triethylamine. These salts may be prepared through direct neutralization during the final stages of purification or via solvent-based methods that facilitate salt formation during crystallization or precipitation. The pharmaceutically acceptable salts described herein are further detailed in Stahl, P. H., & Wermuth, C. G. (Eds.). (2011). Handbook of Pharmaceutical Salts: Properties, Selection, and Use. Wiley-VCH which is incorporated by reference.

The term **"excipients",** within the meaning of the invention, can refer to pharmaceutically acceptable inert substances that are incorporated into a pharmaceutical composition alongside the active compound described herein. Excipients serve various essential functions in the formulation, including but not limited to facilitating the manufacturing process, enhancing the stability and shelf-life of the active ingredient, aiding in the delivery and absorption of the active compound, improving the taste or appearance of the pharmaceutical formulation, and ensuring uniformity and proper dosage.

The expression **"pharmaceutically acceptable excipient, carrier, adjuvant,** or **vehicle"** within the meaning of the invention, can refer to any substance that facilitates the delivery, administration, stability, absorption, or effectiveness of an active pharmaceutical ingredient (API) without causing significant adverse effects or negatively interacting with the API. Carriers provide a medium for the API, ensuring uniform distribution within the dosage form, while excipients serve various functions such as fillers, binders, disintegrants, lubricants, and preservatives. Adjuvants may enhance the therapeutic effect of the API, and vehicles act as solvents or dispersing agents to deliver the API in liquid or semi-solid formulations. Characteristics of pharmaceutically acceptable carriers, adjuvants, or vehicles include biocompatibility, ensuring they are non-toxic and non-irritating to the patient; compatibility, meaning they do not react adversely with the API or other formulation components; and stability, maintaining the integrity and efficacy of the pharmaceutical composition over its intended shelf life.

The term **"halogen,"** as used here, refers to either the elemental forms fluorine, chlorine, bromine, or iodine, as well as their corresponding radicals when acting as substituents in chemical compounds. Specifically, it encompasses fluoro (F), chloro (CI), bromo (Br), or iodo (I) groups that can be incorporated into the compounds of the invention.

The expression **"alkyl group",** within the meaning of the invention, can refer to saturated monovalent hydrocarbon radicals. It is derived from an alkane by removing one hydrogen atom, resulting in a structure with the general formula CₙH₂ₙ₊₁. Alkyl groups can vary in size and structure, ranging from simple methyl (CH₃-) and ethyl (C₂H₅-) groups to larger, more complex chains such as propyl (C₃H₇-), butyl (C₄H₉-), pentyl (C₅H₁₁-), and hexyl (C₆H₁₃-) groups. Alkyl groups may also include branched or ramified structures like isopropyl (CH(CH₃)₂-) and isobutyl (CH₂CH(CH₃)₂-). The terms "C₁₋₄ alkyl group" or "C₁-C₄ alkyl group" refer to both linear and branched alkyl groups containing between one and four carbon atoms. Similarly, "C₁₋₆ alkyl group" or "C₁-C₆ alkyl group" encompasses alkyl groups with one to six carbon atoms. Examples of alkyl groups include, but are not limited to, methyl, ethyl, propyl, butyl, pentyl, hexyl, isopropyl, and isobutyl groups.

The expression **"alkoxy group",** as used herein, refers to an alkyl radical bonded to an oxygen atom, represented as alkyl-O-. The alkoxy radical is attached to a molecule through the oxygen atom. Alkoxy groups can vary in size and structure, encompassing both straight and branched alkyl moieties. The terms "C₁-C₄ alkoxy" and "C₁-C₃ alkoxy" denote alkoxy radicals containing from one to four carbon atoms and from one to three carbon atoms, respectively. Examples of alkoxy groups include, but are not limited to, methoxy (CH₃O-), ethoxy (C₂H₅O-), propoxy (C₃H₇O-), isopropoxy ((CH₃)₂CHO-), and butoxy (C₄H₉O-) groups.

The expression **"amino group"** within the meaning of the invention, can refer to a functional group consisting of a nitrogen atom bonded to one or more substituents. Specifically, it can denote primary amino group (-NH₂), secondary amino group (-NHR) where a nitrogen atom is bonded to one hydrogen atom and one alkyl or aryl group, or a tertiary amino group (-NR₂) where a nitrogen atom is bonded to two alkyl or aryl groups. In particular, an amino group according to the invention can comprise a dialkylamino group, such as a dimethylamino group (-N(CH₃)₂).

The expression **"phenyl group"** within the meaning can refer to a functional group derived from benzene, an aromatic hydrocarbon, by the removal of one hydrogen atom, resulting in the formula (-CH₅). It comprises a six-carbon aromatic ring, specifically a benzene ring, which can be attached to other atoms or functional groups in a molecule through the position where the hydrogen atom was removed. The phenyl group can be substituted at different positions on the ring, leading to derivatives such as ortho-, meta-, and para-substituted phenyl compounds. Examples of compounds containing a phenyl group include, but are not limited to, 1,3-dimethoxybenzene, where the phenyl group is attached to two methoxy group; 1-methoxy-4-(trifluoromethyl)benzene, where it is attached to a methoxy and a trifluoromethyl.

The expression **"cyclic group"** within the meaning of the invention can refer to a functional group comprising one or more ring structures formed by atoms connected in a closed loop. These rings can be saturated or unsaturated, aromatic or non-aromatic, and can consist of carbon atoms alone (carbocyclic) or a combination of carbon and heteroatoms such as nitrogen, oxygen, or sulfur (heterocyclic).

The expression **"ring system"** within the meaning of the invention, can refer to a cyclic structure composed of one or more connected rings. Ring systems can be monocyclic (a single ring) or polycyclic (multiple rings fused together or linked). Ring systems can be carbocyclic, consisting entirely of carbon atoms, or heterocyclic, containing one or more heteroatoms such as nitrogen, oxygen, or sulfur. Ring systems include, but are not limited to, saturated monocyclic rings, saturated polycyclic rings, aromatic monocyclic rings or aromatic polycyclic rings.

The term **"carbocyclic ring system"** refers to a ring composed exclusively of carbon atoms. Carbocyclic ring systems can be monocyclic or polycyclic, and can comprise saturated (alicyclic), as in cycloalkyl groups, and/or aromatic, as in aryl groups.

The expression **"cycloalkyl group"** within the meaning of the invention, can refer to saturated cyclic hydrocarbon radical derived from cycloalkanes by the removal of one hydrogen atom, resulting in a structure with the general formula CₙH₂ₙ₋₁, where n typically ranges from three to six. Examples of cycloalkyl group include, but are not limited to, C3-C6 cycloalkyl.

The expression **"cycloalkyloxy group"** within the meaning of the invention, can refer to an alkoxy group in which the alkyl moiety is a cycloalkyl group. It is represented as cycloalkyl-O-, where the cycloalkyl portion is a saturated cyclic hydrocarbon group. Examples of cycloalkyloxy groups include, but are not limited to, cyclopropyloxy, cyclobutyloxy, cyclopentyloxy, or cyclohexyloxy.

The expression **"aryl group"** within the meaning of the invention, can refer to a functional group derived from an aromatic hydrocarbon. It comprises an aromatic ring, typically a benzene ring (C₆H₅-), which can be attached to other atoms or functional groups in a molecule. The term "C₆-C₁₀ aryl" encompasses aryl groups containing from six to ten carbon atoms. Examples of aryl groups include, but are not limited to, phenyl (C₆H₅-), naphthyl (C₁₀H₇-), anthracyl (C₁₄H₁₀-), and their respective isomers such as 1-naphthyl and 2-anthracyl. Additionally, "aryl" includes fused polycyclic aromatic ring systems, such as bicyclic group, where an aromatic ring is fused to one or more additional rings, aromatic or not, such as heterocyclic rings containing heteroatoms, whether saturated or partially saturated. Examples include, but are not limited to, chromanyl, indanyl (2,3-dihydro-1H-indene) and tetrahydronaphthyl (1,2,3,4-tetrahydronaphthyl), where the point of attachment is on the aromatic ring.

The expression **"heterocyclic ring system"** refers to a ring system containing at least one heteroatom (nitrogen, oxygen, or sulfur) within the ring. Heterocyclic ring systems can be monocyclic or polycyclic, and can be saturated, partially saturated, or aromatic. The ring size can vary, typically containing 3 to 7 ring atoms per ring.

The term **"heteroatom"** within the meaning of the invention, can refer to an atom within a molecule that is not carbon or hydrogen. Specifically, it includes atoms such as nitrogen (N), oxygen (O), sulfur (S), phosphorus (P), silicon (Si), and other elements that can substitute for carbon in organic compounds.

The expression **"heterocyclyl group"** within the meaning of the invention, can refer to a substituent derived from a heterocycle by the removal of one hydrogen atom, resulting in a group that can be attached to other atoms or functional groups within a molecule. A heterocycle refers to a cyclic structure, saturated, partially saturated, or aromatic ring, in which one or more of the atoms in the ring are elements other than carbon, such as nitrogen, oxygen, or sulfur. Heterocycles can be aromatic or non-aromatic and may contain various numbers of heteroatoms within the ring. Examples of heterocyclyl group include, but are not limited to, 4- to 6-member heterocyclyl group, such as azetidine (derived from azetidine, a four-membered ring containing one nitrogen atom), imidazolyl (derived from imidazole, a five-membered ring containing two nitrogen atoms), pyrrolidinyl (derived from pyrrolidine, a five-membered ring containing one nitrogen atom), or thiazolyl (derived from thiazole, a five-membered ring containing both nitrogen and sulfur atoms). These heterocyclyl groups may be optionally substituted by an alkyl group, oxo group or a halogen, allowing for further diversification of the compound's structure, optionally substituted by an alkyl group or a halogen.

The expression **"heteroaryl group"** within the meaning of the invention, can refer to an aryl group in which one or more carbon atoms in the aromatic ring are replaced by heteroatoms such as nitrogen, oxygen, or sulfur. These heteroatoms can be incorporated into the aromatic ring in various positions, leading to structures such as pyridyl (where a nitrogen replaces a carbon in a benzene ring), thiophenyl (an aryl group with sulfur substitution in the aromatic ring), furanyl (an aryl group with oxygen substitution in the aromatic ring) groups, or indolyl (an aryl group derived from indole, containing both nitrogen and fused aromatic rings). Heteroaryl groups maintain the aromatic character of the ring. Additionally, "heteroaryl" includes fused polycyclic aromatic ring systems where an aromatic ring comprising one or more heteroatoms is fused to one or more additional rings, aromatic or not.

The expression **"heterocycloalkyl group"** within the meaning of the invention, can refer to a cycloalkyl group in which one or more carbon atoms are replaced by heteroatoms such as nitrogen, oxygen, or sulfur, thereby forming a heterocyclic ring. Examples of heterocycloalkyl groups include, but are not limited to, a 4- to 6-member heterocycloalkyl group such as azetidinyl (a four-membered ring containing one nitrogen atom, derived from azetidine), morpholinyl (a six-membered ring containing both nitrogen and oxygen atoms, derived from morpholine), piperidinyl (a six-membered ring containing one nitrogen atom, derived from piperidine) or thiazolidinyl (a five-membered ring containing both nitrogen and sulfur atoms, derived from thiazolidine). Heterocycloalkyl groups may be optionally substituted by an alkyl group, oxo group or a halogen, allowing for further structural diversification and modulation of the compound's pharmacological properties.

The expression **"fused multicyclic ring system"** within the meaning of the invention, can refer to multiple rings including at least two rings fused together. The fused multicyclic ring system beside the at least two rings fused together can further comprise linked ring(s). Fused multicyclic ring system can be carbocyclic, consisting entirely of carbon atoms, or heterocyclic, containing one or more heteroatoms such as nitrogen, oxygen, or sulfur.

The expression **"bicyclic ring"** can refer to a ring system composed of two fused rings, which can be both carbocyclic, both heterocyclic, or a combination of carbocyclic and heterocyclic rings. The rings can be saturated, partially saturated, or aromatic. Bicyclic ring can be homocyclic (both rings of the same type) or heterocyclic (rings of different types). Bicyclic ring can be further substituted by cyclic group.

The expression **"polycyclic ring"** can refer to a ring system composed of at least two fused rings, which can be both carbocyclic, both heterocyclic, or a combination of carbocyclic and heterocyclic rings. The rings can be saturated, partially saturated, or aromatic. Polycyclic ring can be homocyclic (both rings of the same type) or heterocyclic (rings of different types). Polycyclic ring can be further substituted by cyclic group.

The expression **"benzo-fused alicyclic group"** refers to a polycyclic ring system formed by the fusion of a benzene ring with a heterocycloalkyl ring or a cycloalkyl ring. The heterocycloalkyl or cycloalkyl portion are a saturated or partially saturated ring. Such expression encompasses the benzo-fused heterocycloalkyl group and benzo-fused cycloalkyl group. Examples include dihydrobenzofuran (a partially saturated furan ring fused to a benzene ring).

The expression **"benzo-fused heterocyclic group"** can refer to a polycyclic ring comprising a benzene ring which is fused to a heterocyclic ring containing at least one heteroatom. The heterocyclic ring can be five- to seven-membered; it is partially saturated. Examples include indoline (benzene fused to a pyrrolidine ring), dihydrobenzofuran (benzene fused to a tetrahydrofuran ring), and dihydrobenzothiophene (benzene fused to a tetrahydrothiophene ring). A "benzo-fused heterocyclic group" can comprise more than two ring systems with for example a ring system fused to the heterocyclic ring such as fused cycloalkyl or spirocyclic systems.

The expression **"benzo-fused oxygen containing heterocyclic group"** can refer to a polycyclic ring comprising a benzene ring which is fused to a heterocyclic ring containing at least one oxygen atom. These polycyclic ring systems can be fused, bridged or spirocyclic systems; They preferably consist in a 9 to 15 atom polycyclic ring system containing at least one oxygen atom and in which one ring is a phenyl. The heterocyclic ring can be five- to seven-membered, fully or partially saturated. Examples include dihydro benzodioxepine, tetrahydro-2-benzoxepine and dihydrobenzofuran. A "benzo-fused oxygen containing heterocyclic group" can comprise more than two ring systems with for example a ring system fused to the heterocyclic ring such-as 3-Oxabicyclo[4.1.0]heptane or 2-Oxabicyclo [3.1.0]hexane or a spirocyclic systems such as 5-Oxaspiro[2.4]heptane or 6-Oxaspiro[2.5]octane.

The expression **"benzo-fused heterocycloalkyl group"** refers to a polycyclic ring system formed by the fusion of a benzene ring with a heterocycloalkyl ring. The heterocycloalkyl portion is a saturated or partially saturated ring that contains one or more heteroatoms such as nitrogen, oxygen, or sulfur. This fused ring system can be saturated or partially saturated. Examples of benzo-fused heterocycloalkyl groups include dihydrobenzofuran (benzene fused to a partially saturated oxygen-containing ring).

The expression **"benzo-fused cycloalkyl group"** refers to a polycyclic ring system formed by the fusion of a benzene ring with a cycloalkyl ring. The cycloalkyl portion is a saturated or partially saturated cyclic hydrocarbon ring. This fused ring system can be saturated or partially saturated and includes both carbocyclic ring. Examples of benzo-fused cycloalkyl groups include indane (benzene fused to cyclopentane) and tetralin (benzene fused to cyclohexane).

The expression **"partially saturated"** within the meaning of the invention, can refer to a molecule, a molecular fragment, or a group that contains both single bonds and multiple bonds (such as double or triple bonds) within its structure. This means that while part of the molecule retains full saturation (i.e., single bonds between atoms), certain regions of the molecule exhibit unsaturation, often involving carbon-carbon or carbon-heteroatom double or triple bonds

The term **"unsaturated"** within the meaning of the invention, can refer to a group or functional group that contains one or more double or triple bonds between carbon atoms or between carbon and heteroatoms (such as nitrogen, oxygen, or sulfur) within its structure.

The term **"substituted"** within the meaning of the invention, can refer to a molecule, a molecular fragment or a group in which one or more hydrogen atoms have been replaced by other atoms or functional groups. The expression **"optionally substituted"** within the meaning of the invention, can refer to a molecule, a molecular fragment or a group that may or may not contain one or more substituent groups attached to its core structure.

The term **"solvate"** is used herein to describe a molecular complex comprising a compound described herein and one or more pharmaceutically acceptable solvent molecules, for example, ethanol.

### Compounds of the Invention

In some embodiments, a compound of the present disclosure is represented by general formula (I): 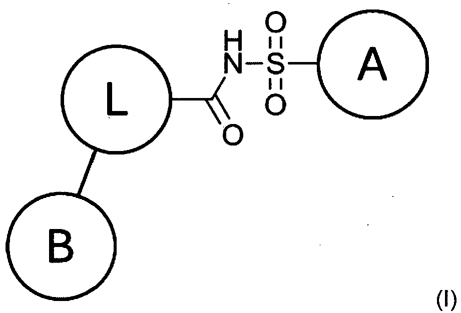 or a pharmaceutically acceptable salt thereof, wherein:
**A represents** an aryl group or a heteroaryl group, such as a **benzo-fused alicyclic group,** preferably a **benzo-fused heterocyclic group;**
wherein A is optionally substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms or a hydroxy group;
   - cycloalkyloxy groups, optionally substituted by one or more alkyl groups and/or halogens; and
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogens; and
**L represents** a fused multicyclic ring system comprising at least two rings; wherein:
   - the fused multicyclic ring system is, as a whole, aromatic or partially saturated;
   - the rings of the fused multicyclic ring system optionally includes one or more heteroatoms; preferably a partially saturated ring of the fused multicyclic ring system can include a heteroatom selected from nitrogen, oxygen, and sulfur; preferably a partially saturated ring or an aromatic ring of the fused multicyclic ring system can include a nitrogen;
   - a carbon atom of one ring within the system is connected to group A via a N-acylsulfonamide function and a different ring is connected to group B; and
**B represents** a five-or six-membered aromatic heterocyclic ring preferably selected from pyrazole, thiazole, and pyridine; said ring being optionally substituted with one or more substituents; preferably, said ring is substituted with one or more substituents selected from alkyl groups such as methyl, halogenoalkyl groups such as fluoromethyl or difluoromethyl, and/or cycloalkyl groups.

In preferred embodiments, the invention relates to a compound of general formula (I) or formula (III) or formula (IVa) or formula (IVb), or a pharmaceutically acceptable salt thereof, wherein:
A represents an aryl group or a heteroaryl group;
wherein A is substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂); and
   - cycloalkyl groups optionally substituted by one or more alkyl groups and/or halogens, such as a C₃-C₅ cycloalkyl group optionally substituted by 1 to 2 fluorine atoms or a hydroxy group;
   - cycloalkyloxy groups, optionally substituted by one or more alkyl groups and/or halogens;
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogens.

In more preferred embodiments, the invention relates to a compound of general formula (I) or formula (III) or formula (IVa) or formula (IVb), or a pharmaceutically acceptable salt thereof, wherein:
**A represents** an aryl group or a heteroaryl group;
wherein A is substituted by at least one alkoxy group, such as a C₁-C₄ alkoxy group;
wherein A is optionally further substituted by one or more membersselected from the group consisting of:
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂); and
   - cycloalkyl groups optionally substituted by one or more alkyl groups and/or halogens, such as a C₃-C₅ cycloalkyl group optionally substituted by 1 to 2 fluorine atoms or a hydroxy group.

In even more preferred embodiments, the invention relates to a compound of general formula (I) or formula (III) or formula (IVa) or formula (IVb), or a pharmaceutically acceptable salt thereof, wherein:
A represents a phenyl group or a benzo-fused cycloalkyl group or a benzo-fused oxygen containing heterocycloalkyl group such as a benzofuran or a pyridine;
wherein A is optionally substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂); and
   - cycloalkyl groups optionally substituted by one or more alkyl groups and/or halogens, such as a C₃-C₅ cycloalkyl group optionally substituted by 1 to 2 fluorine atoms or a hydroxy group.

In even more preferred embodiments, the invention relates to a compound of general formula (I) or formula (III) or formula (IVa) or formula (IVb), or a pharmaceutically acceptable salt thereof, wherein A represents a phenyl group or a benzo-fused cycloalkyl group or a benzo-fused oxygen containing heterocycloalkyl group such as a benzofuran or a pyridine; wherein A is optionally substituted by two alkoxy groups, such as a C₁-C₄ alkoxy group.

In some embodiments, the invention relates to a compound of general formula (I) or formula (II) or formula (IVa) or formula (IVb), or a pharmaceutically acceptable salt thereof, wherein:
**L represents** a fused multicyclic ring system comprising at least two rings; wherein:
- the fused multicyclic ring system is, as a whole, aromatic or partially saturated;
- a carbon atom of one aromatic ring within the fused multicyclic ring system is connected to group A via a N-acylsulfonamide function and a different ring is connected to group B, preferably both rings being fused.

In some embodiments, the invention relates to a compound of general formula (I) or formula (II) or formula (IVa) or formula (IVb), or a pharmaceutically acceptable salt thereof, wherein:
**L represents** a fused multicyclic ring system comprising at least two rings; wherein:
- the fused multicyclic ring system is, as a whole, aromatic or partially saturated;
- a carbon atom of one saturated ring within the fused multicyclic ring system is connected to group A via a N-acylsulfonamide function and a different ring is connected to group B, preferably both rings being fused.

In some embodiments, the invention relates to a compound of general formula (I) or formula (II) or formula (IVa) or formula (IVb), or a pharmaceutically acceptable salt thereof, wherein:
**L represents** a fused multicyclic ring system comprising at least two rings; wherein:
- the fused multicyclic ring system is, as a whole, aromatic or partially saturated;
- a carbon atom of one aromatic ring within the fused multicyclic ring system is connected to group A via a N-acylsulfonamide function and a 6 membered ring, whether aromatic or partially saturated ring is connected to group B, preferably both rings, the aromatic ring connected to group A and the 6 membered ring connected to group B, being fused.

In some embodiments, the invention relates to a compound of general formula (I) or formula (II) or formula (IVa) or formula (IVb), or a pharmaceutically acceptable salt thereof, wherein:
**L represents** a fused multicyclic ring system comprising at least two rings; wherein:
- the fused multicyclic ring system is, as a whole, aromatic or partially saturated;
- a carbon atom of one aromatic ring within the fused multicyclic ring system is connected to group A via a N-acylsulfonamide function and a 6 membered aromatic ring, is connected to group B, both aromatic rings being fused.

In some embodiments, the invention relates to a compound of general formula (I) or formula (II) or formula (IVa) or formula (IVb), or a pharmaceutically acceptable salt thereof, wherein:
**L represents** a fused multicyclic ring system comprising at least two rings; wherein:
- the fused multicyclic ring system is, as a whole, aromatic or partially saturated;
- a carbon atom of a pyridine within the fused multicyclic ring system is connected to group A via a N-acylsulfonamide function and a 6 membered ring, fused to the pyridine, whether aromatic or partially saturated ring, is connected to group B.

In some embodiments, the invention relates to a compound of general formula (I), or formula (II) or formula (III) or formula (VI), or a pharmaceutically acceptable salt thereof, wherein: **B represents** a pyrazole, a thiazole or a pyridine, optionally substituted with one or more substituents.

In some embodiments, the invention relates to a compound of general formula (I), or formula (II) or formula (III), or formula (VI), or a pharmaceutically acceptable salt thereof, wherein: **B represents** a pyrazole or a thiazole, optionally substituted with one or more substituents.

In some embodiments, the invention relates to a compound of general formula (I), or formula (II) or formula (III), or formula (VI), or a pharmaceutically acceptable salt thereof, wherein: **B represents** a pyrazole or a thiazole, substituted with one or more substituents selected from methyl, fluoromethyl or difluoromethyl.

In some embodiments, the invention relates to a compound of general formula (I) or a pharmaceutically acceptable salt thereof, wherein:
A represents an aryl group or a heteroaryl group;
wherein A is substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms or a hydroxy group;
   - cycloalkyloxy groups, optionally substituted by one or more alkyl groups and/or halogens; and
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogens;
L represents a fused multicyclic ring system comprising at least two rings; wherein:
   - the fused multicyclic ring system is, as a whole, aromatic or partially saturated;
   - a carbon atom of one aromatic ring within the fused multicyclic ring system is connected to group A via a N-acylsulfonamide function and a different ring is connected to group B, preferably both rings being fused;
B represents a pyrazole, a thiazole or a pyridine, optionally substituted with one or more substituents.

In preferred embodiments, the invention relates to a compound of general formula (I) or a pharmaceutically acceptable salt thereof, wherein:
A represents an aryl group or a heteroaryl group;
wherein A is substituted by at least one alkoxy group, such as a C₁-C₄ alkoxy group;
wherein A is optionally further substituted by one or more members selected from the group consisting of:
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms or a hydroxy group; and
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogen;
L represents a fused multicyclic ring system comprising at least two rings; wherein:
   - the fused multicyclic ring system is, as a whole, aromatic or partially saturated;
   - a carbon atom of one aromatic ring within the fused multicyclic ring system is connected to group A via a N-acylsulfonamide function and a 6 membered ring, whether aromatic or partially saturated ring is connected to group B, preferably both rings, the aromatic ring connected to group A and the 6 membered ring connected to group B, being fused; B represents a pyrazole, a thiazole, or a pyridine, optionally substituted with one or more substituents selected from methyl, fluoromethyl or difluoromethyl.

In more preferred embodiments, the invention relates to a compound of general formula (I) or a pharmaceutically acceptable salt thereof, wherein:
A represents a phenyl group or a benzo-fused cycloalkyl group or a benzo-fused oxygen containing heterocycloalkyl group such as a benzofuran or a pyridine;
wherein A is optionally substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms or a hydroxy group; and
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogen;
L represents a fused multicyclic ring system comprising at least two rings; wherein:
   - the fused multicyclic ring system is, as a whole, aromatic or partially saturated;
   - a carbon atom of one aromatic ring within the fused multicyclic ring system is connected to group A via a N-acylsulfonamide function and a 6 membered aromatic ring, is connected to group B, both aromatic rings being fused;
B represents a pyrazole, a thiazole, or a pyridine, optionally substituted with one or more substituents selected from methyl, fluoromethyl or difluoromethyl.

In more preferred embodiments, the invention relates to a compound of general formula (I) or a pharmaceutically acceptable salt thereof, wherein:
A represents a phenyl group or a benzo-fused cycloalkyl group or a benzo-fused oxygen containing heterocycloalkyl group such as a benzofuran or a pyridine;
wherein A is optionally substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂)
   - cycloalkyl groups optionally substituted by one or more alkyl groups and/or halogens, such as a C₃-C₅ cycloalkyl group optionally substituted by 1 to 2 fluorine atoms or a hydroxy group; and
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogen;
L represents a fused multicyclic ring system comprising at least two rings; wherein:
   - the fused multicyclic ring system is, as a whole, aromatic or partially saturated;
   - a carbon atom of one aromatic ring within the fused multicyclic ring system is connected to group A via a N-acylsulfonamide function and a 6 membered aromatic ring, is connected to group B, both aromatic rings being fused;
B represents a pyrazole, a thiazole or a pyridine, substituted with one or more substituents selected from methyl, fluoromethyl or difluoromethyl.

In even more preferred embodiments, the invention relates to a compound of general formula (I) or a pharmaceutically acceptable salt thereof, wherein:
A represents a phenyl group or a benzo-fused cycloalkyl group or a benzo-fused oxygen containing heterocycloalkyl group such as a benzofuran or a pyridine;
wherein A is optionally substituted by one or more members selected from the group consisting of:
   - alkoxy groups, such as a C₁-C₄ alkoxy group;
   - alkyl groups, such as a C₁-C₄ alkyl group, linear or branched, optionally substituted by 1 to 3 fluorine atoms or a hydroxy group;
   - halogens;
   - unsubstituted or substituted amino groups, such as dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms or a hydroxy group; and
   - heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogen;
L represents a fused multicyclic ring system comprising at least two rings; wherein:
   - the fused multicyclic ring system is, as a whole, aromatic or partially saturated;
   - a carbon atom of a pyridine within the fused multicyclic ring system is connected to group A via a N-acylsulfonamide function and a 6 membered ring, fused to the pyridine, whether aromatic or partially saturated ring, is connected to group B;
B represents a pyrazole, a thiazole or a pyridine, optionally substituted with one or more substituents selected from methyl, fluoromethyl or difluoromethyl.

In some embodiments, a compound of Formula (I), or pharmaceutically acceptable salt thereof, is a compound of **Formula (II):** 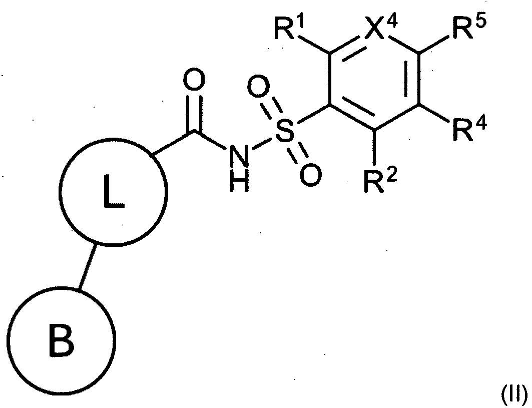 or a pharmaceutically acceptable salt thereof, wherein B, and L are selected as previously described; and wherein:
X⁴ is selected from -C(R³)- or nitrogen;
R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)2); Alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups, and/or halogens and/or hydroxy groups; a halogen; an alkoxy group, such as a C₁-C₄ alkoxy group; a cycloalkyloxy group, optionally substituted by one or more alkyl groups and/or halogens.

In some embodiments, a compound of Formula (I), or pharmaceutically acceptable salt thereof, is a compound of **Formula (III):** 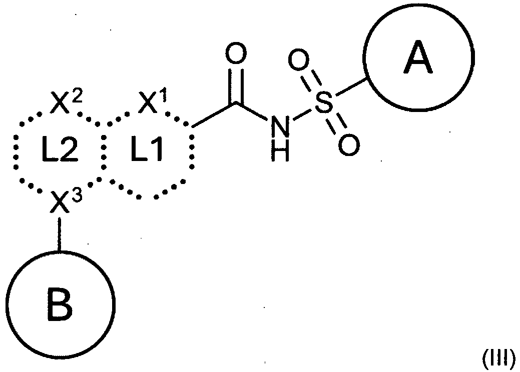 or a pharmaceutically acceptable salt thereof, wherein B, and A are selected as previously described; and wherein:
L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
X¹ represents a nitrogen atom or a carbon atom, each further bonded to a sufficient number of hydrogen atoms to satisfy its valence;
X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and
X³ represents a nitrogen atom or a carbon atom further bonded to a sufficient number of hydrogen atoms to satisfy its valence.

In some embodiments, a compound of Formula (I), or pharmaceutically acceptable salt thereof, is a compound of **Formula (IVa):** 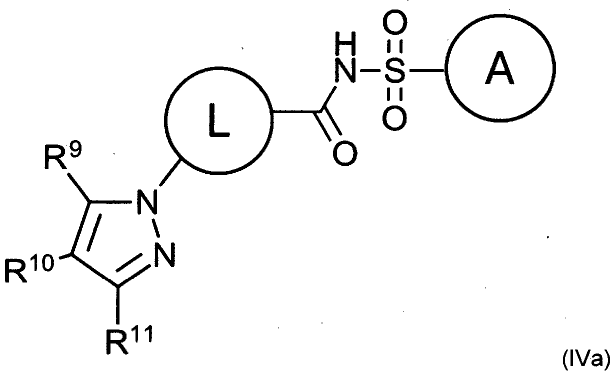 or a pharmaceutically acceptable salt thereof, wherein A, and L are selected as previously described; and wherein: R⁹, R¹⁰ and R¹¹ each independently represent H; or an alkyl group, such as a linear or branched C₁-C₄ alkyl group, optionally substituted by 1 to 3 fluorine atoms; or a cycloalkyl group.

In some embodiments, a compound of Formula (I), or pharmaceutically acceptable salt thereof, is a compound of **Formula (IVb):** 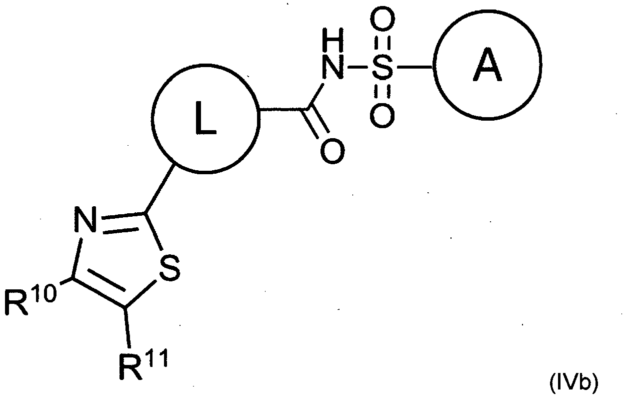 or a pharmaceutically acceptable salt thereof, wherein A, and L are selected as previously described; and wherein: R¹⁰ and R¹¹ each independently represent H; or an alkyl group, such as a linear or branched C₁-C₄ alkyl group, optionally substituted by 1 to 3 fluorine atoms; or a cycloalkyl group.

In some embodiments, a compound of Formula (I), or pharmaceutically acceptable salt thereof, is a compound of **Formula (Va):** 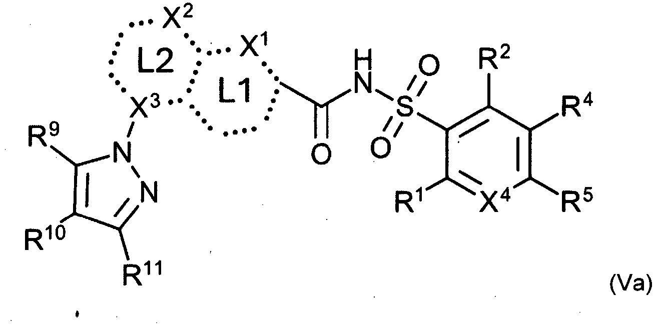 or a pharmaceutically acceptable salt thereof, wherein:
X⁴ is selected from -C-R³ or nitrogen;
R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)2); alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens;
L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
X¹ represents a nitrogen atom or a carbon atom, each further bonded to a sufficient number of hydrogen atoms to satisfy its valence;
X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and X³ represents a carbon atom further bonded to a sufficient number of hydrogen atoms to satisfy its valence; and
R⁹, R¹⁰ and R¹¹ each independently represent H; or an alkyl group, such as a linear or branched C₁-C₄ alkyl group, optionally substituted by 1 to 3 fluorine atoms; or a cycloalkyl group.

In some embodiments, a compound of Formula (I), or pharmaceutically acceptable salt thereof, is a compound of **Formula (Vb):** 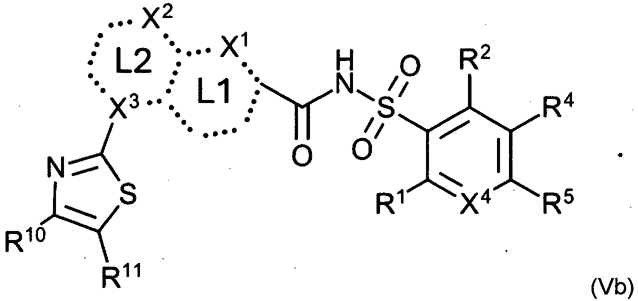 or a pharmaceutically acceptable salt thereof, wherein:
X⁴ is selected from -C-R³ or nitrogen;
R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)2); alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens;
L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
X¹ represents a nitrogen atom or a carbon atom, each further bonded to a sufficient number of hydrogen atoms to satisfy its valence;
X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and
X³ represents a nitrogen atom or a carbon atom further bonded to a sufficient number of hydrogen atoms to satisfy its valence; and
R¹⁰ and R¹¹ each independently represent H; or an alkyl group, such as a linear or branched C₁-C₄ alkyl group, optionally substituted by 1 to 3 fluorine atoms; or a cycloalkyl group.

In some embodiments, the present invention provides compounds of formula (II), or (Va), or (Vb), supra, or (VI) infra, or a pharmaceutically acceptable salt thereof, in which R¹ is selected from H; an alkyl group, such as a C₁-C₄ alkyl group, linear or branched, optionally substituted by 1 to 3 fluorine atoms or a hydroxy group; a cycloalkyl group, optionally substituted by one or more alkyl groups and/or halogens and/or hydroxy; a cycloalkyloxy group, optionally substituted by one or more alkyl groups and/or halogens; or a substituted or unsubstituted amino group, such as dialkylamino groups, such as a dimethylamino group (-N(CH₃)₂.

In a particular embodiment, the present invention provides compounds of formula (II) or (Va), or (Vb) supra, or a pharmaceutically acceptable salt thereof, in which R¹ is selected from a hydrogen atom; a halogen; an alkoxy group; or a cycloalkyloxy group, optionally substituted by one or more alkyl groups and/or halogens and/or hydroxy. Preferably, R¹ is selected from a hydrogen atom; a halogen; or an alkoxy group. More preferably, R¹ is selected from a hydrogen atom; or an alkoxy group. Even more preferably, R¹ is selected from an alkoxy group, such as a methoxy or ethoxy group.

In some embodiments, the present invention provides compounds of formula (II), or (Va), or (Vb), supra, or (VI) infra, or a pharmaceutically acceptable salt thereof, in which R² is selected from H; an alkyl group, such as a C₁-C₄ alkyl group, linear or branched, optionally substituted by 1 to 3 fluorine atoms or a hydroxy group; a cycloalkyl group, optionally substituted by one or more alkyl groups and/or halogens and/or hydroxy; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂.

In a particular embodiment, the present invention provides compounds of formula (II) or (Va) or (Vb), supra, or (VI) infra, or a pharmaceutically acceptable salt thereof, in which R² is selected from a hydrogen atom; a halogen; an alkoxy group; or a cycloalkyloxy group, optionally substituted by one or more alkyl groups and/or halogens and/or hydroxy. Preferably, R² is selected from a hydrogen atom; a halogen; or an alkoxy group. More preferably, R² is selected from a hydrogen atom; or an alkoxy group. Even more preferably, R² is selected from an alkoxy group, such as a methoxy or ethoxy group.

In some embodiments, the present invention provides compounds of formula (II) or (Va) or (Vb), supra, or (VI) infra, or a pharmaceutically acceptable salt thereof, in which R¹ and R² each independently represent an alkoxy group, such as a methoxy or ethoxy group.

In some embodiments, the present invention provides compounds of formula (II), or (Va), or (Vb), supra, or (VI) infra, or a pharmaceutically acceptable salt thereof, in which X⁴ is nitrogen.

In some embodiments, the present invention provides compounds of formula (II), or (Va), or (Vb), supra, or (VI) infra, or a pharmaceutically acceptable salt thereof, in which X⁴ represents -C-R³.

In some embodiments, the present invention provides compounds of formula (II), or (Va), or (Vb), supra, or (VI) infra, or a pharmaceutically acceptable salt thereof, in which when X⁴ represents -C-R³, R³ is selected from H; an alkyl group, such as a C₁-C₄ alkyl group, linear or branched, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group such as a C₁-C₄ alkoxy group; a cycloalkyloxy group, optionally substituted by one or more alkyl groups and/or halogens and/or hydroxy; or a substituted or unsubstituted amino group, such as dialkylamino groups, such as a dimethylamino group (-N(CH₃)₂.

In a particular embodiment, the present invention provides compounds of formula (II) or (Va), or (Vb), supra, or (VI) infra, or a pharmaceutically acceptable salt thereof, in which when X⁴ represents -C-R³, R³ is selected from a hydrogen atom; an alkyl group, such as a C₁-C₄ alkyl group, linear or branched, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a cycloalkyloxy group, optionally substituted by one or more alkyl groups and/or halogens; or a substituted or unsubstituted amino group, such as dialkylamino groups, such as a dimethylamino group (-N(CH₃)₂). Preferably, R³ is selected from a hydrogen atom and a C₁-C₄ alkyl group, linear or branched, substituted by 1 to 3 fluorine atoms or a hydroxy group; a heterocyclyl group substituted by one or more alkyl groups and/or halogens. More preferably, R³ is selected from a hydrogen atom and a C₁-C₄ alkyl group, linear or branched, substituted by 1 to 3 fluorine atoms or a hydroxy group. Even more preferably, R³ represents a hydrogen atom.

In some embodiments, the present invention provides compounds of formula (II), or (Va), or (Vb), supra, or (VI) infra, or a pharmaceutically acceptable salt thereof, in which R⁴ is selected from H; an alkyl group, such as a C₁-C₄ alkyl group, linear or branched, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂.

In a particular embodiments, the present invention provides compounds of formula (II) or (Va), or (Vb), supra, or (VI) infra, or a pharmaceutically acceptable salt thereof, in which R⁴ is selected from a hydrogen atom; an alkyl group, such as a C₁-C₄ alkyl group, linear or branched, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a cycloalkyloxy group, optionally substituted by one or more alkyl groups and/or halogens; or a substituted or unsubstituted amino group, such as dialkylamino groups, such as a dimethylamino group (-N(CH₃)₂). Preferably, R⁴ is selected from a hydrogen atom and a C₁-C₄ alkyl group, linear or branched, substituted by 1 to 3 fluorine atoms or a hydroxy group; or a heterocyclyl group optionally substituted by one or more alkyl groups, hydroxy groups, alkoxy groups, dialkylamino groups, oxo groups and/or halogens. More preferably, R⁴ is selected from a hydrogen atom and a C₁-C₄ alkyl group, linear or branched, substituted by 1 to 3 fluorine atoms or a hydroxy group or a pyrrolidine or piperidine, optionally substituted by one or more alkyl groups, hydroxy groups, alkoxy groups, dialkylamino groups, oxo groups and/or halogens. Even more preferably, R⁴ represents a hydrogen atom.

In some embodiments, the present invention provides compounds of formula (II), or (Va), or (Vb), supra, or (VI) infra, or a pharmaceutically acceptable salt thereof, in which R⁵ is selected from H; an alkyl group, such as a C₁-C₄ alkyl group, linear or branched, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂.

In a particular embodiment, the present invention provides compounds of formula (II) or (Va), or (Vb), supra, or (VI) infra, or a pharmaceutically acceptable salt thereof, in which R⁵ is selected from a hydrogen atom, a halogen atom or a 4- to 6-member heterocyclyl group, such as azetidine, pyrrolidine, piperidine or piperazine, optionally substituted by one or more alkyl groups, oxo groups, hydroxy groups, dialkylamino groups and/or halogens. More preferably, R⁵ represents a hydrogen atom.

In some embodiments, the present invention provides compounds of formula (II) or (Va), or (Vb), supra, or (VI) infra, or a pharmaceutically acceptable salt thereof, in which R² and R⁴ taken together, a heterocycloalkyl group such as a 5- or 6-member heterocycloalkyl group or a heteroaromatic ring or a cycloalkyl group such as a 5- or 6-member cycloalkyl group, optionally substituted by one or more alkyl groups and/or halogens; while the remaining R¹, R⁵, and, when present, R³, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a 4- to 6-member heterocyclyl group, optionally substituted by one or more alkyl groups, oxo groups, hydroxy groups, dialkylamino groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂).

In some embodiments, the present invention provides compounds of formula (II) or (Va), or (Vb), supra, or (VI) infra, or a pharmaceutically acceptable salt thereof, in which R⁴ and R⁵ taken together, a heterocycloalkyl group such as a 5- or 6-member heterocycloalkyl group, or a heteroaromatic ring, or a cycloalkyl group such as a 5- or 6-member cycloalkyl group, optionally substituted by one or more alkyl groups and/or halogens; while the remaining R¹ and R², and when present, R³, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a 4- to 6-member heterocyclyl group, optionally substituted by one or more alkyl groups, oxo groups and/or halogens; a halogen; an alkoxy group such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂).

In some embodiments, a compound of Formula (I) or pharmaceutically acceptable salt thereof, is a compound of Formula (VI): 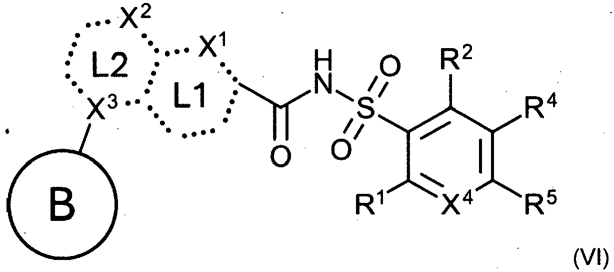 or a pharmaceutically acceptable salt thereof, wherein
L2 is aromatic; X¹ represents nitrogen further bonded to a sufficient number of hydrogen atoms to satisfy its valence; X² represents -C-R⁶; X³ is carbon; R¹ and R² are independently alkoxy group, such as a C₁-C₄ alkoxy group;
and wherein B, L1, R⁶, X⁴, R⁴, R⁵ are selected as previously described.

In some embodiments; the present invention provides compounds of formula (III) or (Va), or (Vb), supra, or a pharmaceutically acceptable salt thereof, in which L1-L2 represents a fused multicyclic ring system comprising at least two rings; wherein: a pyridine within the fused multicyclic ring system (in particular a carbon atom) is connected to group A via a N-acylsulfonamide function and a 6 membered ring, fused to the pyridine, whether aromatic or partially saturated ring, is connected to group B.

In a preferred embodiment, the present invention provides compounds of formula (III) or (Va), or (Vb), supra, or a pharmaceutically acceptable salt thereof, wherein a pyridine within the fused multicyclic ring system (in particular a carbon atom) is connected to group A via a N-acylsulfonamide function and a 6 membered aromatic ring, fused to the pyridine, is connected to group B.

In some embodiments, a compound of Formula (I) or Formula (II), or pharmaceutically acceptable salt thereof, is a compound of Formula (IIa): 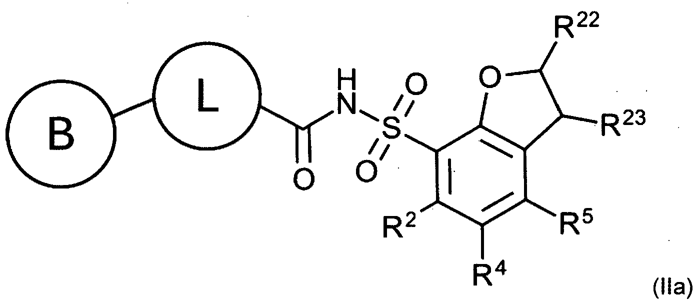 or a pharmaceutically acceptable salt thereof, wherein B, L, R², R⁴, and R⁵, are selected as previously described; and R²² and R²³, each independently, represent H; or an alkyl group.

In some embodiments, a compound of Formula (I) or Formula (II), or pharmaceutically acceptable salt thereof, is a compound of Formula (IIb): 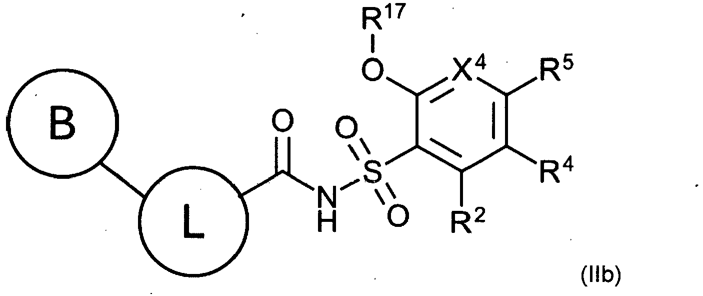 or a pharmaceutically acceptable salt thereof, wherein B, L, X⁴, R², R⁴, and R⁵, are selected as previously described; and wherein R¹⁷ represents an alkyl group or a cycloalkyl group.

In some embodiments, a compound of Formula (I) or Formula (II), or pharmaceutically acceptable salt thereof, is a compound of Formula (IIb-1): 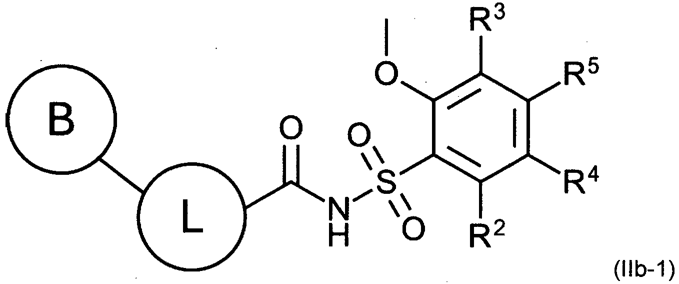 or a pharmaceutically acceptable salt thereof, wherein B, L, R², R³, R⁴, and R⁵, are selected as previously described.

In some embodiments, a compound of Formula (I) or Formula (II), or pharmaceutically acceptable salt thereof, is a compound of Formula (IIc): 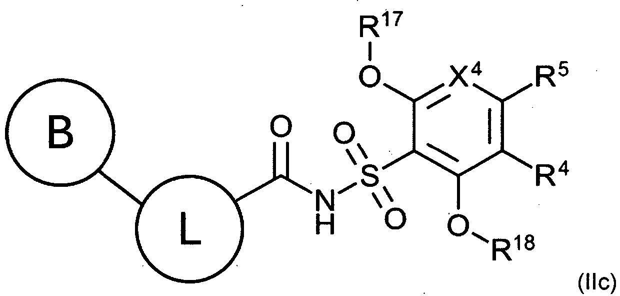 or a pharmaceutically acceptable salt thereof, wherein B, L, X⁴, R⁴, and R⁵, are selected as previously described; and wherein R¹⁷ and R¹⁸ represents, each independently, an alkyl group or a cycloalkyl group.

In some embodiments, a compound of Formula (I) or Formula (II), or pharmaceutically acceptable salt thereof, is a compound of Formula (Ild): 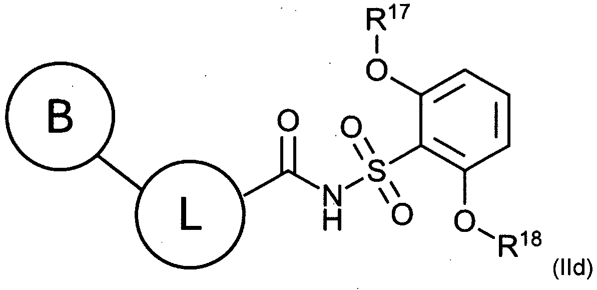 or a pharmaceutically acceptable salt thereof, wherein B and L are selected as previously described; and wherein R¹⁷ and R¹⁸ represents, each independently, an alkyl group or a cycloalkyl group.

In some embodiments, a compound of Formula (I) or Formula (II), or pharmaceutically acceptable salt thereof, is a compound of Formula (IId-1): 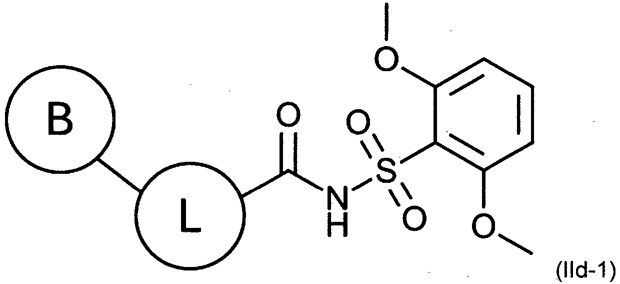 or a pharmaceutically acceptable salt thereof, wherein B and L are selected as previously described.

In some embodiments, a compound of Formula (I) or Formula (II), or pharmaceutically acceptable salt thereof, is a compound of Formula (Ile): 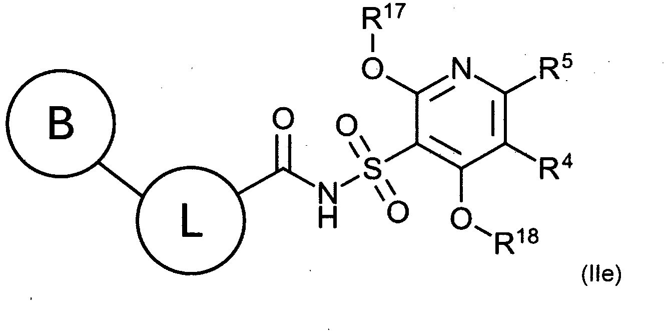 or a pharmaceutically acceptable salt thereof, wherein B, L, R⁴, and R⁵, are selected as previously described; and wherein R¹⁷ and R¹⁸ represents, each independently, an alkyl group or a cycloalkyl group.

In some embodiments, a compound of Formula (I) or Formula (II), or pharmaceutically acceptable salt thereof, is a compound of Formula (lle-1): 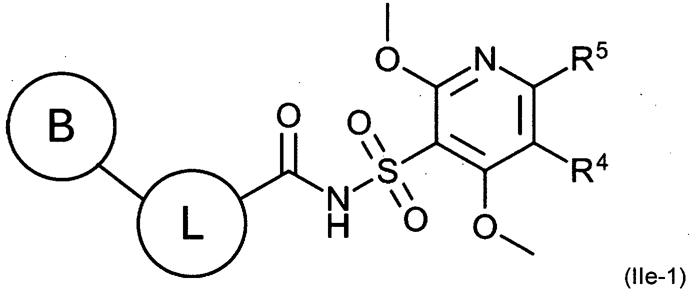 or a pharmaceutically acceptable salt thereof, wherein B, L, R⁴, and R⁵, are selected as previously described.

In some embodiments, a compound of Formula (I) or Formula (II), or pharmaceutically acceptable salt thereof, is a compound of Formula (IIe-2): 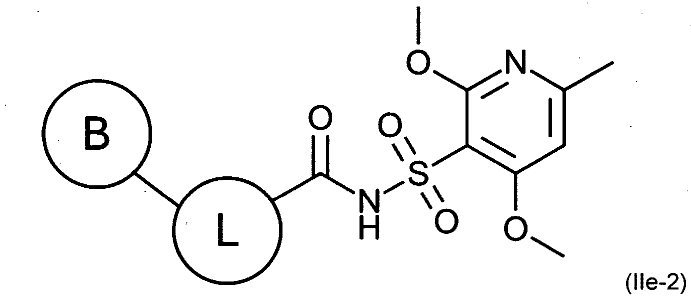 or a pharmaceutically acceptable salt thereof, wherein B and L are selected as previously described.

In some embodiments, a compound of Formula (I) or Formula (III), or pharmaceutically acceptable salt thereof, is a compound of Formula (IIIa-1): 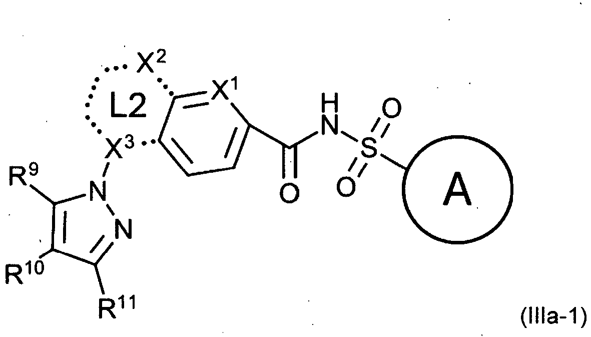 or a pharmaceutically acceptable salt thereof, wherein A, L2, X¹, X², X³, R⁹, R¹⁰, and R¹¹, are selected as previously described. Preferably, X¹ represents a nitrogen atom; X² represents a carbon atom substituted by R⁶ group as previously described; X³ represents a carbon atom; and L2 is aromatic.

In some embodiments, a compound of Formula (I) or Formula (III), or pharmaceutically acceptable salt thereof, is a compound of Formula (IIIa-2): 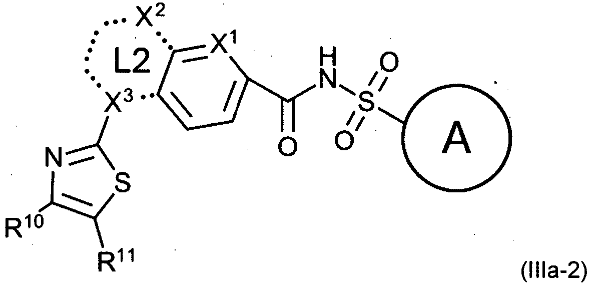 or a pharmaceutically acceptable salt thereof, wherein A, L2, X¹, X², X³, R¹⁰, R¹¹, are selected as previously described. Preferably, X¹ represents a nitrogen atom; X² represents a carbon atom substituted by R⁶ group as previously described; X³ represents a carbon atom; and L2 is aromatic.

In some embodiments, a compound of Formula (I) or Formula (III), or pharmaceutically acceptable salt thereof, is a compound of Formula (IIIb-1): 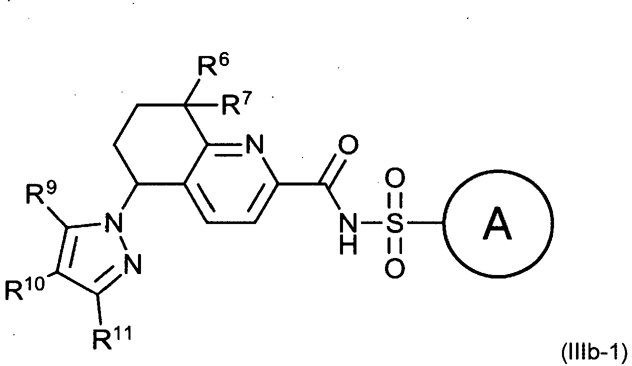 or a pharmaceutically acceptable salt thereof, wherein A, R⁶, R⁷, R⁹, R¹⁰, and R¹¹, are selected as previously described. Preferably, R⁶ and R⁷ together forms a C₃-C₅ cycloalkyl.

In some embodiments, a compound of Formula (I) or Formula (III), or pharmaceutically acceptable salt thereof, is a compound of Formula (Illb-2): 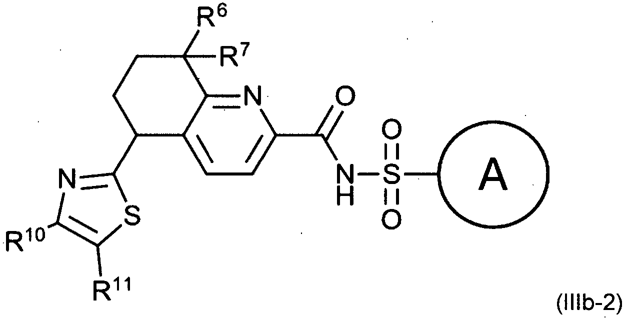 or a pharmaceutically acceptable salt thereof, wherein A, R⁶, R⁷, R⁹, R¹⁰, and R¹¹, are selected as previously described. Preferably, R⁶ and R⁷ together forms a C₃-C₅ cycloalkyl.

In some embodiments, a compound of Formula (I) or Formula (III), or pharmaceutically acceptable salt thereof, is a compound of Formula (Illc-1): 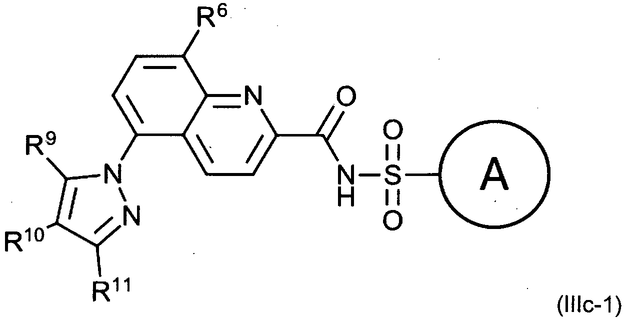 or a pharmaceutically acceptable salt thereof, wherein A, R⁶, R⁹, R¹⁰, and R¹¹, are selected as previously described. Preferably, R⁹ is selected from hydrogen or methyl, and each of R¹⁰ and R¹¹ independently represents hydrogen.

In some embodiments, a compound of Formula (I) or Formula (III), or pharmaceutically acceptable salt thereof, is a compound of Formula (IIIc-2): 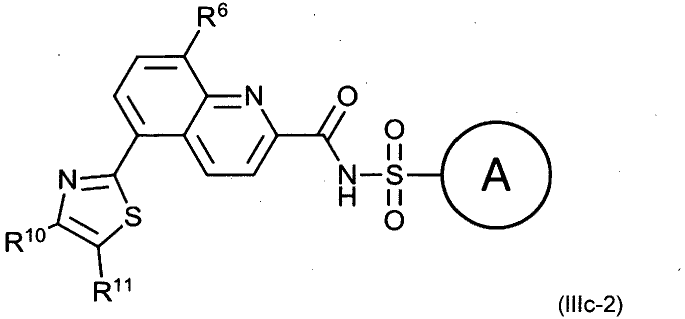 or a pharmaceutically acceptable salt thereof, wherein A, R⁶, R¹⁰, and R¹¹, are selected as previously described. Preferably, R⁶ is selected from halogen, cyclopropyl or methyl; and each of R¹⁰ and R¹¹ independently represents hydrogen.

In some embodiments, a compound of Formula (I) or Formula (III), or pharmaceutically acceptable salt thereof, is a compound of Formula (IIId-1): 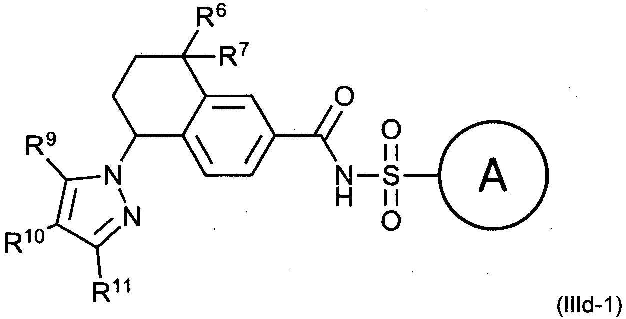 or a pharmaceutically acceptable salt thereof, wherein A, R⁶, R⁷, R⁹, R¹⁰, and R¹¹, are selected as previously described. Preferably, each of R⁶, R⁷, R⁹, R¹⁰, and R¹¹ independently represents hydrogen. More preferably, R⁶ and R⁷ together forms a C₃-C₅ cycloalkyl group; and each of R⁹, R¹⁰, and R¹¹ independently represents hydrogen.

In some embodiments, a compound of Formula (I) or Formula (III), or pharmaceutically acceptable salt thereof, is a compound of Formula (IIId-2): 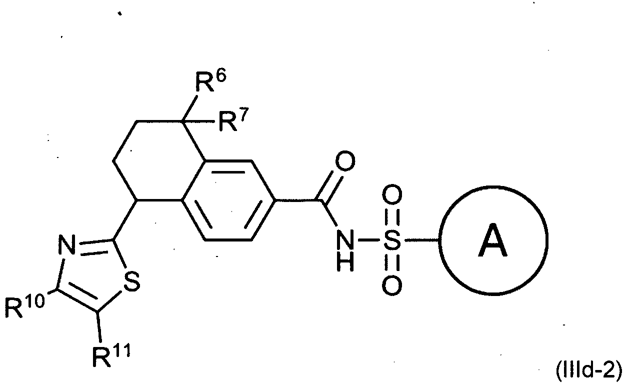 or a pharmaceutically acceptable salt thereof, wherein A, R⁶, R⁷, R¹⁰, and R¹¹, are selected as previously described. Preferably, each of R⁶, R⁷, R¹⁰, and R¹¹ independently represents hydrogen. More preferably, R⁶ and R⁷ together forms a C₃-C₅ cycloalkyl group; and each of R¹⁰, and R¹¹ independently represents hydrogen.

In some embodiments, a compound of Formula (I) or Formula (III), or pharmaceutically acceptable salt thereof, is a compound of Formula (Ille-1): 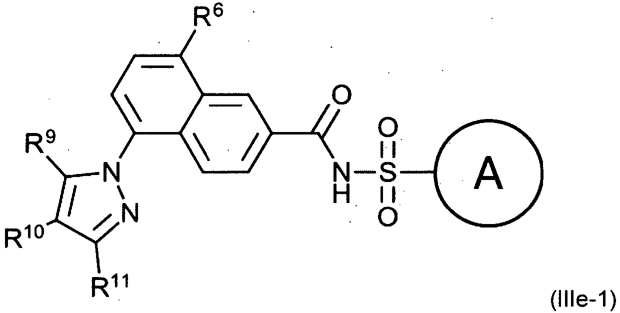 or a pharmaceutically acceptable salt thereof, wherein A, R⁶, R⁹, R¹⁰, and R¹¹, are selected as previously described. Preferably, each of R⁹, R¹⁰, and R¹¹ independently represents hydrogen; and R⁶ is selected from hydrogen, fluorine, chlorine, cyclopropyl, or methyl.

In some embodiments, a compound of Formula (I) or Formula (III), or pharmaceutically acceptable salt thereof, is a compound of Formula (IIId-2): 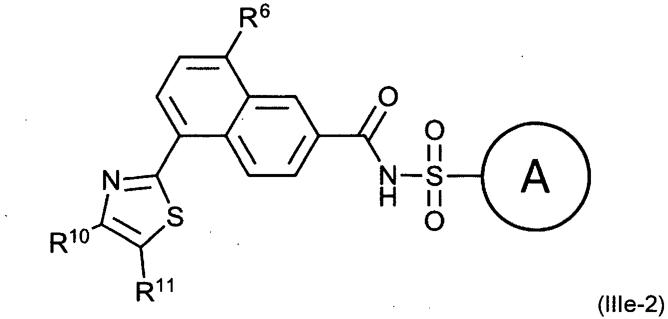 or a pharmaceutically acceptable salt thereof, wherein A, R⁶, R¹⁰, and R¹¹, are selected as previously described. Preferably, each of R¹⁰, and R¹¹ independently represents hydrogen; and R⁶ is selected from hydrogen, fluorine, chlorine, cyclopropyl, or methyl.

In some embodiments, a compound of Formula ((I), (II), (IIa), (lib), (IIb-1), (IIc), (IIc-1), (IId), (Ild-1), (IIe), (Ile-1), (Ile-2), (Illa-1), (Illa-2), (IIIb-1), (Illb-2), (Illc-1), (IIIc-2), (IIId-1), (IIId-2), (Ille-1), (IIIe-2), (IVa), (IVb), (Va), (Vb), (VI), or a pharmaceutically acceptable salt thereof, is a compound wherein the compound has the structure of a compound shown in Table 1, or a pharmaceutically acceptable salt thereof.

**Table 1**

| (e1 refers to enantiomer 1; e2 refers to enantiomer 2) | |
|---|---|
| **No.** | **Structure** |
| 1 | 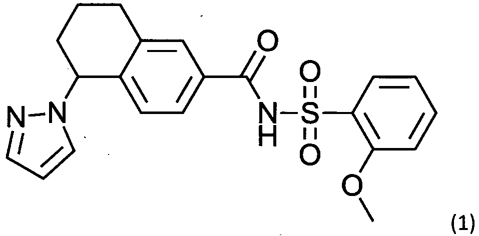 |
| | 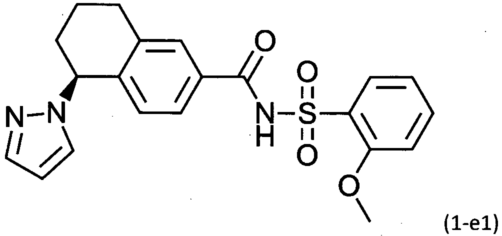 |
| | 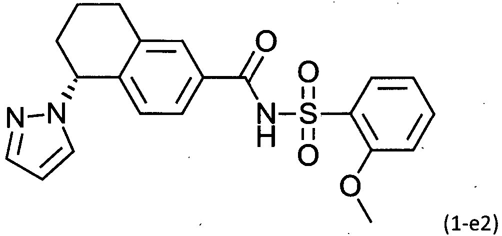 |
| 2 | 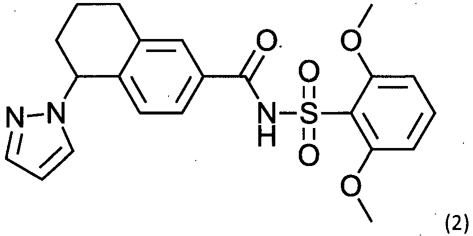 |
| | 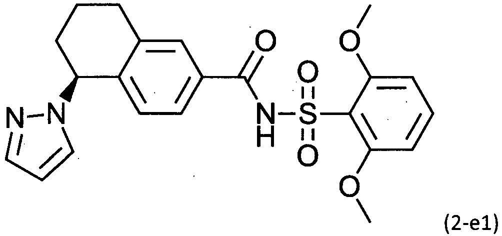 |
| | 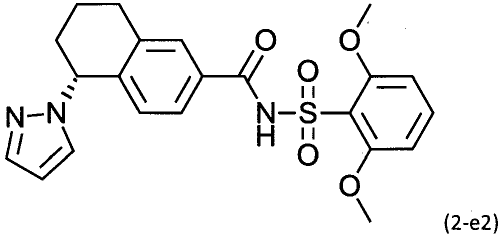 |
| 3 | 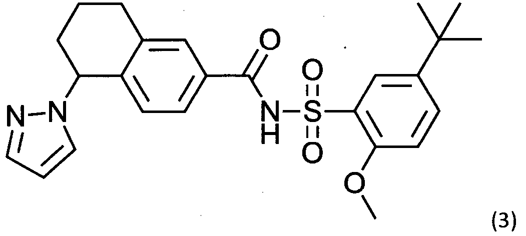 |
| | 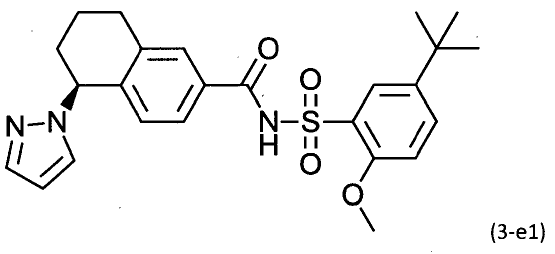 |
| | 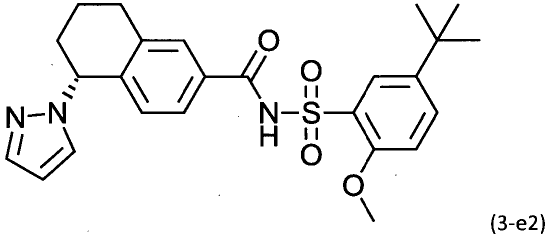 |
| 4 | 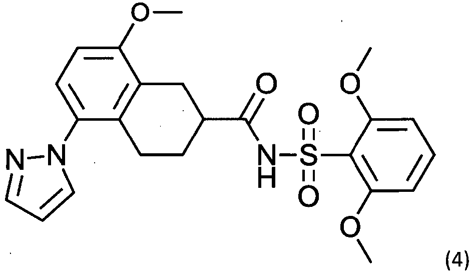 |
| | 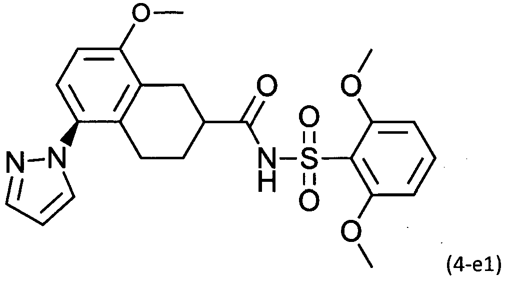 |
| | 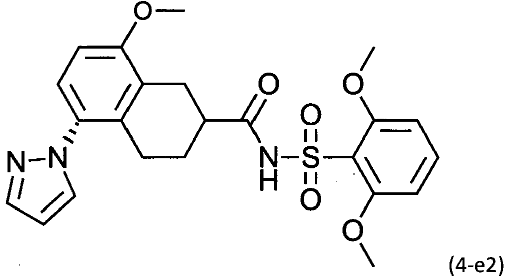 |
| 5 | 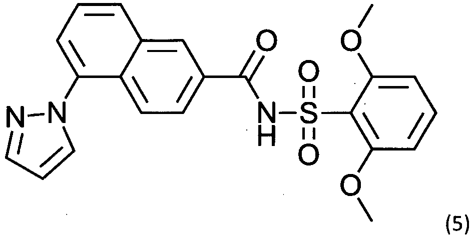 |
| | 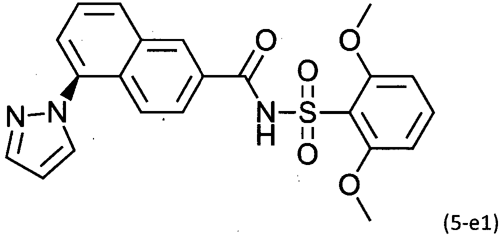 |
| | 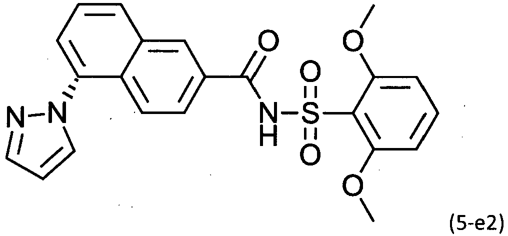 |
| 6 | 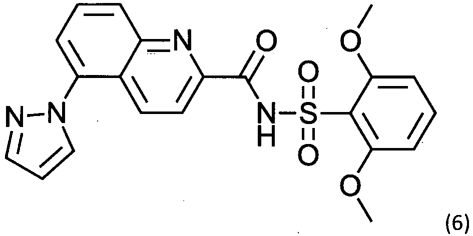 |
| | 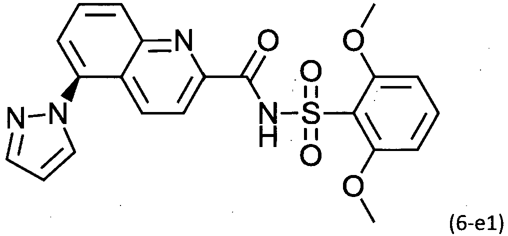 |
| | 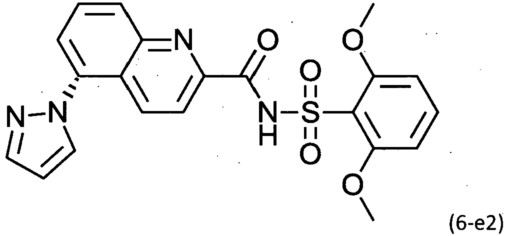 |
| 7 | 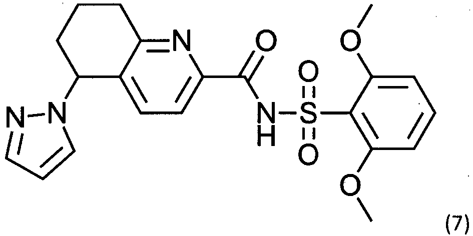 |
| | 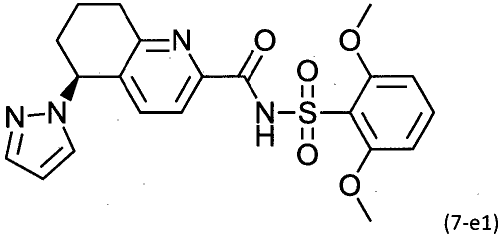 |
| | 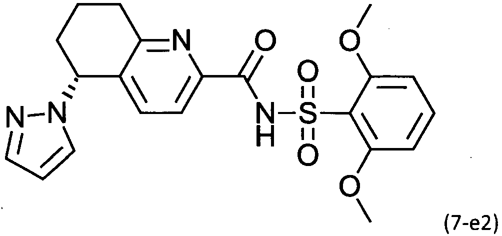 |
| 8 | 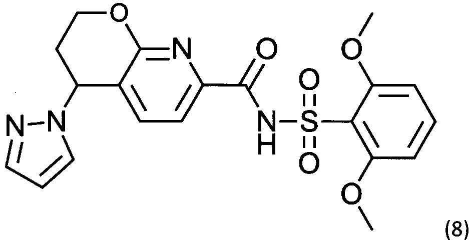 |
| | 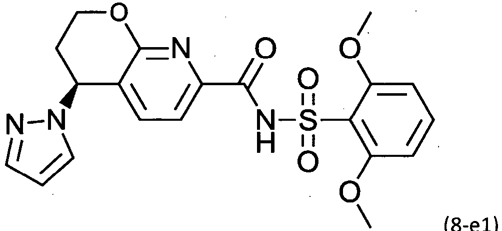 |
| | 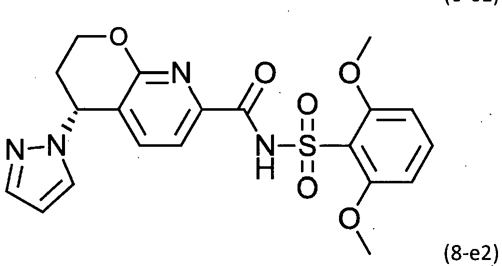 |
| 9 | 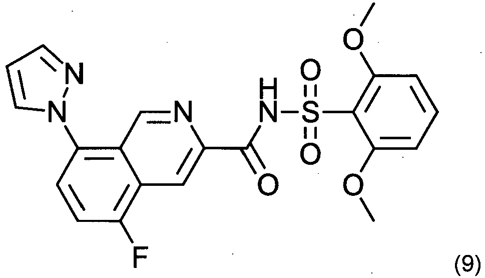 |
| | 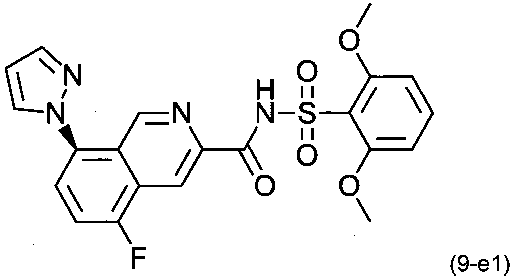 |
| | 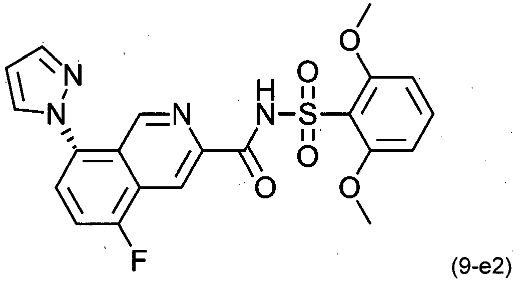 |
| 10 | 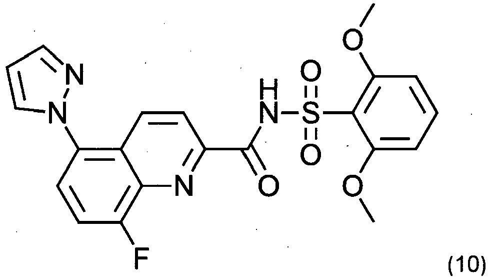 |
| | 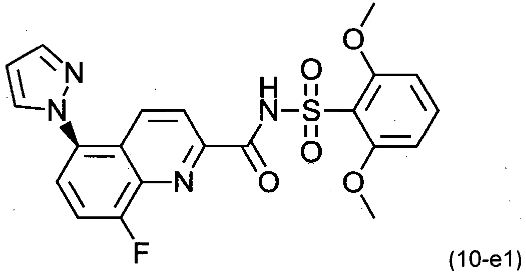 |
| | 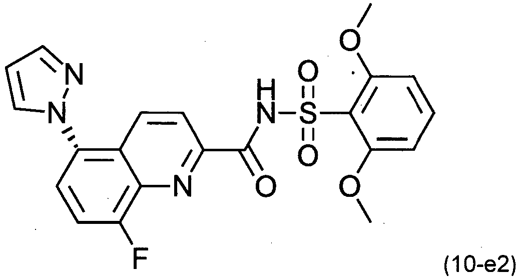 |
| 11 | 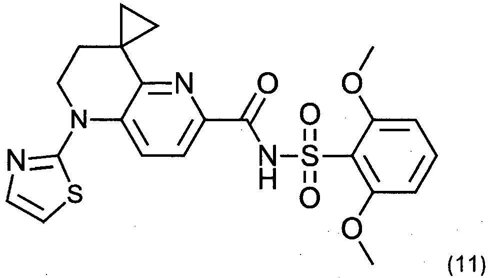 |
| | 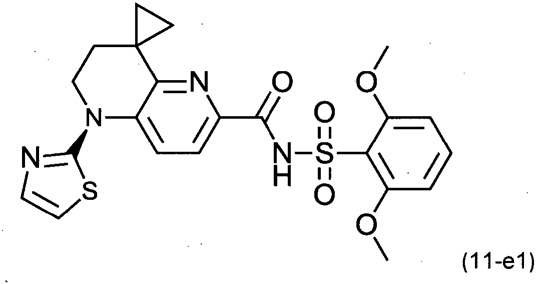 |
| | 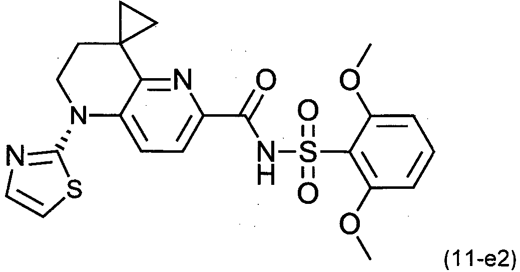 |
| 12 | 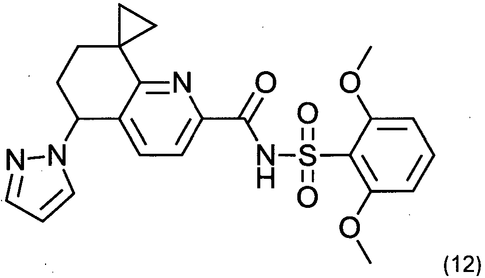 |
| | 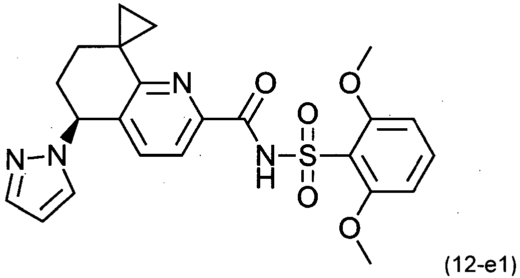 |
| | 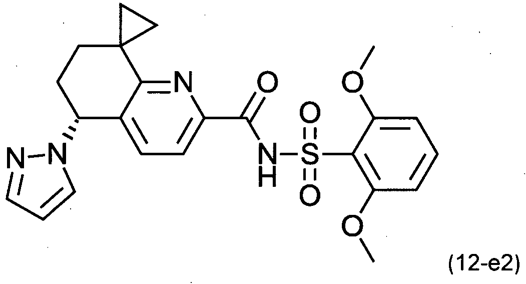 |
| 13 | 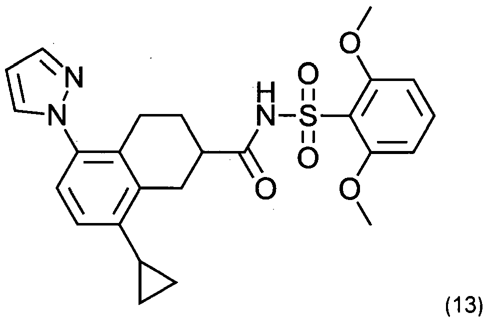 |
| | 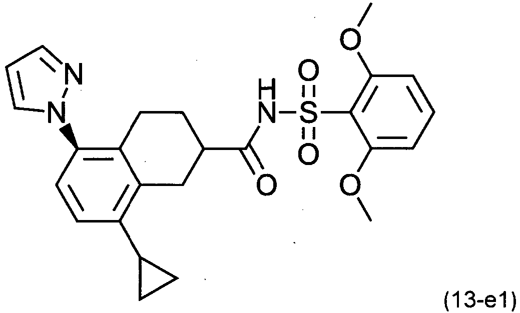 |
| | 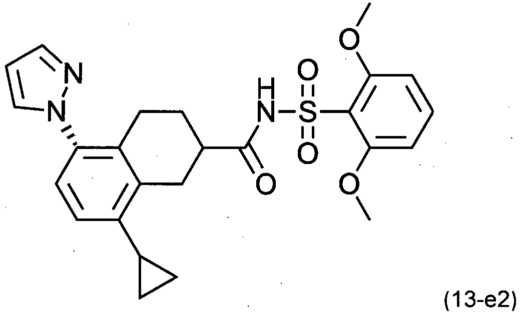 |
| 14 | 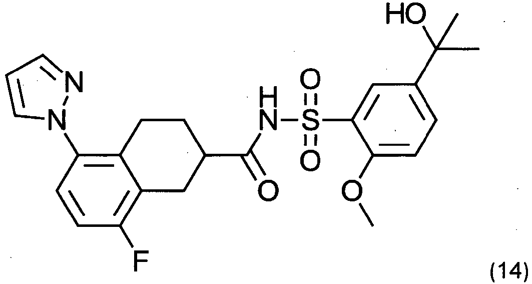 |
| | 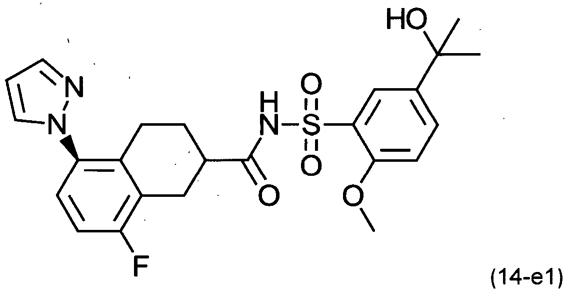 |
| | 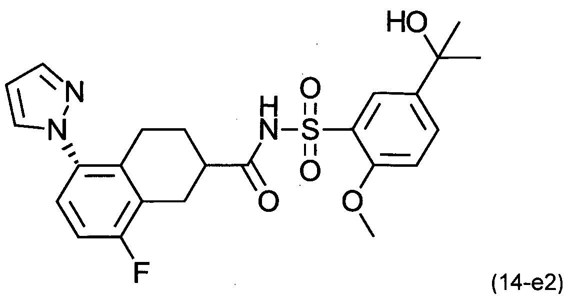 |
| 15 | 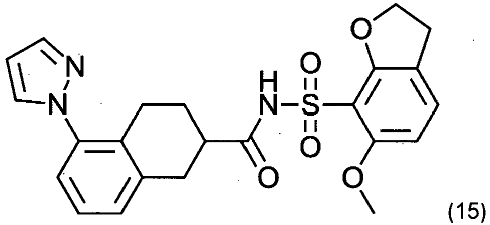 |
| | 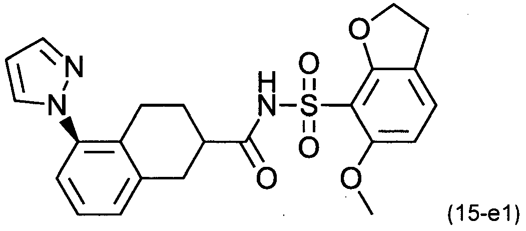 |
| | 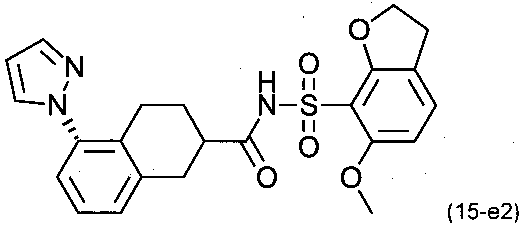 |
| 16 | 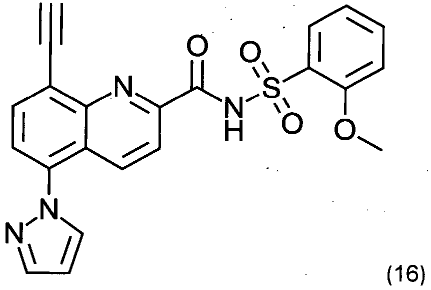 |
| | 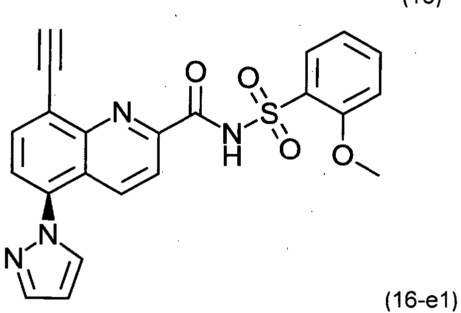 |
| | 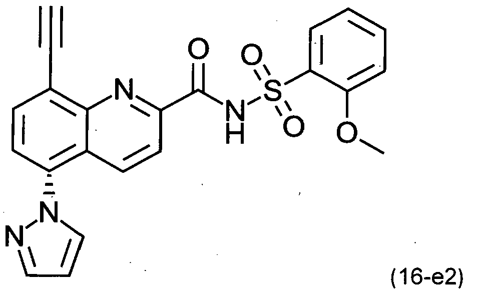 |
| 17 | 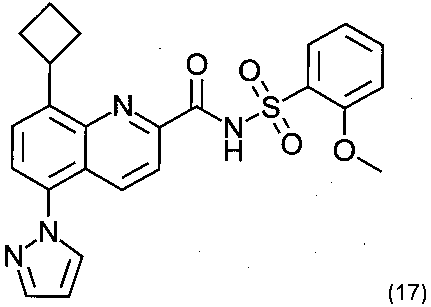 |
| | 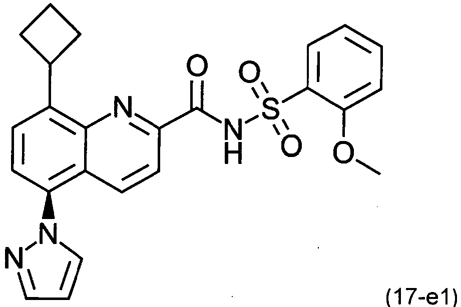 |
| | 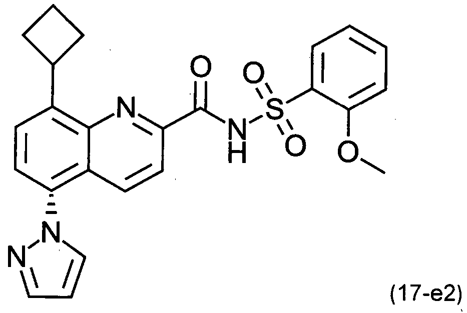 |
| 18 | 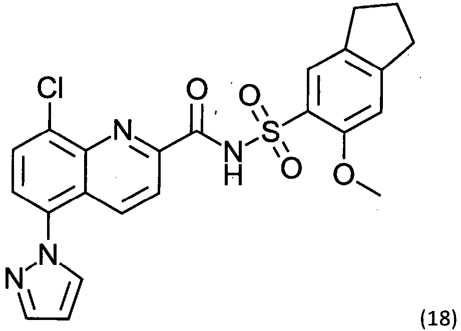 |
| | 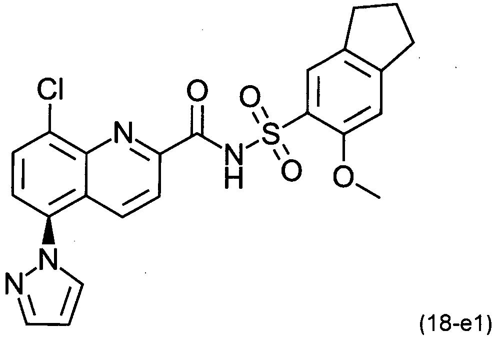 |
| | 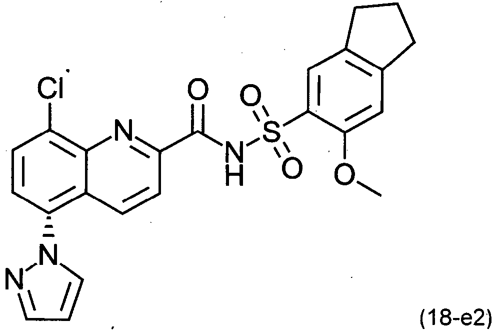 |
| 19 | 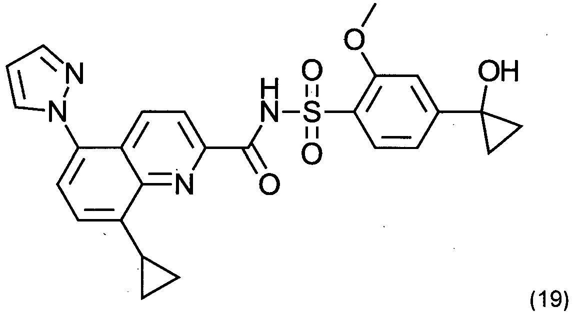 |
| | 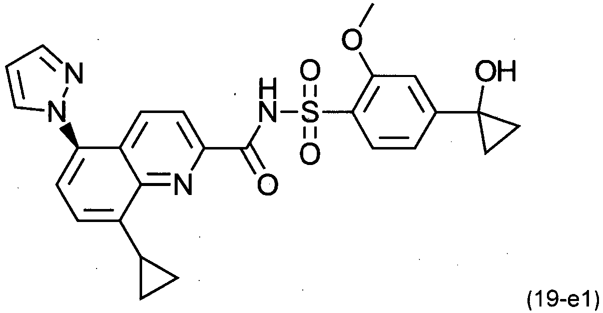 |
| | 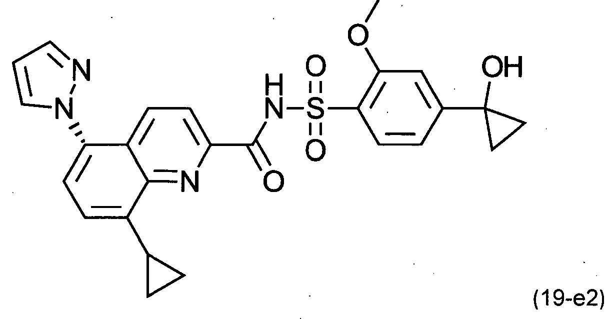 |
| 20 | 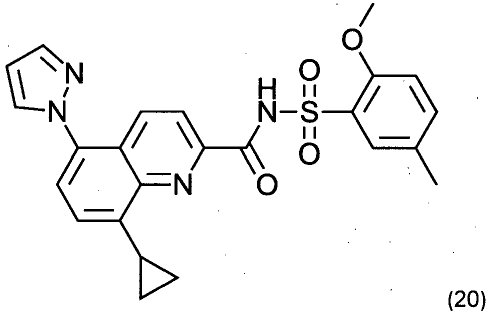 |
| | 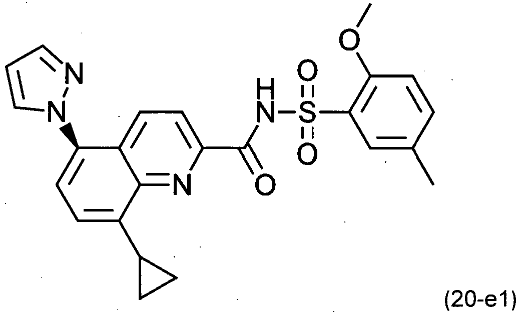 |
| | 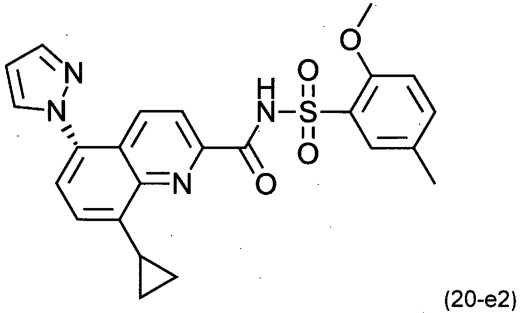 |
| 21 | 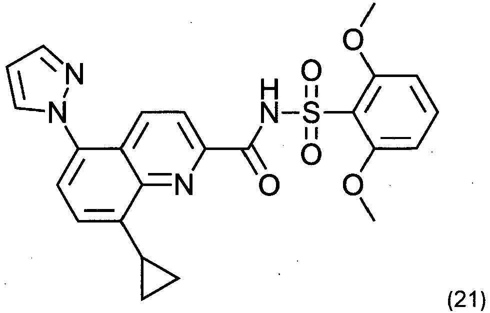 |
| | 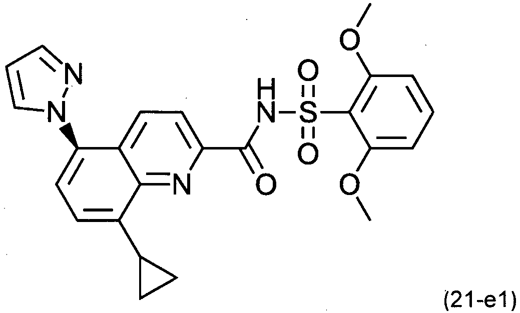 |
| | 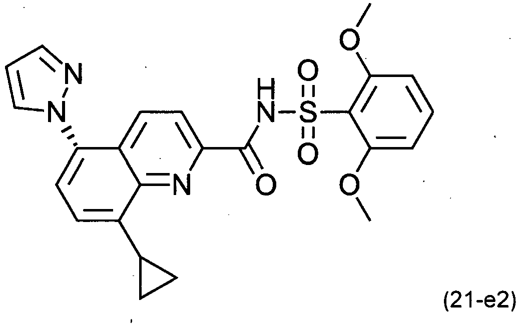 |
| 22 | 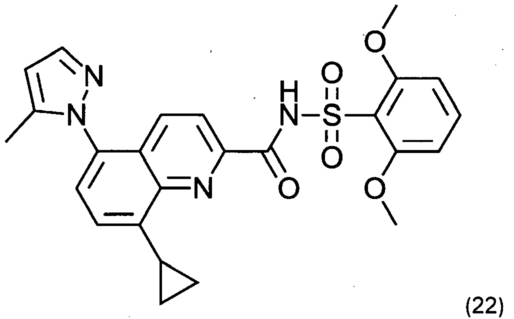 |
| | 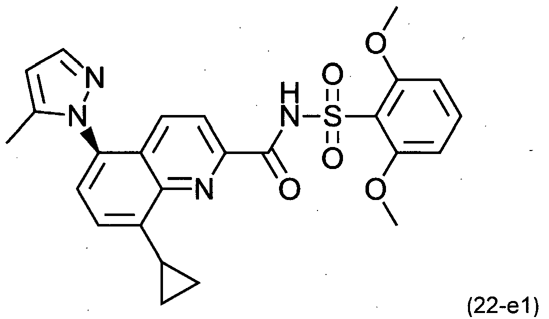 |
| | 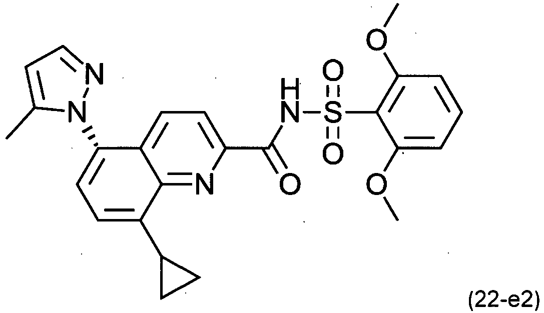 |
| 23 | 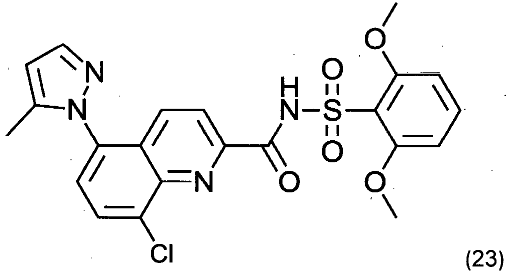 |
| | 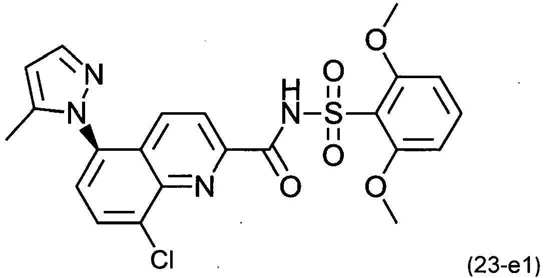 |
| | 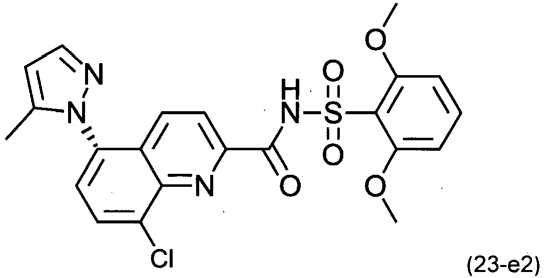 |
| 24 | 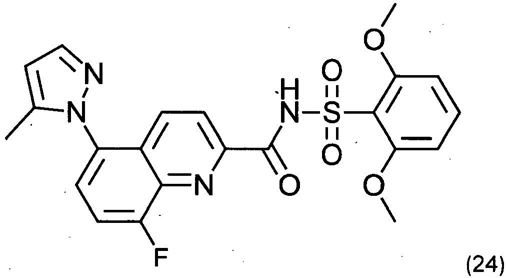 |
| | 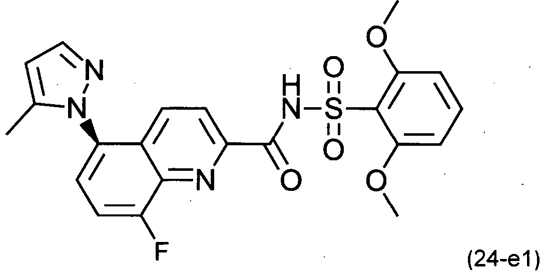 |
| | 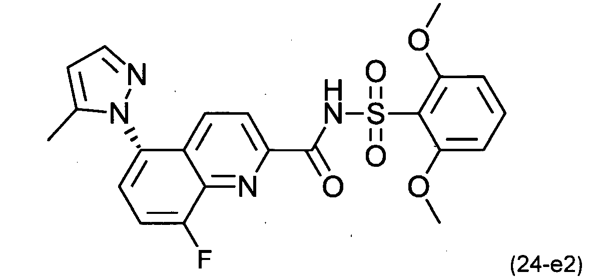 |
| 25 | 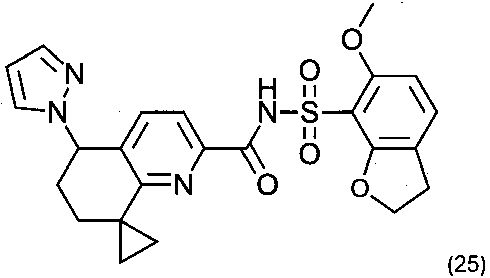 |
| | 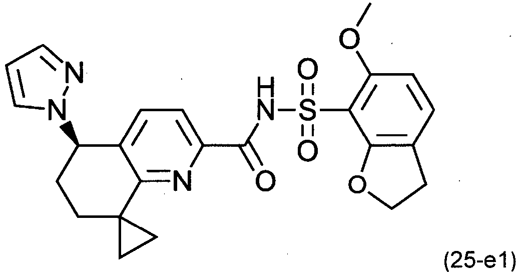 |
| | 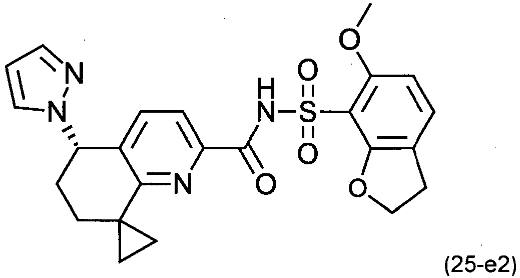 |
| 26 | 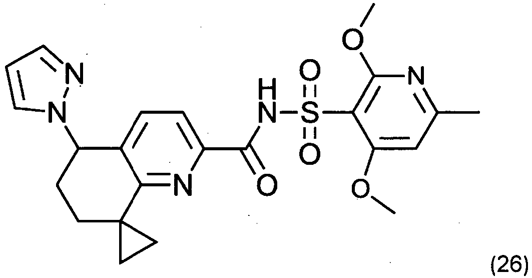 |
| | 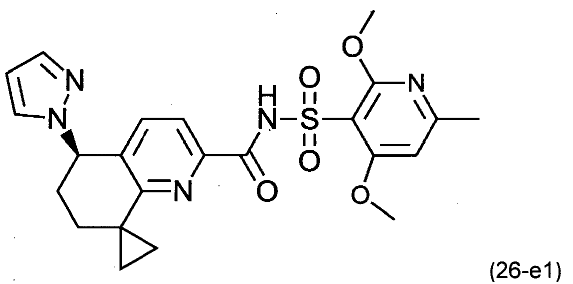 |
| | 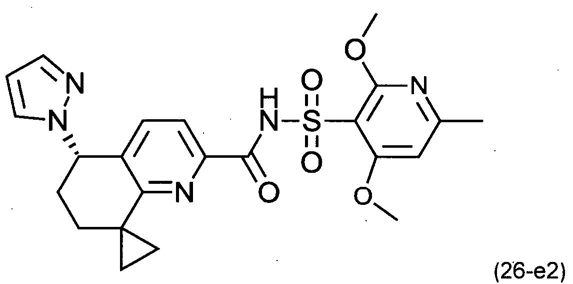 |
| 27 | 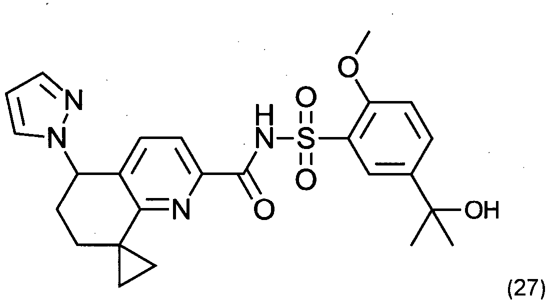 |
| | 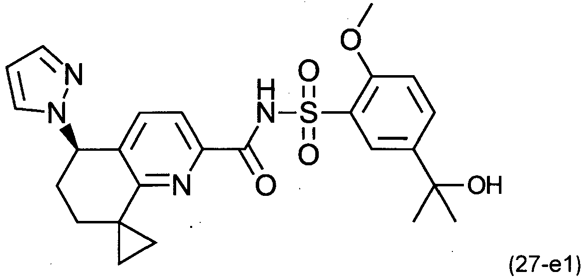 |
| | 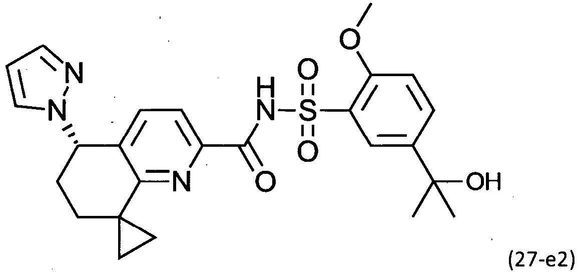 |
| 28 | 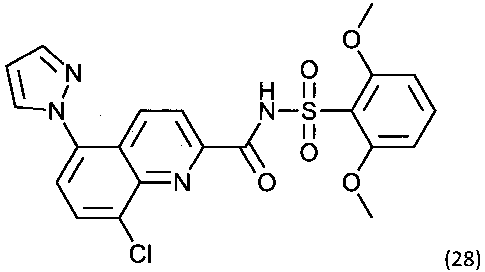 |
| | 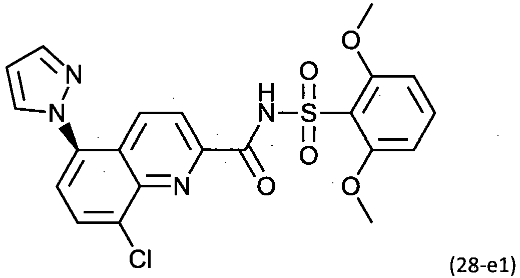 |
| | 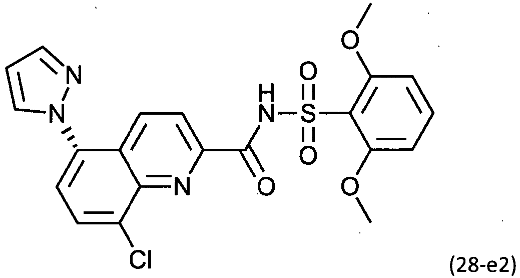 |
| 29 | 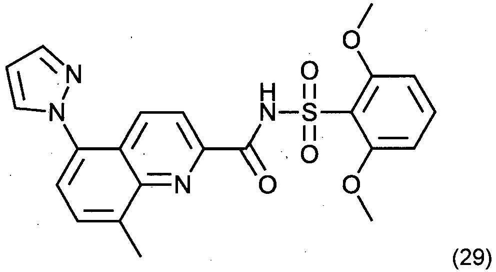 |
| | 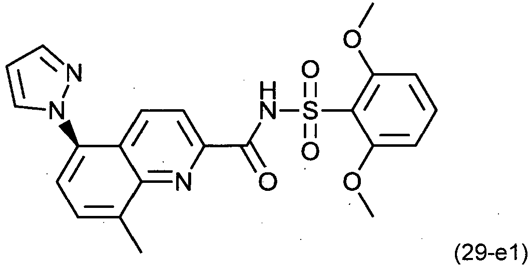 |
| | 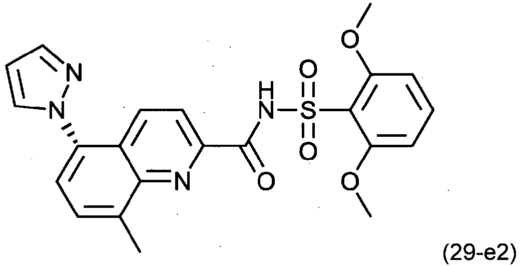 |
| 30 | |
| | 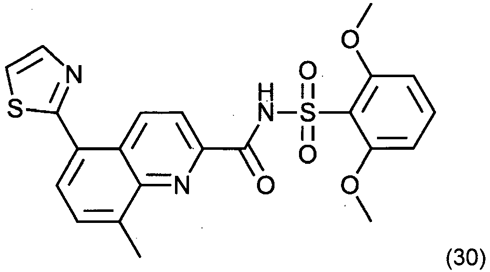 |
| | 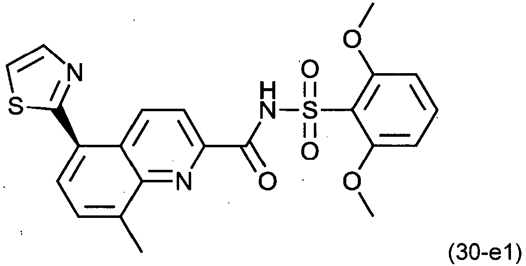 |
| | 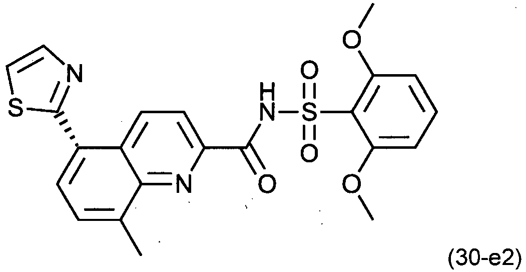 |
| 31 | 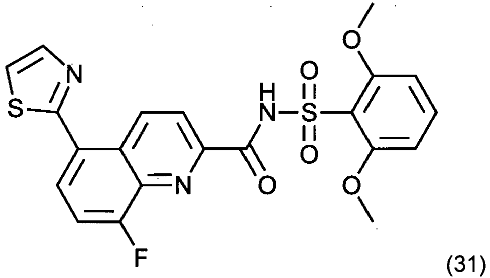 |
| | 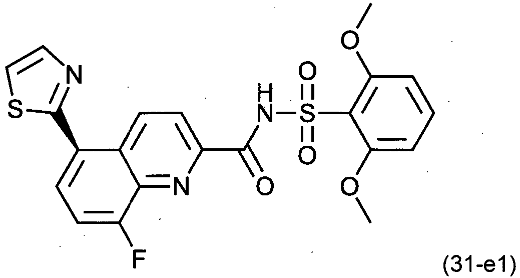 |
| | 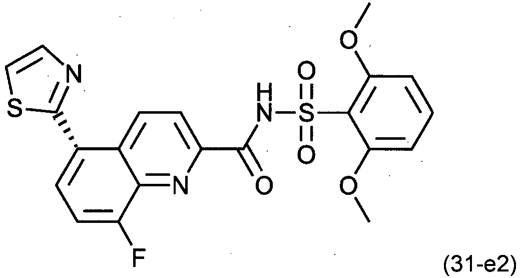 |
| 32 | 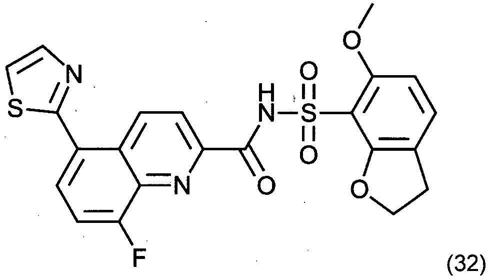 |
| | 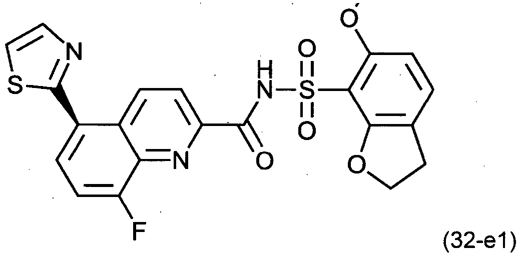 |
| | 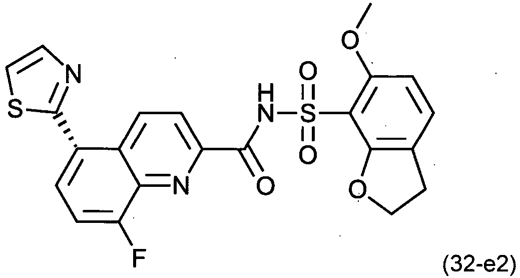 |
| 33 | 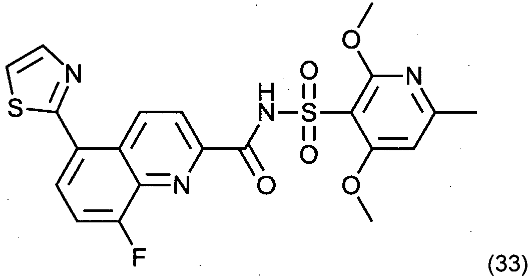 |
| | 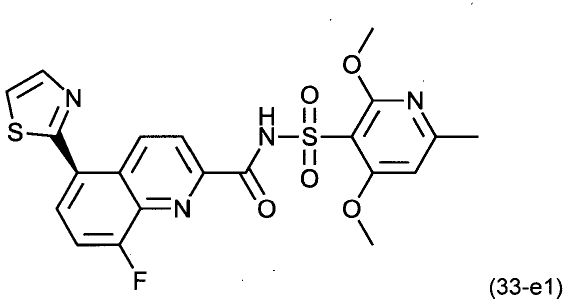 |
| | 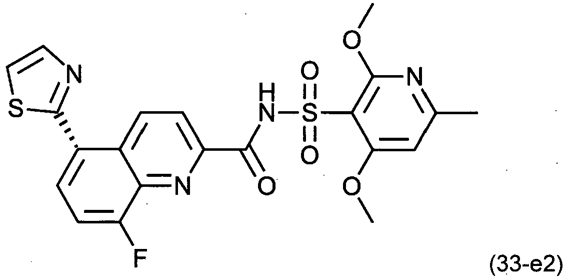 |
| 34 | 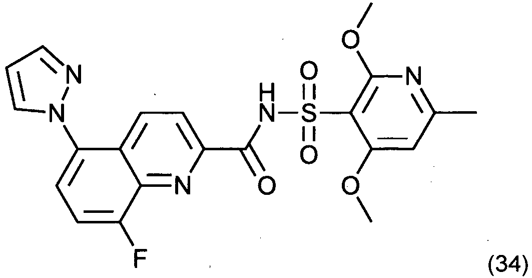 |
| | 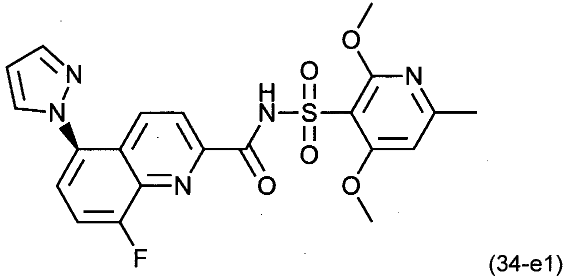 |
| | 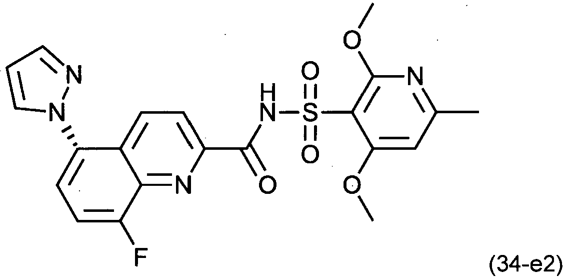 |

### Pharmaceutical Compositions

The present invention relates to pharmaceutical compositions comprising at least one active compound of Formula (I), (II), (IIa), (Ilb), (IIb-1), (IIc), (IIc-1), (IId), (IId-1), (Ile), (Ile-1), (Ile-2), (IIIa-1), (Illa-2), (IIIb-1), (Illb-2), (IIIc-1), (Illc-2), (IIId-1), (Illd-2), (Ille-1), (IIIe-2), (IVa), (IVb), (Va), (Vb), (VI) or a pharmaceutically acceptable salt thereof. These compositions are formulated with one or more pharmaceutically acceptable excipients suitable for human or animal use, aiming to provide effective treatment for cancer and related disorders.

The compounds of the present invention may be administered alone or in combination with various pharmaceutically acceptable carriers, adjuvants, diluents, fillers, buffers, stabilizers, preservatives, lubricants, or other materials commonly used in pharmaceutical formulations. These excipients are typically approved by regulatory authorities or are generally regarded as safe for human or animal use.

Pharmaceutically acceptable excipients include, but are not limited to, carriers such as lactose, dextrose, sucrose, mannitol, sorbitol, cellulose, and starch; diluents like water or other solvents; glidants and lubricants such as talc, magnesium stearate, and stearic acid; preservatives like methylparaben and propylparaben; buffering agents; chelating agents; polymers; gelling agents; viscosifying agents; and solvents. Auxiliary agents may also be included, such as wetting agents, suspending agents, sweetening agents, flavoring agents, colorants, and any combination thereof.

The pharmaceutical compositions can be prepared in various conventional forms, including tablets, capsules (soft or hard gelatin), dragees, troches, lozenges, solutions, suspensions, injectables, ointments, pastes, creams, lotions, powders, eye or ear drops, impregnated textiles, and products for topical application. These compositions may also be formulated to provide a desired release profile, such as immediate or controlled release.

In particular, the dosage forms for these compositions can take various conventional forms, including but not limited to: solid dosage forms, liquid dosage forms, injectable preparations, transdermal forms, rectal and vaginal formulations and powders and sprays.

Solid dosage forms can for example be selected among: tablets, capsules (soft or hard gelatin), dragees, troches, and lozenges. Tablets can be prepared by compression or molding techniques and may contain binders (e.g., povidone, gelatin), fillers or diluents (e.g., lactose, microcrystalline cellulose), lubricants (e.g., magnesium stearate), disintegrants (e.g., sodium starch glycolate), and preservatives. Capsules can be filled with the active compound in powder or pellet form along with appropriate excipients. The active compounds can also be formulated into microencapsulated forms with excipients. Solid dosage forms may have coatings such as enteric coatings or release-controlling coatings to modify the release profile of the active compound. Buffering agents may be included to maintain the stability of the active compound.

Liquid dosage forms can for example include emulsions, microemulsions, solutions, and suspensions designed for oral administration. These formulations may contain inert diluents like water, solubilizing agents, and emulsifiers such as ethyl alcohol, isopropyl alcohol, and various oils (e.g., cottonseed, olive, castor oils). Adjuvants like wetting agents, emulsifying and suspending agents, sweetening agents, flavoring agents, and perfuming agents may also be included

Injectable preparations such as sterile aqueous or oleaginous suspensions are generally formulated using dispersing, wetting, or suspending agents. Sterile injectable solutions, suspensions, or emulsions can be prepared in nontoxic, parenterally acceptable diluents or solvents like water, Ringer's solution, or isotonic sodium chloride solution. Fixed oils such as synthetic mono- or diglycerides and fatty acids like oleic acid may be used as solvents or suspending media.

Topical and transdermal forms comprise compositions such as ointments, pastes, creams, lotions, gels, solutions, sprays, and transdermal patches. These may be formulated with suitable fats, oils, waxes, or polymers to achieve desired topical or transdermal absorption rates. Patches may also employ rate-controlling membranes or polymer matrices to control the release of the active compounds.

Rectal and vaginal formulations may be in the form of suppositories or gels, incorporating the active compounds into bases such as cocoa butter, polyethylene glycol, or other suitable non-irritating materials.

Powders and Sprays, for topical or inhalation use, may be prepared as powders or aerosol sprays. Propellants like chlorofluorohydrocarbons may be used for spray formulations.

The formulation process for these compositions follows conventional pharmaceutical techniques, ensuring uniform mixing of the active compound with the chosen excipients. Manufacturing steps may include granulation, encapsulation, or coating, depending on the desired release characteristics of the final product. Controlled-release formulations can be achieved through the application of suitable coatings or matrix systems that modulate the release profile of the active ingredient.

### Administration and dosage

The compounds of the present invention, including their pharmaceutically acceptable salts, may be administered to patients using various routes, depending on the nature of the disease, the desired therapeutic effect, and the formulation of the pharmaceutical composition. The choice of administration route and dosage form ensures that the active compounds are delivered effectively to the intended site of action with an appropriate pharmacokinetic profile, taking into consideration factors like bioavailability, absorption rate, and patient compliance. Suitable routes of administration include oral, nasal, buccal, dermal, intradermal, transdermal, parenteral (including intravenous, intramuscular, and subcutaneous), rectal, intraurethral, and topical applications.

The compound described herein may be administered to a subject in an effective amount to achieve the desired therapeutic effect. The appropriate dosage and dosing regimen can vary based on several factors, including the activity of the compound, the route and timing of administration, the rate of excretion, the duration of treatment, any concurrent medications, the severity of the condition being treated, and characteristics of the patient such as species, sex, age, weight, general health, and medical history. Determining the optimal dosage involves balancing the therapeutic benefits against potential risks or side effects and is ultimately at the discretion of the treating physician.

Administration of the compound can be performed in a single dose or multiple doses, either continuously or intermittently throughout the course of treatment. The compound may be administered via any convenient route, whether systemically or at the site of desired action. Routes of administration include, but are not limited to, oral ingestion; topical application (such as transdermal, intranasal, ocular, buccal, and sublingual routes); pulmonary delivery via inhalation or insufflation therapy using an aerosol through the mouth or nose; rectal or vaginal administration; and parenteral routes, including various forms of injection such as subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intravitreal, and intrasternal injections. Additionally, the compound may be administered through the implantation of a depot, for example, subcutaneously, intramuscularly, or intravitreally.

The dosing frequency may vary depending on the needs of the patient and the judgment of the physician. A single dose may be administered hourly, daily, or weekly. For instance, administration may occur once every 1 hour, 2 hours, 3 hours, 4 hours, 6 hours, 8 hours, 12 hours, 16 hours, or once every 24 hours. Alternatively, the compound may be administered once every 1 day, 2 days, 3 days, 4 days, 5 days, 6 days, or once every 7 days. In some cases, the compound may be administered once every week, every two weeks, every three weeks, or once every four weeks. In certain embodiments, administration may occur once every month. The dosage and frequency may be adjusted over the course of treatment based on the patient's response and any side effects experienced.

Therapeutically effective amounts of the compound generally range from about 0.00001 mg/kg to about 10 mg/kg of body weight per day. More specific dosage ranges include from about 0.0001 mg/kg to about 10 mg/kg per day, from about 0.001 mg/kg to about 1 mg/kg per day, from about 0.01 mg/kg to about 1 mg/kg per day, and from about 0.05 mg/kg to about 0.5 mg/kg per day. Alternatively, the compound may be administered in doses ranging from about 0.01 mg to about 1000 mg per dose. Examples of therapeutically effective amounts include doses from about 0.01 mg to about 100 mg, from about 0.1 mg to about 100 mg, from about 1 mg to about 100 mg, from about 1 mg to about 50 mg, and from about 1 mg to about 10 mg per dose. Specific dose amounts may be about 1 mg, 2 mg, 3 mg, 4 mg, 5 mg, 6 mg, 7 mg, 8 mg, 9 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, or about 100 mg per dose. Higher doses may include about 100 mg, 125 mg, 150 mg, 175 mg, 200 mg, 225 mg, 250 mg, 275 mg, 300 mg, 350 mg, 400 mg, 450 mg, or about 500 mg per dose.

In specific embodiments, the compound may be administered to a human patient according to the following dosage regimens: about 5 mg or about 10 mg administered three or four times daily; about 20 mg or about 40 mg administered three or four times daily; about 50 mg or about 75 mg administered three or four times daily; about 100 mg or about 125 mg administered twice daily; about 100 mg administered three times daily; about 150 mg administered twice daily; or about 200 mg administered twice daily.

The duration of treatment may vary depending on the condition being treated and the patient's response. Treatment may be administered for at least about one week, at least about two weeks, at least about three weeks, one month, at least about two months, at least about three months, at least about six months, at least about twelve months, or potentially extending for the lifetime of the individual. The dosage or dosing frequency may be adjusted over the course of treatment based on the patient's response and the judgment of the administering physician.

In some cases, the active compounds of the present invention may be administered in combination with other therapeutic agents, such as chemotherapeutic drugs, immunotherapy agents, or radiation therapy, for a synergistic effect in treating diseases such as cancer. The combination therapy may be administered concurrently, sequentially, or as a fixed-dose combination, with careful consideration given to potential interactions and the pharmacokinetics of the combined agents.

### Methods and uses

The present invention relates to methods and uses concerning the treatment of diseases and disorders, for example hyperproliferative disorders, particularly cancer, by administering therapeutically effective amounts of compounds of Formula (I), (II), (Ila), (IIb), (IIb-1), (llc), (IIc-1), (IId), (IId-1), (IIe), (Ile-1), (Ile-2), (IIIa-1), (Illa-2), (Illb-1), (Illb-2), (IIIc-1), (IIIc-2), (IIId-1), (IIId-2), (Ille-1), (IIIe-2), (IVa), (IVb), (Va), (Vb), (VI) or their pharmaceutically acceptable salts. These compounds are valuable as medicaments for treating conditions mediated by KAT6A, a histone acetyltransferase implicated in cancer progression.

The pharmaceutical compositions of the present invention are intended for use as medicaments for example in the treatment of hyperproliferative disorders, in particular in cancer and related disorders. The active compounds may be formulated to provide a therapeutically effective amount sufficient to achieve the desired therapeutic benefit without causing unwanted side effects. Dosages and modes of administration can be determined and adjusted by healthcare professionals based on factors like the type and severity of the disease, patient characteristics, and preliminary evidence from clinical studies.

The compounds of the present invention effectively inhibit KAT6A activity, thereby modulating pathological processes associated with KAT6A-mediated diseases. Inhibition of KAT6A can suppress cancer cell growth and proliferation, making these compounds significant therapeutic agents in oncology.

Cancers that can be treated using these compounds encompass a wide range, including but not limited to brain gliomas, glioblastomas, astrocytomas, multiforme, Bannayan-Zonana syndrome, Cowden disease, Lhermitte-Duclos disease, breast cancer (such as estrogen receptor-positive [ER+] breast cancer, ER+ HER2- breast cancer, and locally advanced or metastatic ER+ HER2- breast cancer), colon cancer, head and neck cancer, kidney cancer, liver cancer, lung cancer (including non-small cell lung cancer and locally advanced or metastatic non-small cell lung cancer), bone cancer, colorectal cancer, germ cell cancer, melanoma, ovarian cancer, pancreatic cancer, adenocarcinomas (including ductal and adenosquamous carcinoma), acinar cell carcinoma, glucagonoma, insulinoma, prostate cancer (including castration-resistant forms and locally advanced or metastatic cases), sarcoma, thyroid cancer, various leukemias (such as lymphoblastic T cell leukemia, chronic myelogenous leukemia, chronic lymphocytic leukemia, hairy-cell leukemia, acute lymphoblastic leukemia, acute myelogenous leukemia, chronic neutrophilic leukemia, plasmacytoma, mantle cell leukemia, megakaryoblastic leukemia, multiple myeloma, promyelocytic leukemia, erythroleukemia), malignant lymphomas (including Hodgkin's and non-Hodgkin's lymphoma, Burkitt's lymphoma, follicular lymphoma), neuroblastoma, bladder cancer, urothelial cancer, vulval cancer, uterine cancer, cervical cancer, endometrial cancer, renal cancer, mesothelioma, esophageal cancer, salivary gland cancer, hepatocellular cancer, gastric cancer, nasopharyngeal cancer, buccal cancer, cancers of the mouth, gastrointestinal stromal tumors (GIST), neuroendocrine cancers, testicular cancer, and virus-related cancers.

Moreover, the compounds may be used in treating other disorders associated with abnormal cell growth, such as benign proliferative diseases including psoriasis, benign prostatic hypertrophy, or restenosis. They may also be applied in methods of treating disorders associated with angiogenesis, such as age-related macular degeneration, proliferative diabetic retinopathy, rheumatoid arthritis, osteoporosis, Paget's disease, humoral hypercalcemia of malignancy, coronary restenosis, and certain microbial infections.

In more preferred embodiment, the compounds of the invention are used in treating or preventing a disease selected among: breast cancer(such as estrogen receptor-positive [ER+] breast cancer, ER+ HER2- breast cancer, and locally advanced or metastatic ER+ HER2- breast cancer), prostate cancer, ovarian cancer, cervical cancer, lung adenocarcinoma, colon and rectal adenocarcinomas, medulloblastoma, glioblastoma and blood cancer such as acute myeloid leukaemia. In more preferred embodiment, the invention relates to a method for treating or prophylaxis of a disease selected from a KATs overexpressing cancer.

One embodiment of the invention provides a pharmaceutical composition comprising a compound of any of the aforementioned formulas, or a pharmaceutically acceptable salt thereof, for use in the treatment, management, or prophylaxis of a disease, disorder, or condition in a subject.

The subject is typically a human in need of such treatment. The diseases or disorders targeted include those mediated by KAT6A, with a particular focus on various forms of cancer.

One embodiment of the invention provides a method of treating, managing, or preventing a disease, disorder, or condition in a subject by administering a therapeutically effective amount of a compound of any of the aforementioned formulas, or a pharmaceutically acceptable salt thereof.

### Combination therapy

Compounds of Formula Formula (I), (II), (IIa), (IIb), (Ilb-1), (IIc), (IIc-1), (IId), (IId-1), (IIe), (IIe-1), (Ile-2), (Illa-1), (IIIa-2), (IIIb-1), (IIIb-2), (IIIc-1), (IIIc-2), (IIId-1), (IIId-2), (IIIe-1), (IIIe-2), (IVa), (IVb), (Va), (Vb), (VI) or their pharmaceutically acceptable salts described herein may be used in combination with other therapeutic agents for the treatment of diseases such as cancer. These compounds can be administered alongside additional therapeutic agents, including chemotherapeutic agents, biological agents, targeted therapies, or agents that alleviate symptoms associated with the disease. The combination therapy may involve administering the compounds and additional agents either in a single pharmaceutical formulation or in separate formulations, delivered simultaneously or sequentially.

The compounds may act synergistically with chemotherapy, radiotherapy, or targeted therapies, enhancing the overall therapeutic effect. For instance, they can be combined with fibroblast growth factor receptor 1 (FGFR1) inhibitors, nuclear hormone receptor-targeting therapies, or immune checkpoint inhibitors. Notably, the compounds may be used in conjunction with bromodomain and extraterminal domain (BET) inhibitors, which reversibly bind to the bromodomains of BET proteins BRD2, BRD3, BRD4, and BRDT.

Inhibition of histone acetyltransferase (KAT) proteins of the MYST family by these compounds reduces lysine acetylation of histones and other nuclear proteins. This action sensitizes tumor cells to chemotherapy and radiotherapy by attenuating DNA damage repair processes, such as the repair of DNA double-strand breaks, thereby increasing the efficacy of cancer cell eradication induced by these therapies. Consequently, the compounds are expected to combine effectively with low-dose chemotherapy or radiotherapy.

The compounds may be administered in conjunction with radiotherapeutic or chemotherapeutic regimens. Suitable chemotherapeutic agents include, but are not limited to, platinum compounds (e.g., cisplatin, carboplatin, oxaliplatin), alkylating agents (e.g., cyclophosphamide, ifosfamide), antitumor antibiotics (e.g., doxorubicin, bleomycin), taxanes (e.g., paclitaxel, docetaxel), antimetabolites (e.g., 5-fluorouracil, methotrexate), nucleoside analogues (e.g., fludarabine), and topoisomerase inhibitors (e.g., irinotecan). Suitable biological agents include monoclonal antibodies (e.g., rituximab, trastuzumab, bevacizumab), enzymes (e.g., L-asparaginase), cytokines (e.g., interferons, interleukins), growth factors, cancer vaccines, and gene therapy vectors.

In scenarios where the compounds are used to abrogate regulatory T cell (Treg) suppression, they may be combined with immune checkpoint inhibitors, such as those targeting PD-1, PD-L1, or CTLA-4, to enhance anti-tumor immunity. Additionally, combining these compounds with radiotherapy may further diminish Treg function within tumors, potentially improving therapeutic outcomes.

These treatment methods may be applied to subjects for whom other treatments have failed or have had limited success, such as those with cancers refractory to standard-of-care treatments. The compounds can be administered prior to, simultaneously with, or after other therapeutic modalities, including chemotherapy, surgery, hormone therapy, or radiation. The combinations described are illustrative and not limiting, and the compounds may be used with one or more additional agents to achieve the desired therapeutic effect.

Combination therapies may involve co-administering the compounds with estrogen receptor antagonists or partial antagonists, aromatase inhibitors (e.g., letrozole), Selective Estrogen Receptor Modulators (SERMs) such as tamoxifen, endoxifene, raloxifene, toremifene, lasofoxifene, ospemifene, elacestrant, or bazedoxifene, and Selective Estrogen Receptor Degraders (SERDs) like fulvestrant, camizestrant, palazestrant, imlunestrant, elacestrant, or giredestrant. Complete Estrogen Receptor Antagonists (CERANs) such as fulvestrant or palazestrant can also be used in combination.

Additionally, the compounds may be combined with other anti-cancer agents, including HER2 inhibitors (e.g., tucatinib, trastuzumab, pertuzumab, ado-trastuzumab emtansine, trastuzumab deruxtecan, lapatinib, neratinib), mTOR inhibitors (e.g., everolimus, sirolimus, temsirolimus, LY3023414), CDK4/6 inhibitors (e.g., palbociclib, abemaciclib, ribociclib, lerociclib, trilaciclib, SHR6390), CDK2 inhibitors (e.g., PF-07104091), CDK4-selective inhibitors (e.g., PF-07220060), PI3 kinase inhibitors (e.g., perifosine, CAL101, BEZ235, XL147, XL765, GDC-0941, IPI-145), PIK3CA inhibitors (e.g., alpelisib, taselisib, LY3023414, inavolisib, STX-478, RLY-2608, LOXO-783, OKI-219, TOS-358), aromatase inhibitors (e.g., aminoglutethimide, testolactone, anastrozole, letrozole, exemestane, vorozole, formestane, fadrozole, 4-hydroxyandrostenedione, 1,4,6-androstatrien-3,17-dione, 4-androstene-3,6,17-trione), antibodies or inhibitors targeting PD-1, PD-L1, or CTLA-4, and inhibitors of EGFR, PGFR, or IGFR (e.g., erlotinib, gefitinib). USP1 inhibitors and AKT inhibitors such as capivasertib are also suitable for combination therapy.

The examples and preparations presented herein further illustrate and exemplify the compounds described, along with the methods for their preparation. It should be noted that the scope of the embodiments described is not limited in any way by these examples and preparations.

In the following examples, unless otherwise specified, molecules with a single chiral center are provided as racemic mixtures. Molecules with two or more chiral centers are also presented as racemic mixtures of diastereomers unless stated otherwise. Methods for obtaining single enantiomers or diastereomers are well-known to those skilled in the art.

In light of the descriptions provided herein, the compounds described herein can be prepared by processes known in the chemical arts. Specific processes for the manufacture of these compounds are included as additional features of the embodiments and are illustrated in the reaction schemes provided below and in the experimental section.

### EXAMPLES

The following examples are provided to illustrate specific embodiments of the invention and are not intended to limit the scope thereof. Variations and modifications will become apparent to those skilled in the art upon reading this specification. The full scope of the invention should be determined by the claims and their equivalents.

### I. Synthesis of Sulfonamide Intermediates

### Intermediate INT_{A-1}: 2,6-dimethoxybenzenesulfonamide (Intermediate INT_{A-1})

### Scheme SCH_{INTA1}

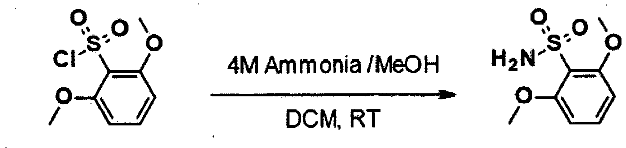

To a solution of 2,6-dimethoxybenzenesulfonyl chloride (2.62 g, 11.07 mmol) in DCM (25 mL) at room temperature was added 4M ammonia solution in MeOH (27.68 mL, 110.7 mmol). The reaction mixture was stirred at room temperature for 30 min. The solvent was removed under reduced pressure and water was added. The reaction mixture was filtered and the resulting solid was washed with acetonitrile. The solid was suspended in dry toluene and evaporated to dryness under reduced pressure to afford 2,6-dimethoxybenzenesulfonamide **(Intermediate INT_{A-1})** (1.80 g, 74 % yield) as a white solid. LCMS (ES, m/z): 218.1 [M+H]⁺.

### Intermediate INT_{A-2}: 2-methoxybenzenesulfonamide (Intermediate INT_{A-2})

### Scheme SCH_{INTA2}.

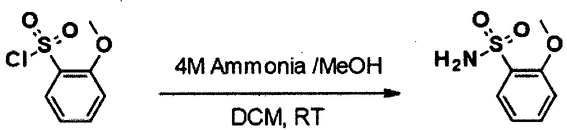

To a mixture of 2-methoxybenzenesulfonyl chloride (3.00 g, 14.51 mmol) in DCM (20 mL) at room temperature was added 4M ammonia solution in MeOH (18.14 mL, 72.59 mmol). The reaction mixture was stirred at room temperature for 16 h. The precipitate was filtered and washed with DCM. Water was added to the filtrate. After separation, the organic layer was concentrated under reduced pressure. The residue was purified by silica gel column chromatography eluted with cyclohexane/(ethyl acetate/ethanol 3:1) as eluent from 100/0 to 0/100 to afford 2-methoxybenzenesulfonamide **(Intermediate INT_{A-2})** (2.8 g, 98 % yield) as a beige solid. LCMS (ES, m/z): 188.2 [M+H]⁺.

### Intermediate INT_{A-3}: 2-methoxy-5-(trifluoromethyl)benzenesulfonamide (Intermediate INT_{A-3})

### Scheme SCH _{INTA3}.

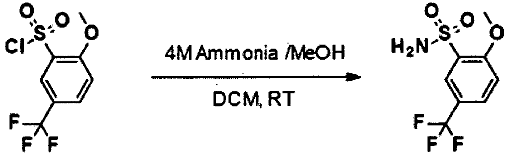

To a mixture of 2-methoxy-5-(trifluoromethyl)benzenesulfonyl chloride (2.50 g, 9.10 mmol) in DCM (30 mL) at room temperature was added 4M ammonia solution in MeOH (22.8 mL, 91 mmol). The reaction mixture was stirred at room temperature for 1 h. The reaction mixture was concentrated to dryness under reduced pressure then diluted with water. The suspension was stirred for 10 min then filtered and dried to afford 2-methoxy-5-(trifluoromethyl)benzenesulfonamide **(Intermediate INT_{A-3})** (2.0 g, 82 % yield) as a white solid. LCMS (ES, m/z): 255.0 [M-H]⁺.

### Intermediate INT_{A-4}: 5-(3-fluoroazetidin-1-yl)-2-methoxybenzenesulfonamide TFA salt (Intermediate INT_{A-4})

### Scheme SCH_{INTA4}

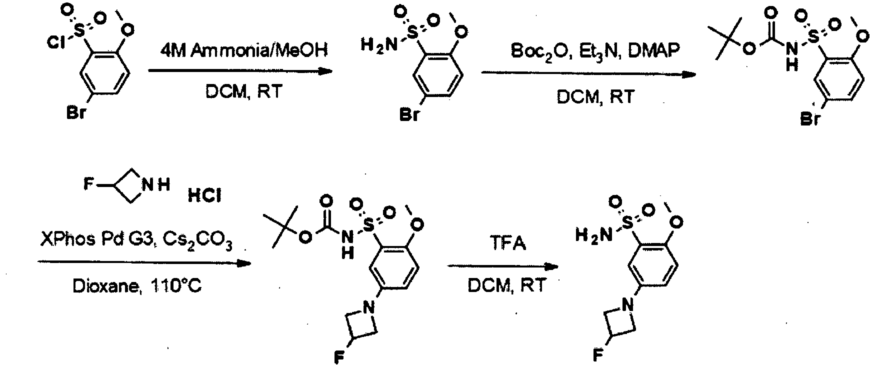

**Step 1:** To a mixture of 5-bromo-2-methoxybenzenesulfonyl chloride (4.83 g, 16.9 mmol) in DCM (84.6 mL) at room temperature was added 4M ammonia solution in MeOH (42.3 mL, 169 mmol). The reaction mixture was stirred at room temperature for 30 min. The reaction mixture was concentrated under reduced pressure then diluted with water. The suspension was stirred for 10 min then filtered and dried to afford 5-bromo-2-methoxybenzenesulfonamide (4.41 g, 93 % yield) as a white solid. LCMS (ES, m/z): 266.1-268.1 [M+H]⁺.

**Step 2:** To a solution of 5-bromo-2-methoxybenzenesulfonamide (425 mg, 1.59 mmol) in DCM (15.9 mL) was added Et₃N (668 µL, 4.79 mmol) and Boc₂O (440 µL, 1.91 mmol). The reaction mixture was stirred at room temperature for 16 h. Water was added and the layers were separated. The organic one was washed with brine, dried over anhydrous sodium sulfate, filtered and concentrated under reduced pressure to afford *tert-*butyl ((5-bromo-2-methoxyphenyl)sulfonyl)carbamate (810 mg, Qte yield) as a clear oil which was engaged in the next step without further purification. LCMS (ES, m/z): 364.1-366.1 [M-H]⁺.

**Step 3:** A mixture of *tert*-butyl ((5-bromo-2-methoxyphenyl)sulfonyl)carbamate (585 mg, 1.59 mmol), 3-fluoroazetidine hydrochloride (712 mg, 6.38 mmol) and cesium carbonate (4.16 g, 12.7 mmol) in dioxane (15.9 mL) at room temperature was degassed with argon for 5 min. XPhos Pd G3 (202 mg, 239 µmol) was added. The reaction mixture was stirred at 110 °C for 6 h under MW irradiations. The reaction mixture was filtered, a saturated NH₄Cl aqueous solution and ethyl acetate were added and the resulting suspension was stirred for 30 min. The pH of the aqueous phase was adjusted to 2 by addition of 1N HCl aqueous solution. After separation, the aqueous phase was extracted with ethyl acetate. The combined organic phases were dried over anhydrous sodium sulfate, filtered and concentrated under pressure. The residue was purified by silica gel column chromatography eluting with cyclohexane/ethyl acetate from 100/0 to 50/50 to afford tert-butyl ((5-(3-fluoroazetidin-1-yl)-2-methoxyphenyl)sulfonyl)carbamate (400 mg, 69.5 % yield) as a yellowish oil. LCMS (ES, m/z): 361.2 [M+H]⁺.

Step 4: To a mixture of tert-butyl ((5-(3-fluoroazetidin-1-yl)-2-methoxyphenyl) sulfonyl)carbamate (150 mg, 416 µmol) in DCM (8.3 mL) at 0°C was added TFA (274 µL, 4.16 mmol) portion wise. The reaction mixture was stirred at room temperature for 16 min. The reaction mixture was concentrated under reduced pressure and dried to afford 5-(3-fluoroazetidin-1-yl)-2-methoxybenzenesulfonamide TFA salt **(Intermediate INT_{A-4})** (185 mg, Qte yield) as a brown oil which was used in the next step without purification. LCMS (ES, m/z): 261.2 [M+H]⁺.

### Intermediate INT_{A-5}: 2-chloro-6-methoxyquinoline-7-sulfonamide (Intermediate INT_{A-5})

### Scheme SCH_{INTA5}

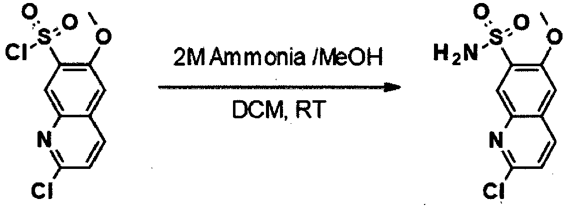

To a mixture of 2-chloro-6-methoxyquinoline-7-sulfonyl chloride (1.0 g, 3.42 mmol) in DCM (17.1 mL) at room temperature was added 2M ammonia solution in MeOH (17.12 mL, 34.23 mmol). The reaction mixture was stirred at room temperature for 6 h. The reaction mixture was concentrated under reduced pressure. The residue was purified by reverse phase C₁₈ column chromatography eluted with water/acetonitrile from 100/0 to 25/75 to afford 2-chloro-6-methoxyquinoline-7-sulfonamide **(Intermediate INT_{A-5})** (920 mg, 98 % yield) as a white solid. LCMS (ES, m/z): 273.0 [M+H]⁺.

### Intermediate INT_{A-6}: 6-methoxyquinoline-7-sulfonamide (Intermediate INT_{A-6})

### Scheme SCH_{INTA6}.

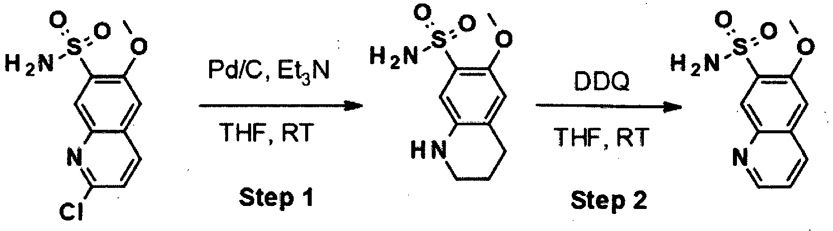

**Step 1:** To a solution of 2-chloro-6-methoxyquinoline-7-sulfonamide **(Intermediate INT_{A-5})** (410 mg, 1.28 mmol) in THF (12.8 mL) at room temperature under argon was added Pd/C 10% (136 mg, 128 µmol). The reaction mixture was stirred at room temperature for 16 h under hydrogen atmosphere. The reaction mixture was filtered over a pad of Celite. The filtrate was diluted in ethyl acetate and water. After separation, the organic phase was washed with brine, dried over anhydrous magnesium sulfate, filtered and concentrated under reduced pressure. The residue was triturated with DCM, filtered and dried to afford 6-methoxy-1,2,3,4-tetrahydroquinoline-7-sulfonamide (50 mg, 15 % yield) as a grey solid. LCMS (ES, m/z): 243.2 [M+H]⁺.

**Step 2:** To a solution of 6-methoxy-1,2,3,4-tetrahydroquinoline-7-sulfonamide (130 mg, 537 µmol) in THF (5.4 mL) at room temperature was added DDQ (244 mg, 1.07 mmol). The reaction mixture was stirred at room temperature for 16 h. The reaction mixture was filtered over a pad of Celite. The filtrate was concentrated under reduced pressure. The residue was purified by silica gel column chromatography eluted with DCM/MeOH from 100/0 to 97/3. The resulting compound was triturated with a mixture of DCM/MeOH, filtered and dried to afford 6-methoxyquinoline-7-sulfonamide (50 mg, 37 % yield) as a white solid. LCMS (ES, m/z): 239.1 [M+H]⁺.

### Intermediate INT_{A-7}: 2,3-dihydrobenzofuran-7-sulfonamide (Intermediate INT_{A-7})

### Scheme SCH_{INTA7}.

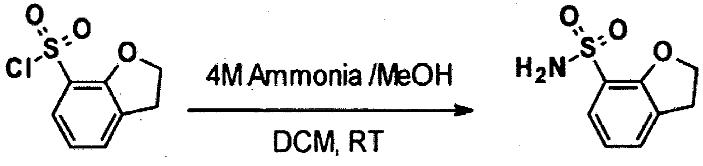

To a mixture of 2,3-dihydrobenzofuran-7-sulfonyl chloride (2.00 g, 9.15 mmol) in DCM (15 mL) at room temperature was added 4M ammonia solution in MeOH (22.9 mL, 91.5 mmol). The reaction mixture was stirred at room temperature for 30 min. The crude mixture was purified by reverse phase C18 column chromatography eluted with H2O/acetonitrile from 100/0 to 20/80 to afford 2,3-dihydrobenzofuran-7-sulfonamide (Intermediate INT4) (1.46 g, 80 % yield) as a white solid. LCMS (ES, m/z): 200.1 [M+H]⁺.

### Intermediate INT_{A-8}: 5-tert-butyl-2-methoxy-benzenesulfonamide (Intermediate INT_{A-}8)

### Scheme SCH_{INTA8}.

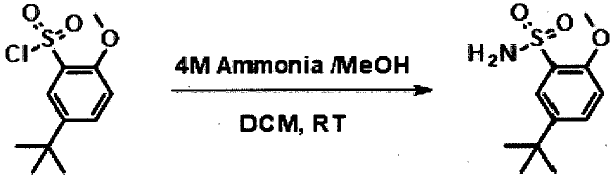

To a mixture of 5-(tert-butyl)-2-methoxybenzenesulfonyl chloride (2.00 g, 7.65 mmol) in DCM (30 mL) at 20°C, was added 4M ammonia solution in MeOH (19.12 mL, 76.46 mmol). The reaction mixture was stirred at 20°C for 1h. The crude mixture was filtered and the filtrate was concentrated to dryness to afford 5-(tert-butyl)-2-methoxybenzenesulfonamide **(Intermediate INT_{A-8})** (1.95 g, 95 % yield,) as a white solid. LCMS (ES, m/z): 244.1 [M+H]⁺.

### Intermediates INT_{A-1} to INT_{A-12}

Intermediates INT_{A-1} to INT_{A-12}, as listed in Table 2, were synthesized by methods analogous to those described above for Intermediates INT_{A-1}, INT_{A-2}, INT_{A-3}, INT_{A-4}, INT_{A-5} INT_{A-6} and INT_{A-8} utilizing suitable starting materials and standard procedures familiar to those skilled in the art. In some cases, commercially available sulfonyl chloride building blocks were employed directly.

**Table 2. Intermediates A**

| **Intermediate** | **Structure** |
|---|---|
| **INT_{A-1}** | 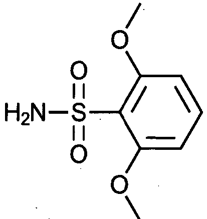 |
| **INT_{A-2}** | 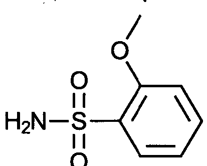 |
| **INT**_{**A**-**3**} | 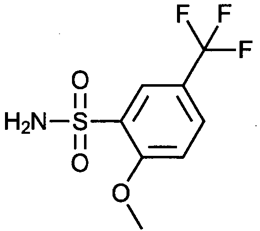 |
| **INT_{A-4}** | 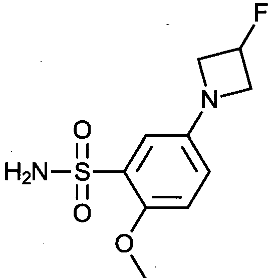 |
| **INT_{A-5}** | 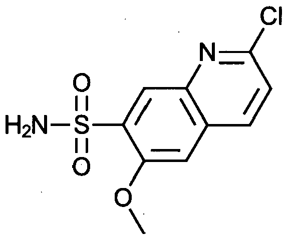 |
| **INT_{A-6}** | 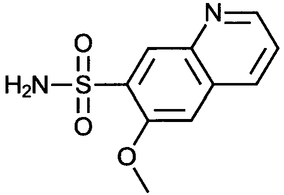 |
| **INT_{A-7}** | 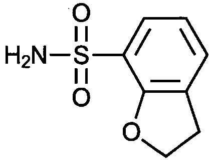 |
| **INT_{A-8}** | 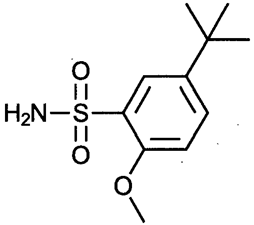 |
| **INT**_{**A**-**9**} | 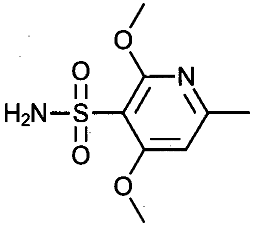 |
| **INT_{A-10}** | 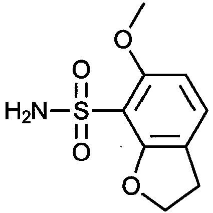 |
| **INT_{A11}** | 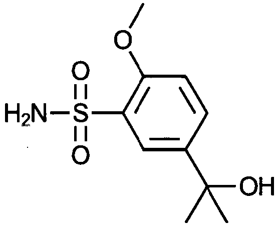 |
| **INT_{A-12}** | 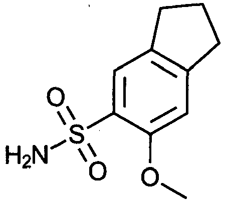 |

### II. Synthesis Benzoic Acid Intermediates

### Intermediate INT_{B-0}: 1-(methylsulfonyl)-1H-pyrazole (Intermediate INT_{B-0}) Scheme SCH_{INTB0}.

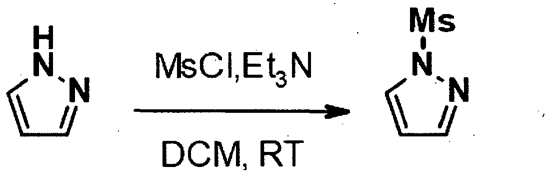

To a solution of 1H-pyrazole (1.50 g, 22.03 mmol) in DCM (60 mL) under argon at 0°C was added triethylamine (3.07 mL, 22.03 mmol) followed by the addition of methanesulfonyl chloride (1.71 mL, 22.03 mmol). The reaction was allowed to warm at room temperature and stirred for 16 h. The reaction mixture was quenched with saturated NH₄Cl aqueous solution and diluted with water. Organic layer was successively washed with water and brine, dried over anhydrous sodium sulfate, filtered and passed through SPE NH₂ column to afford 1-(methylsulfonyl)-1H-pyrazole (**Intermediate INT_{B-0})** (3.0 g, 93 % yield) as a colorless oil. LCMS (ES, m/z): 147.0 [M+H]⁺.

### Intermediate INT_{B-1}: 5-(1H-pyrazol-1-yl)-5,6,7,8-tetrahydronaphthalene-2-carboxylic acid (Intermediate INT_{B-1}) Scheme SCH_{1NTB1}.

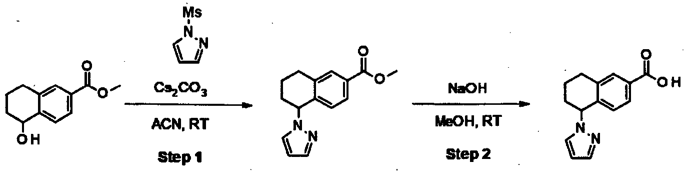

**Step** 1: To a mixture of methyl 5-hydroxy-5,6,7,8-tetrahydronaphthalene-2-carboxylate (1.0 g, 4.84 mmol) in acetonitrile (20 mL) at room temperature was added 1-(methylsulfonyl)-1H-pyrazole **(Intermediate INT_{B-0})** (850 mg, 5.81 mmol) then Cs₂CO₃ (4.73 g, 14.5 mmol). The reaction mixture was stirred at room temperature for 16 h. The reaction mixture was diluted with water and extracted with ethyl acetate. The combined organic layers were washed with brine, dried over anhydrous sodium sulfate, filtered and concentrated under reduced pressure. The residue was purified by silica gel column chromatography eluted with cyclohexane/ethyl acetate from 100/0 to 75/25 to afford methyl 5-(1H-pyrazol-1-yl)-5,6,7,8-tetrahydronaphthalene-2-carboxylate (870 mg, 67 % yield) as a colorless oil. LCMS (ES, m/z): 257.3 [M+H]⁺.

**Step 2:** To a mixture of methyl 5-(1H-pyrazol-1-yl)-5,6,7,8-tetrahydronaphthalene-2-carboxylate (850 mg, 3.32 mmol) in methanol (10 mL) was added a 1N NaOH aqueous solution (4.97 mL, 4.97 mmol) was stirred at room temperature for 2 h. The solvent was removed under reduced pressure and the crude was diluted with water (20 mL) and acidified with 1N HCl aqueous solution until pH=1. The resulting precipitate was filtered and dried to afford 5-(1H-pyrazol-1-yl)-5,6,7,8-tetrahydronaphthalene-2-carboxylic acid **(Intermediate INT_{B-1})** (590 mg, 70 % yield) as a yellow solid. LCMS (ES, m/z): 243.3 [M+H]⁺.

### Intermediate INT_{B-2}: 5-(1H-pyrazol-1-yl)-2-naphthoic acid (Intermediate INT_{B-2}) Scheme SCH_{INTB2}

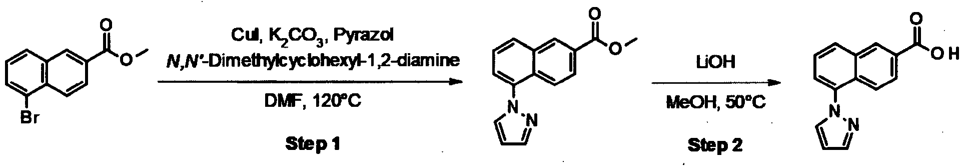

**Step 1:** A mixture of 1H-pyrazole (555 mg, 8.15 mmol), methyl 5-bromo-2-naphthoate (1.08 g, 4.07 mmol), potassium carbonate (1.69 g, 12.2 mmol) and *N,N*'-dimethyl-1,2-cyclohexanediamine (579 mg, 4.07 mmol) in DMF (8 mL) was degassed with argon for 5 min then copper(I) iodide (776 mg, 4.07 mmol) was added and the reaction mixture was stirred at 120 °C for 48 h. The reaction mixture was filtered over a Celite pad and the filtrate was concentrated under reduced pressure to afford methyl 5-(1H-pyrazol-1-yl)-2-naphthoate (3.3 g, 100 % yield) which was directly engaged in the next step without further purification. LCMS (ES, m/z): 253.1 [M+H]⁺.

**Step 2:** To a mixture of methyl 5-(1H-pyrazol-1-yl)-2-naphthoate (3.30 g, 4.32 mmol) in methanol (20 mL) at room temperature was added LiOH (310 mg, 13.0 mmol). The reaction mixture was stirred at 50 °C for 16 h. The solvent was removed under reduced pressure. The residue was dissolved in water and ethyl acetate. After separation, the aqueous layer was acidified with 1N HCl aqueous solution until pH=1 then extracted with ethyl acetate. The organic layer was washed with brine, dried over anhydrous sodium sulfate, filtered and concentrated under reduced pressure. The residue was purified by silica gel column chromatography eluted with dichloromethane/methanol from 100/0 to 95/5. The resulting product was triturated in acetonitrile, filtered and dried to afford 5-(1H-pyrazol-1-yl)-2-naphthoic acid **(Intermediate INT_{B-2}**) (333 mg, 29 % yield) as a white solid. LCMS (ES, m/z): 239.1 [M+H]⁺.

### Intermediate INT_{B-3}: 5-(1H-pyrazol-1-yl)quinoline-2-carboxamide (Intermediate INT_{B-3}) Scheme SCH_{INTB3}.

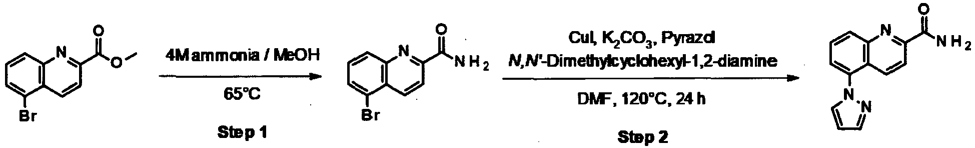

**Step 1:** A mixture of methyl 5-bromoquinoline-2-carboxylate (2.0 g, 7.51 mmol) in 4M ammonia solution in methanol (8.2 mL, 375 mmol) was stirred at 65 °C for 4 h in a sealed tube. The solvent was removed under reduced pressure and the residue was triturated in water (50 mL), filtered and dried to afford 5-bromoquinoline-2-carboxamide (1.60 g, 81 % yield) as an orange powder. LCMS (ES, m/z): 251.0 [M+H]⁺.

**Step 2:** A mixture of 1H-pyrazole (542 mg, 7.96 mmol) 5-bromoquinoline-2-carboxamide (1.0 g, 3.98 mmol), potassium carbonate (2.20 g, 15.9 mmol), *N,N'-*bismethyl-1,2-cyclohexanediamine (566 mg, 3.98 mmol) in DMF (30 mL) was degassed in argon for 5 min then copper(I) iodide (758 mg, 3.98 mmol) was added and the reaction mixture was stirred at 120 °C for 24 h. The reaction mixture was filtered. Water was added and the resulting precipitate was filtered, rinsed with ethyl acetate. Saturated NH₄Cl aqueous solution and ethyl acetate were added. After separation, the aqueous layer was extracted with ethyl acetate and the combined organic layers were washed with brine, dried over anhydrous sodium sulfate, filtered and concentrated under reduced pressure. The residue was purified by silica gel column chromatography eluted with cyclohexane/(ethyl acetate/ethanol 3/1) from 100/0 to 50/50 to afford 5-(1H-pyrazol-1-yl)quinoline-2-carboxamide **(Intermediate INT_{B-3})** (600 mg, 60 % yield) as a yellow powder. LCMS (ES, m/z): 239.1 [M+H]⁺.

### Intermediate INT_{B-4}: 5-(1H-pyrazol-1-yl)-5,6,7,8-tetrahydroquinoline-2-carboxamide (Intermediate INT_{B-4}) Scheme SCH_{INTB4}

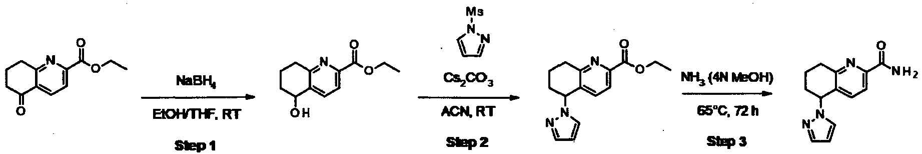

**Step 1:** To a mixture of ethyl 5-oxo-5,6,7,8-tetrahydroquinoline-2-carboxylate (500 mg, 2.28 mmol) in ethanol (10 mL) and THF (10 mL) at room temperature was added NaBH₄ (104 mg, 2.74 mmol). The reaction mixture was stirred at room temperature for 16 h then diluted with water (50 mL) and ethyl acetate (50 mL). The organic layer was washed with water. The combined aqueous layers were acidified until pH=7 with 1N HCl aqueous solution (2.7 mL) then extracted with ethyl acetate. The combined organic layers were washed with brine, dried over anhydrous sodium sulfate, filtered and concentrated under reduced pressure to afford ethyl 5-hydroxy-5,6,7,8-tetrahydroquinoline-2-carboxylate (390 mg, 73 % yield) as a colorless oil. LCMS (ES, m/z): 222.2 [M+H]⁺.

**Step** 2: To a mixture of ethyl 5-hydroxy-5,6,7,8-tetrahydroquinoline-2-carboxylate (380 mg, 1.72 mmol) in ACN (20 mL) at room temperature was added 1-(methylsulfonyl)-1H-pyrazole **(Intermediate INT_{B-0})** (251 mg, 1.72 mmol) and Cs₂CO₃ (1.12 g, 3.43 mmol). The reaction mixture was stirred at room temperature for 48 h. The reaction mixture was concentrated under reduced pressure then partitioned between water (200 mL) and ethyl acetate (200 mL). After separation, the organic layer was washed with water then brine, dried over anhydrous sodium sulfate, filtered and concentrated under reduced pressure. The residue was purified by silica gel column chromatography eluted cyclohexane/(ethyl acetate/ethanol 3/1) from 100/0 to 0/100 to afford ethyl 5-(1H-pyrazol-1-yl)-5,6,7,8-tetrahydroquinoline-2-carboxylate (120 mg, 19 % yield) as a colorless oil. LCMS (ES, m/z): 272.2 [M+H]⁺.

**Step 3:** A mixture of ethyl 5-(1H-pyrazol-1-yl)-5,6,7,8-tetrahydroquinoline-2-carboxylate (130 mg, 479 µmol) in 4M ammonia solution in methanol (408 mg, 24.0 mmol) was stirred at 65 °C for 72 h in a sealed tube. The solvent was removed under reduced pressure and the residue was triturated in water, filtered and dried to afford 5-(1H-pyrazol-1-yl)-5,6,7,8-tetrahydroquinoline-2-carboxamide **(Intermediate INT_{B-4})** (120 mg, 100 % yield) as a beige powder. LCMS (ES, m/z): 243.2 [M+H]⁺.

### Intermediate INT_{B-5}: 7-bromo-4-(1H-pyrazol-1-yl)-3,4-dihydro-2H-pyrano[2,3-b]pyridine (Intermediate INT_{B-5}) Scheme SCH_{INTB5}

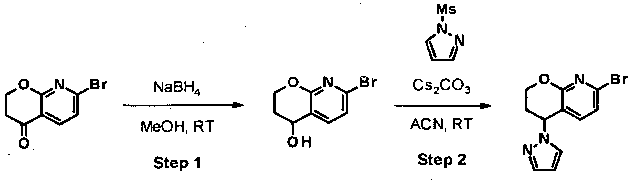

**Step 1:** To a mixture of 7-bromo-2,3-dihydro-4H-pyrano[2,3-b]pyridin-4-one (1.00 g, 4.39 mmol) in methanol (22 mL) was added NaBH₄ (199 mg, 5.26 mmol). The reaction mixture was stirred at room temperature for 2 h. The solvent was removed under reduced pressure. The crude material was dissolved in dichloromethane and water. After separation, the organic layer was dried over anhydrous sodium sulfate, filtered and concentrated under reduced pressure to afford 7-bromo-3,4-dihydro-2H-pyrano[2,3-b]pyridin-4-ol (957 mg, 90% yield) as an orange oil. LCMS (ES, m/z): 230.0 [M+H]⁺.

**Step 2:** To a mixture of 7-bromo-3,4-dihydro-2H-pyrano[2,3-b]pyridin-4-ol (950 mg, 4.13 mmol) in acetonitrile (21 mL) at room temperature was added 1-(methylsulfonyl)-1H-pyrazole **(Intermediate INT_{B-0})** (905 mg, 6.19 mmol) and Cs₂CO₃ (4.04 g, 12.4 mmol). The reaction mixture was stirred at 90 °C for 16 h. The reaction mixture was filtered on a Celite pad and the solvent was removed under reduced pressure. The residue was purified by silica gel column chromatography eluted with cyclohexane/ethyl acetate from 100/0 to 50/50 to afford 7-bromo-4-(1H-pyrazol-1-yl)-3,4-dihydro-2H-pyrano[2,3-b]pyridine **(Intermediate INT_{B-5})** (660 mg, 54 % yield) as a yellow solid. LCMS (ES, m/z): 280.1 [M+H]⁺.

### Intermediate INT_{B-6}: 8-methoxy-5-(1H-pyrazol-1-yl)-1,2,3,4-tetrahydronaphthalene-2-carboxylic acid (Intermediate INT_{B-6}) Scheme SCH_{INTB6}

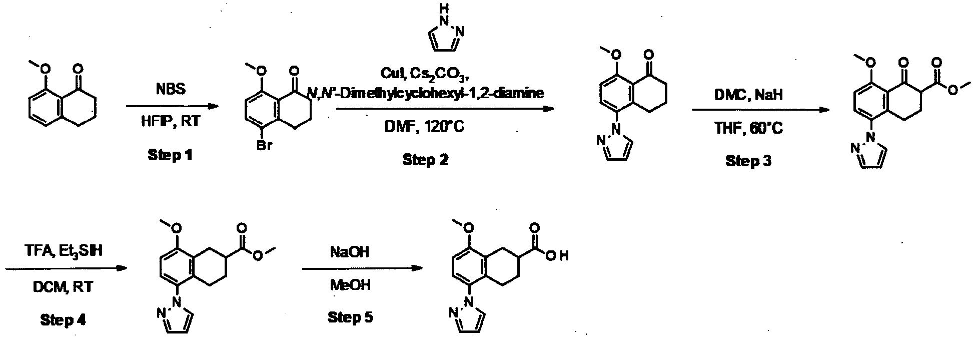

**Step 1:** To a mixture of 8-methoxy-3,4-dihydronaphthalen-1(2H)-one (1.00 g, 5.68 mmol) in HFIP (28 mL) at room temperature was added NBS (1.11 g, 6.24 mmol). The reaction mixture was stirred at room temperature for 30 min. The crude mixture was purified by silica gel column chromatography eluted with cyclohexane/ethyl acetate from 100/0 to 50/50 to afford 5-bromo-8-methoxy-3,4-dihydronaphthalen-1(2H)-one (1.63 g, 93% yield) as a yellow oil. LCMS (ES, m/z): 255.1 [M+H]⁺.

**Step 2:** A mixture of 1H-pyrazole (801 mg, 11.8 mmol), 5-bromo-8-methoxy-3,4-dihydronaphthalen-1(2H)-one (1.50 g, 5.88 mmol), Cs₂CO₃ (5.75 g, 17.6 mmol), *N,N'-*bismethyl-1,2-cyclohexanediamine (836 mg, 5.88 mmol) in DMF (29 mL) was degassed with argon for 5 min then copper(I) iodide (1.12 g, 5.88 mmol) was added. The reaction mixture was stirred at 120 °C for 48 h. The reaction mixture was diluted with ethyl acetate and saturated NH₄Cl aqueous solution then filtered over a Celite pad. After separation, the combined organic layers were washed with brine, dried over anhydrous sodium sulfate, filtered and concentrated under reduced pressure. The residue was purified by silica gel column chromatography eluted with cyclohexane/ethyl acetate from 100/0 to 50/50 to afford 8-methoxy-5-(1H-pyrazol-1-yl)-3,4-dihydronaphthalen-1(2H)-one (424 mg, 28 % yield) as a red solid. LCMS (ES, m/z): 243.2 [M+H]⁺.

**Step 3:** To a mixture of sodium hydride (60% dispersion in mineral oil) (86.6 mg, 2.16 mmol) in THF (4 mL) was added 8-methoxy-5-(1H-pyrazol-1-yl)-3,4-dihydronaphthalen-1(2H)-one (350 mg, 1.44 mmol) in THF (4 mL) followed by dimethyl carbonate (146 µL, 1.73 mmol). The reaction mixture was stirred 60 °C at 16 h. The reaction mixture was dissolved in ethyl acetate and 1N HCl aqueous solution. After separation, the organic layer was dried over anhydrous sodium sulfate, filtered and concentrated under reduced pressure. The residue was purified by silica gel column chromatography eluted with cyclohexane/ethyl acetate from 100/0 to 50/50 to afford methyl 8-methoxy-1-oxo-5-(1H-pyrazol-1-yl)-1,2,3,4-tetrahydronaphthalene-2-carboxylate (323 mg, 71 % yield) as orange oil. LCMS (ES, m/z): 301.1 [M+H]⁺.

**Step 4:** To a mixture of methyl 8-methoxy-1-oxo-5-(1H-pyrazol-1-yl)-1,2,3,4-tetrahydronaphthalene-2-carboxylate (270 mg, 899 µmol) in dichloromethane (4.5 mL) and TFA (4.5 mL) at room temperature was added triethylsilane (359 µL, 2.25 mmol). The reaction mixture was stirred at room temperature for 16 h. The crude material was dissolved in ethyl acetate and NaHCO₃ aqueous solution. After separation, the organic layer was dried over anhydrous sodium sulfate, filtered and concentrated under reduced pressure. The residue was purified by silica gel column chromatography eluted with cyclohexane/ethyl acetate from 100/0 to 50/50 to afford methyl 8-methoxy-5-(1H-pyrazol-1-yl)-1,2,3,4-tetrahydronaphthalene-2-carboxylate (200 mg, 47 % yield) as a yellow oil. LCMS (ES, m/z): 287.2 [M+H]⁺.

**Step 5:** To a mixture of methyl 8-methoxy-5-(1H-pyrazol-1-yl)-1,2,3,4-tetrahydronaphthalene-2-carboxylate (145 mg, 506 µmol) in methanol (5 mL) was added 1N NaOH aqueous solution (608 µL, 608 µmol). The reaction mixture was stirred at room temperature for 16 h. The solvent was evaporated under reduced pressure. The crude material was dissolved in water and 1N HCl aqueous solution. The resulting precipitate was filtered and dried under reduced pressure to afford 8-methoxy-5-(1H-pyrazol-1-yl)-1,2,3,4-tetrahydronaphthalene-2-carboxylic acid **(Intermediate INT_{B-6})** (125 mg, 86 % yield) as a white solid. LCMS (ES, m/z): 273.1 [M+H]⁺.

### Intermediates INT_{B-1} to INT_{B-6}

The following intermediates from Intermediate **INT_{B-1}** to Intermediate **INT_{B-6}** listed in Table 3 were prepared by following similar procedures described above for Intermediate **INT_{B-1}** and **INT_{B-6}** using appropriate reagents with suitable modifications known to the one skilled in the art.

**Table 3. Intermediates B**

| **Intermediate** | **Structure** |
|---|---|
| **INT_{B-1}** | 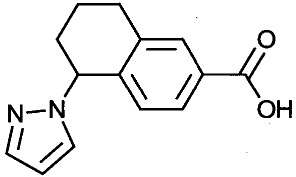 |
| **INTB-2** | 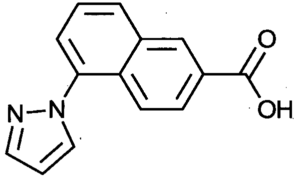 |
| **INT_{B-3}** | 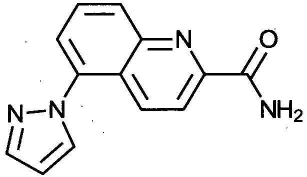 |
| **INT_{B-4}** | 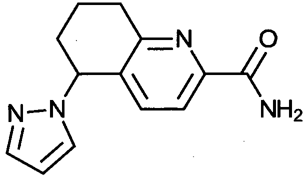 |
| **INT_{B-5}** | 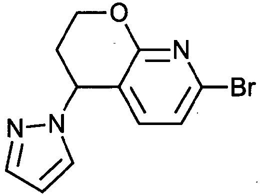 |
| **INT_{B-6}** | 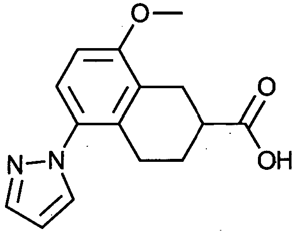 |

Compounds of the present invention can be synthesized following the processes outlined here after.

### Example EXA₁: N-((2-methoxyphenyl)sulfonyl)-5-(1H-pyrazol-1-yl)-5,6,7,8-tetrahydro naphthalene-2-carboxamide (Example EXA₁ - Compound 1) Scheme SCH_{EXA1}.

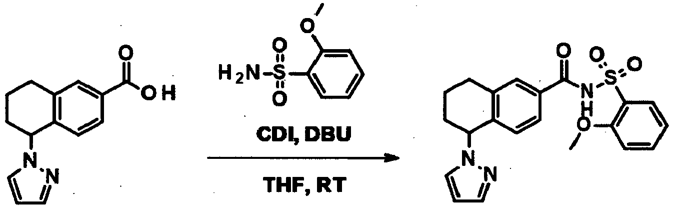

To a mixture of 5-(1H-pyrazol-1-yl)-5,6,7,8-tetrahydronaphthalene-2-carboxylic acid (**Intermediate INT_{B-1}**) (100 mg, 413 µmol) in THF (2 mL) at room temperature was added CDI (87 mg, 537 µmol). The reaction mixture was stirred at room temperature under argon for 30 min. Then 2-methoxybenzenesulfonamide (**Intermediate INT_{A-2}**) (85 mg, 454 µmol) and DBU (189 mg, 1.24 mmol) were added. The reaction mixture was stirred at room temperature under argon for 16 h. The crude material was dissolved in ethyl acetate and saturated NH₄Cl aqueous solution. After separation, the organic layer was dried over anhydrous sodium sulfate, filtered and concentrated under reduced pressure. The residue was purified by reverse phase C₁₈ column chromatography eluted H₂O/ACN 100/0 to 00/100 to afford *N*-((2-methoxyphenyl)sulfonyl)-5-(1H-pyrazol-1-yl)-5,6,7,8-tetrahydronaphthalene-2-carboxamide (**Example EXA₁** - **Compound 1**) (9.3 mg, 5.2 % yield) as a white solid. LCMS (ES, m/z): 412.3 [M+H]⁺.
¹H NMR (400 MHz, DMSO-d6) δ 7.80-7.74 (m, 1H), 7.72 (s, 1H), 7.62-7.57 (m, 1H), 7.54 (dd, J = 7.66, 1.66 Hz, 1H), 7.46 (dd, J = 1.70, 0.54 Hz, 1H), 7.39- 7.31 (m, 1H), 7.01-6.89 (m, 2H), 6.61 (d, J = 8.24 Hz, 1H), 6.24 (t, J = 1.93 Hz, 1H), 5.62-5.56 (m, 1H), 3.73-3.66 (br. s, 3H), 2.96-2.75 (m, 2H), 2.27-2.08 (m, 2H), 1.95-1.72 (m, 2H).

### Example EXA₂: N-((2,6-dimethoxyphenyl)sulfonyl)-5-(1H-pyrazol-1-yl)-5,6,7,8 tetrahydronaphthalene-2-carboxamide (Example EXA₂ - Compound 2)

### Scheme SCH_{EXA2}.

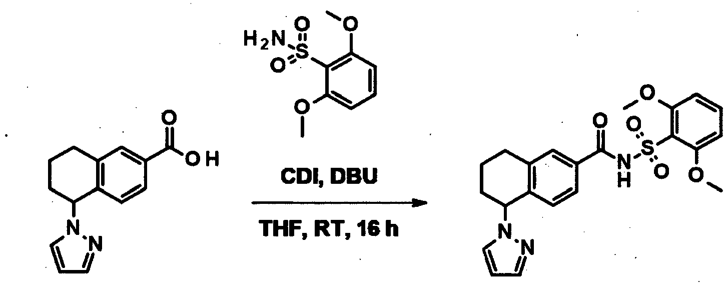

To a mixture of 5-(1H-pyrazol-1-yl)-5,6,7,8-tetrahydronaphthalene-2-carboxylic acid (**Intermediate INT_{B-1}**) (100 mg, 413 µmol) in THF (2 mL) at room temperature was added CDI (87 mg, 537 µmol). The reaction mixture was stirred at room temperature under argon for 30 min. Then 2,6-dimethoxybenzenesulfonamide (**Intermediate INT_{A-1}**) (98 mg, 454 µmol) and DBU (189 mg, 1.24 mmol) were sequentially added at room temperature. The reaction mixture was stirred at room temperature under argon for 16 h. The crude material was dissolved in ethyl acetate and saturated NH₄Cl aqueous solution. After separation, the organic layer was dried over anhydrous sodium sulfate, filtered and concentrated under reduced pressure. The residue was purified by silica gel column chromatography eluted with cyclohexane/ethyl acetate/ethanol 100/0/0 to 60/30/10 to afford *N*-((2,6-dimethoxyphenyl)sulfonyl)-5-(1H-pyrazol-1-yl)-5,6,7,8-tetrahydronaphthalene-2-carboxamide (**Example EXA₂** - **Compound 2**) (53 mg, 28 % yield) as a white solid. LCMS (ES, m/z): 442.3 [M+H]⁺.
¹H NMR (400 MHz, DMSO-*d6*) δ 12.13-12.10 (br. s, 1H), 7.75 (s, 2H), 7.58 (dd, J = 1.63, 8.26 Hz, 1H), 7.52-7.41 (m, 2H), 6.81-6.75 (m, 2H), 6.68 (d, J = 8.16 Hz, 1H), 6.28 (t, J = 2.22 Hz, 1H), 5.68-5.62 (m, 1H), 3.77 (s, 6H), 2.98-2.79 (m, 2H), 2.26-2.14 (m, 2H), 2.04-1.92 (m, 1H), 1.91-1.77 (m, 1H).

### Example EXA₃: N-((5-(tert-butyl)-2-methoxyphenyl)sulfonyl)-5-(1H-pyrazol-1-yl)-5,6,7,8-tetrahydronaphthalene-2-carboxamide (Example EXA₃ - Compound 3)

### Scheme SCH_{EXA3}.

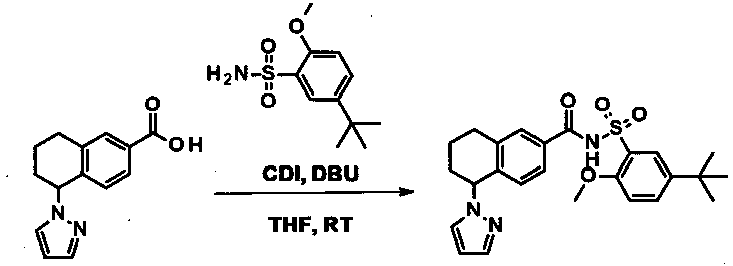

To a mixture of 5-(1H-pyrazol-1-yl)-5,6,7,8-tetrahydronaphthalene-2-carboxylic acid (**Intermediate INT_{B-1}**) (100 mg, 413 µmol) in THF (2.0 mL) at room temperature was added CDI (87 mg, 537 µmol). The reaction mixture was stirred at room temperature under argon for 30 min. Then 5-(*tert*-butyl)-2-methoxybenzenesulfonamide (**Intermediate INT_{A-8}**) (110 mg, 454 µmol) and DBU (189 mg, 1.24 mmol) were added. The mixture was stirred at room temperature under argon for 16 h. The crude material was dissolved in ethyl acetate and saturated NH₄Cl aqueous solution. After separation, the organic layer was dried over anhydrous sodium sulfate, filtered and concentrated under reduced pressure. The residue was purified by silica gel column chromatography eluted with cyclohexane/ethyl acetate/ethanol 100/0/0 to 60/30/10 to afford *N*-((5-(tert-butyl)-2-methoxyphenyl)sulfonyl)-5-(1H-pyrazol-1-yl)-5,6,7,8-tetrahydronaphthalene-2-carboxamide **(Example EXA₃** - **Compound 3)** (69 mg, 34 % yield) as a white solid. LCMS (ES, m/z): 468.4 [M+H]⁺.
¹H NMR (400 MHz, DMSO-*d6*) δ 12.39-12.36 (br. s, 1H), 7.85 (d, J = 2.40 Hz, 1H), 7.76-7.72 (m, 2H), 7.71-7.67 (m, 1H), 7.56 (dd, J = 2.39, 8.57 Hz, 1H), 7.47 (dd, J = 0.58, 1.74 Hz, 1H), 7.15 (d, J = 8.60 Hz, 1H), 6.67 (d, J = 8.30 Hz, 1H), 6.28 (t, J = 2.03 Hz, 1H), 5.64 (t, J = 6.77 Hz, 1H), 3.82 (s, 3H), 2.97-2.76 (m, 2H), 2.28-2.13 (m, 2H), 2.03-1.92 (m, 1H), 1.90-1.75 (m, 1H), 1.30 (s, 9H).

### Example EXA₄: N-((2,6-dimethoxyphenyl)sulfonyl)-8-methoxy-5-(1H-pyrazol-1-yl)-1,2,3,4-tetrahydronaphthalene-2-carboxamide (Example EXA₄ - Compound 4)

### Scheme SCH_{EXA4}.

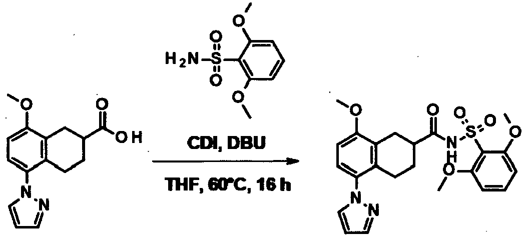

To a mixture of 8-methoxy-5-(1H-pyrazol-1-yl)-1,2,3,4-tetrahydronaphthalene-2-carboxylic acid (**Intermediate INT_{B-6}**) (125 mg, 459 µmol) in DMF (2.30 mL) at 60°C was added CDI (96.8 mg, 597 µmol). The reaction mixture was stirred at 60°C under argon for 1 h. Then 2,6-dimethoxybenzenesulfonamide (**Intermediate INT_{A-1}**) (120 mg, 551 µmol) and DBU (208 µL, 1.38 mmol) were sequentially added at 60°C. The reaction mixture was stirred at 60°C under argon for 16 h. The crude material was dissolved in ethyl acetate and saturated NH₄Cl aqueous solution. After separation, the organic layer was dried over anhydrous sodium sulfate, filtered and concentrated under reduced pressure. The residue was purified by reverse phase C₁₈ column chromatography eluted with H₂O/ACN 100/0 to 0/100 to afford *N*-((2,6-dimethoxyphenyl)sulfonyl)-8-methoxy-5-(1H-pyrazol-1-yl)-1,2,3,4-tetrahydronaphthalene-2-carboxamide (**Example EXA₄** - **Compound 4**) (29 mg, 13 % yield) as a white solid. LCMS (ES, m/z): 472.3 [M+H]⁺.
¹H-NMR (400 MHz, DMSO-*d6*) δ 7.83 (dd, J=0.6, 2.3 Hz, 1H), 7.65 (dd, J=0.7, 1.8 Hz, 1H), 7.32 (s, 1H), 7.11 (d, J=8.4 Hz, 1H), 6.87 (d, J=8.8 Hz, 1H), 6.73 - 6.62 (m, 2H), 6.43 (dd, J=1.8, 2.3 Hz, 1H), 3.84 (s, 3H), 3.78 - 3.71 (m, 6H), 2.87 (dd, J=5.7, 18.0 Hz, 1H), 2.48 - 2.43 (m, 1H), 2.27 (td, J=4.2, 17.5 Hz, 1H), 1.92 - 1.83 (m, 1H), 1.45 - 1.35 (m, 1H).

### Example EXA₅: N-((2,6-dimethoxyphenyl)sulfonyl)-5-(1H-pyrazol-1-yl)-2-naphthamide (Example EXA₅ - Compound 5)

### Scheme SCH_{EXA5}.

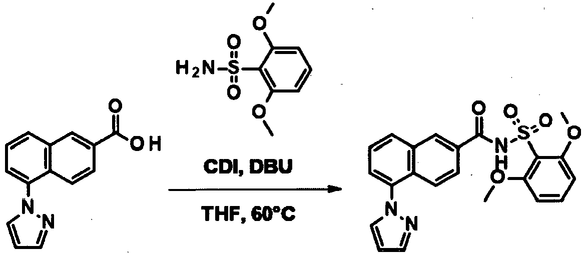

To a mixture of 5-(1H-pyrazol-1-yl)-2-naphthoic acid (**Intermediate INT_{B-2}**) (128 mg, 537 µmol) in DMF (2 mL) at 60 °C was added CDI (113 mg, 698 µmol). The reaction mixture was stirred at 60 °C under argon for 1 h. Then 2,6-dimethoxybenzenesulfonamide (**Intermediate INT_{A-1}**) (140 mg, 644 µmol) and DBU (245 mg, 1.61 mmol) were sequentially added at 60 °C. The mixture was stirred at 60 °C under argon for 16 h. The reaction mixture was diluted with 1N HCl aqueous solution, extracted with ethyl acetate, washed with water then brined, dried over anhydrous sodium sulfate and concentrated under reduced pressure. The residue was purified by silica gel column chromatography eluted with cyclohexane/(ethyl acetate/ethanol (3/1)) from 100/0 to 50/50. The resulting product was triturated in hot acetone, filtered and dried to afford N-((2,6-dimethoxyphenyl)sulfonyl)-5-(1H-pyrazol-1-yl)-2-naphthamide **(Example EXA₅ - Compound 5)** (5.4 mg, 2.2 % yield) as a white solid. LCMS (ES, m/z): 438.3 [M+H]⁺.
¹H NMR (400 MHz, DMSO-*d6*) δ 8.59 (1H, s), 8.21 (1H, d, J=1.7 Hz), 8.14 (1H, d, J=7.9 Hz), 8.07 (1H, d, J=8.8 Hz), 7.86 (1H, s), 7.69 (1H, d, J=9.0 Hz), 7.64 - 7.58 (2H, m), 7.25 (1H, t, J=8.2 Hz), 6.64 (1H, s), 6.62 - 6.60 (2H, m), 3.64 (6H, s).

### Example EXA₆: N-((2,6-dimethoxyphenyl)sulfonyl)-5-(1H-pyrazol-1-yl)-2-naphthamide (Example EXA₆ - Compound 6)

### Scheme SCH_{EXA6}.

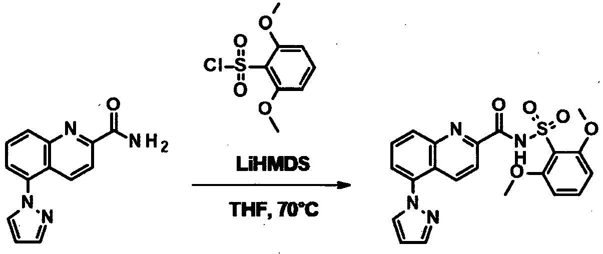

To a mixture of 5-(1H-pyrazol-1-yl)quinoline-2-carboxamide (**Intermediate INT_{B-3}**) (250 mg, 1.05 mmol) and 2,6-dimethoxybenzenesulfonyl chloride (100 mg, 1.05 mmol) in THF (20 mL) at 70°C was dropwise added lithium bis(trimethylsilyl)amide solution (834 µL, 4.20 mmol) over 5 min. The reaction mixture was stirred at 70 °C for 3 h. The reaction mixture was quenched with methanol then concentrated under reduced pressure. The residue was purified by silica gel column chromatography eluted with cyclohexane/(ethyl acetate/ethanol 3/1)) from 100/0 to 0/100. The resulting product was triturated in a mixture acetonitrile/diethyl ether (1/1) to afford *N*-((2,6-dimethoxyphenyl)sulfonyl)-5-(1H-pyrazol-1-yl)quinoline-2-carboxamide **(Example EXA₆** - **Compound 6)** (8 mg, 2 % yield) as a yellow solid. LCMS (ES, m/z): 439.2 [M+H]⁺.
¹H NMR (400 MHz, DMSO-*d6*) δ 8.31 - 8.27 (2H, m), 8.15 (1 H, d, J=8.4 Hz), 8.11 (1H, d, J=9.2 Hz), 7.91 - 7.89 (1H, m), 7.87 (1H, t, J=7.5 Hz), 7.71 (1H, d, J=7.0 Hz), 7.27 (1H, t, J=8.2 Hz), 6.67 - 6.63 (3H, m), 3.67 (6H, s).

### Example EXA₇: N-((2,6-dimethoxyphenyl)sulfonyl)-5-(1H-pyrazol-1-yl)-5,6,7,8-tetrahydro quinoline-2-carboxamide (Example EXA₇ - Compound 7)

### Scheme SCH_{EXA7}

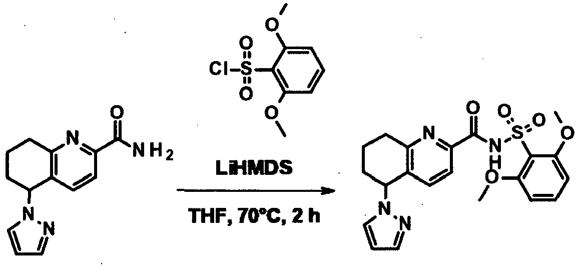

To a mixture of 5-(1H-pyrazol-1-yl)-5,6,7,8-tetrahydroquinoline-2-carboxamide (**Intermediate INT_{B-4}**) (120 mg, 495 µmol) and 2,6-dimethoxybenzenesulfonyl chloride (117 mg, 495 µmol) in THF (5 mL) at 70°C was dropwise added lithium bis(trimethylsilyl)amide solution (394 µL, 1.98 mmol) over 5 min. The reaction mixture was stirred at 70 °C for 2 h. The reaction mixture was quenched with methanol then concentrated under reduced pressure. The crude was purified by preparative HPLC to afford *N*-((2,6-dimethoxyphenyl)sulfonyl)-5-(1H-pyrazol-1-yl)-5,6,7,8-tetrahydroquinoline-2-carboxamide (**Example EXA7 - Compound 7**) (2 mg, 0.8 % yield) as a white solid. LCMS (ES, m/z): 443.2 [M+H]⁺.
¹H NMR (400 MHz, DMSO-*d6*) δ 11.23 (1H, s), 7.86 - 7.84 (1H, m), 7.73 (1H, d, J=8.0 Hz), 7.54 (1H, t, J=8.3 Hz), 7.50 (1H, dd, J=0.6, 1.9 Hz), 7.20 - 7.16 (1H, m), 6.81 (2H, d, J=8.5 Hz), 6.31 (1H, dd, J=2.0, 2.2 Hz), 5.80 - 5.75 (1H, m), 3.80 (6H, s), 3.12 - 3.08 (2H, m), 2.28 - 2.23 (2H, m), 2.11 - 2.08 (1H, m), 2.02 - 1.93 (1H, m).

### Example EXA₈: N-((2,6-dimethoxyphenyl)sulfonyl)-4-(1H-pyrazol-1-yl)-3,4-dihydro-2H-pyrano[2,3-b]pyridine-7-carboxamide (Example EXA₈ - Compound 8)

### Scheme SCH_{EXA8}

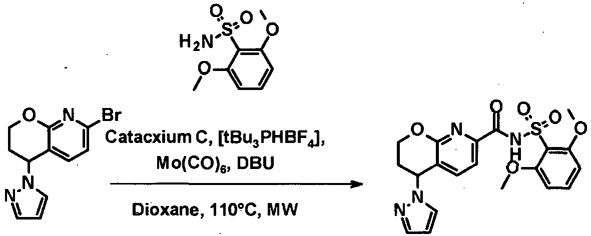

To a solution of 7-bromo-4-(1H-pyrazol-1-yl)-3,4-dihydro-2H-pyrano[2,3-b]pyridine (**Intermediate INT_{B-5}**) (200 mg, 713 µmol) in dioxane (14 mL) was added DBU (322 µL, 2.14 mmol), 2,6-dimethoxybenzenesulfonamide (**Intermediate INT_{A-1}**) (310 mg, 1.42 mmol), carbon monooxide - molybdenum (6:1) (188 mg, 713 µmol), tri-tert-butylphosphonium tetrafluoroborate (41 mg, 142 µmol) cataCXium C (66.9 mg, 71.3 µmol) and finally DBU (322 µL, 2.14 mmol) .The reaction mixture was stirred at 110 °C for 2 h under microwave irradiations. The crude material was dissolved in dichloromethane and saturated NH₄Cl aqueous solution. After separation, the organic layer was dried over anhydrous sodium sulfate, filtered and concentrated under reduced pressure. The residue was purified by preparative HPLC to afford N-((2,6-dimethoxyphenyl)sulfonyl)-4-(1H-pyrazol-1-yl)-3,4-dihydro-2H-pyrano[2,3-b]pyridine-7-carboxamide (**Example EXA8 - Compound 8**) (70 mg, 21 % yield) as a white solid.
LCMS (ES, m/z): 445.1 [M+H]+.
¹H-NMR (400 MHz, CDCl3) δ 10.56 - 10.53 (m, 1H), 7.73 (d, J=7.8 Hz, 1H), 7.64 - 7.62 (m, 1H), 7.44 (t, J=8.5 Hz, 1H), 7.42 (dd, J=0.9, 7.9 Hz, 1H), 7.37 (dd, J=0.4, 2.4 Hz, 1H), 6.64 (d, J=8.6 Hz, 2H), 6.36 (t, J=2.1 Hz, 1H), 5.72 - 5.66 (m, 1H), 4.55 (t, J=5.4 Hz, 2H), 3.96 (s, 1H), 2.71 - 2.63 (m, 1H), 2.54 - 2.46 (m, 1H).

### II. Biological Examples

### Abbreviations:

Acetyl coenzyme A : AcetylCoA or AC-Coa
Bovine serum albumin : BSA
Dithiothreitol : DTT
Ethylenediaminetetraacetic acid : EDTA
Trizma Hydrochloride : Tris-HCL

### KAT6A Enzyme Activity Assay (AlphaScreen Method)

### Materials and Instruments

Active KAT6A/MOZ protein: Active motif # 81223 _ lot. 2122003
H4 peptide: Upstate Biotechnology # 12-405 lot 3743857 - 100 µg
AcetylCoA: Sigma # A2056 lot 0000303137 - 5 mg
Anti-Histone H4 (acetyl K8) antibody:
   Cell Signaling # 2594S lot 11 (a-H4AcK8-1) 73 µg/mL
   Abcam # ab15823 lot 1029174-1 (a-H4AcK8-2) 0.9 mg/mL
Trizma Hydrochloride solution pH8 (Sigma # T3038 lot SLBL2857V) 100 mM
NaCl (Sigma # S5150 lot SLBH4802V) 15 mM
EDTA (Sigma # E7889 lot SLBD5434V) 1 mM
Tween-20 (Sigma # P7949 lot SZBC2920V) 0.01%
BSA (Jackson v 001-000-173) 0.02% (= 0.2 mg/mL)
DTT (Sigma # 43816) 1mM
384-well plate: AlphaPlate (PerkinElmer # 6005350)
TopSeal-A PLUS (PerkinElmer # 6050185)
AlphaLISA beads :
   Protein A acceptor (PerkinElmer # AL101C lot 3178960)
   Streptavidin donor beads (PerkinElmer # 6760002S lot 3082282)

### Assay preparation:

Assay Buffer with DTT: Composed of 100 mM Tris-HCl (pH 8), 15 mM NaCl, 1 mM EDTA, 0.01% Tween-20, 0.02% BSA, 1 mM DTT.

Compounds Solutions: Compounds solutions 3x were prepared in the assay buffer with DTT from solution comprising different concentrations of compounds in DMSO.

KAT6A Enzyme Solution 3x: Prepared a 60 nM for a use at a final concentration of 20 nM in the 3× assay buffer with DTT.

H4 peptide, antibodies, Ac-Coa 3X: Contains 150nM H4 peptide; 3 µM Ac-CoA and 1/1167 Cell signaling # 2594S and 1/1000 Abcam # ab15823 in the 1× assay buffer with DTT.

Detection Reagent AlphaLisa beads 2.2X: Consists of AlphaScreen Protein A Acceptor Beads and AlphaScreen Streptavidin Donor Beads (2.93 or 8.8 µg/mL (final concentration, all prepared in the 1× assay buffer.

### Assay Procedure:

The prepared enzyme solution was added to a 384-well plate distribute of 4µL compounds 3x or DMSO and 4 µL KAT6A enzyme 3x, then vortexed and incubated 15 minutes at 30°C.

4 µL Mix antibodies + AcetylCoA + H4 peptide 3x or 4 µL (Mix antibodies + H4 peptide) 3x (condition w/o AcetylCoA) is added, then the plate is vertically centrifugated, vortexed and incubated 1h at 30°C with a film.

10 µL mix AlphaLISA beads 2.2 x in Assay buffer with DTT is added, and the plate is vortexed and incubated 5h at 23 °C protected from light before reading results with Envison.

### Cellular Assay ZR75-1 H3K23ac

Materials and reagents:
Cell line: ZR-75-1 (Human breast ductal carcinoma, ECACC #87012601)
Cell line passage number: P'13
Medium: RPMI 1640 (Gibco #41965062) supplemented with:
   ∘ 10% Fetal Bovine Serum (FBS, Pan Biotech #P30-3306)
   ∘ 1% Glutamax (Gibco #35050038)
   o 1% Sodium pyruvate (Gibco #11360-039)
   o 4.5 g/L glucose
Plates: 96-well Nunc^{™} Edge^{™} Nunclon Delta surface plates (ThermoFisher #167425)
Histone H3 Monoclonal Antibody (6D3B9), ThermoFisher # MA5-31759 lot XG3638535
Acetyl-Histone H3 (Lys23) (D6Y7M) rabbit mAb, Cell Signaling # 14932
Glo Substrate Reagent Pack DY993: R&D Systems lot P392320
HRP secondary antibodies Jackson Immuno Research Laboratories HRP DONKEY IGG ANTI RABBIT IGG (H+(Interchim # 711-035-152)
Envision luminometer in luminescent reading mode

### Cell Seeding

ZR-75-1 cells (passage 13) are seeded into two 96-well plates. Each well receives 25,000 cells in 200 µL of RPMI 1640 medium supplemented with 10% FBS, 1% Glutamax, 1% sodium pyruvate, and 4.5 g/L glucose. The plates are incubated for 24 hours at 37°C and 5% CO2 to allow the cells to adhere and reach an appropriate confluence for treatment.

### Compound Treatment

The compounds are prepared for treatment. Stock solutions of the compounds are diluted in DMSO. The final working concentrations for treatment are achieved by diluting the compounds dilutions in the medium. After aspirating the culture medium from each well, 180 µL of fresh incubation medium is added. Then, 20 µL of the compound solutions are added to each well, bringing the final volume to 200 µL per well. The plates are incubated for another 24 hours under the same conditions (37°C, 5% CO2).

### Cell Lysis and Preparation for ELISA

After 24 hours of compound incubation, the medium is aspirated from the wells, and the cells are lysed. The lysis buffer is prepared by combining 0.4 N HCl with 2 mM sodium butyrate. Each well receives 100 µL of this lysis buffer and is then agitated for 1 hour at 4°C. After lysis, the lysates are neutralized by adding 75 µL of neutralization buffer (1 N sodium phosphate dibasic pH 12.5 + 2 mM sodium butyrate + 1 protease inhibitor tablet). The plate is then agitated for 5 minutes at room temperature, and the entire plate is frozen at -80°C for 1 hour before further processing.

### ELISA Assay for Histone H3 Acetylation (Lys23) - ELISA plate preparation

The ELISA procedure begins with coating the ELISA plate. A half-area 96-well white OptiPlate is coated with Histone H3 monoclonal antibody (6D3B9) at 2 µg/mL in coating buffer (10 mM Tris-HCl pH 8.0, 10 mM NaCl). The antibody is diluted from its stock solution and distributed at 25 µL per well in the ELISA plate. The plate is sealed and incubated overnight at 4°C.

After overnight incubation, the ELISA plate is washed three times with 200 µL of PBS + 0.05% Tween20. The plate is then blocked by adding 100 µL per well of blocking buffer (PBS + 0.05% Tween20 + 1% BSA), and it is incubated for 1 hour at room temperature. After blocking, the plate is washed three more times with 200 µL of the wash buffer.

### ELISA Assay for Histone H3 Acetylation (Lys23) - HRP Substrate Addition and Plate Reading

The previously prepared cell lysates are thawed on ice and added to the ELISA plate. Each well receives 50 µL of cell lysate, and the plate is incubated for 1.5 hours at room temperature. In parallel, blanks are prepared using hydrochloric acid and sodium phosphate dibasic buffers containing sodium butyrate. After incubation, the plate is washed three times with the wash buffer.

Next, the primary antibody, Acetyl-Histone H3 (Lys23) rabbit mAb (D6Y7M), is diluted to 0.1 µg/mL in the blocking buffer and 25 µL is added to each well. The plate is sealed and incubated for 1.5 hours at room temperature. The plate is washed again, and then a secondary HRP-conjugated antibody (Donkey IgG anti-Rabbit IgG, diluted to 0.4 µg/mL in blocking buffer) is added at 50 µL per well. The plate is incubated for 1 hour at room temperature, followed by three washes with the wash buffer.

The HRP substrate is prepared by mixing Glo Reagent A and Glo Reagent B in a 1:2 ratio to produce 7 mL of luminescent substrate. Fifty microliters of this substrate mixture are added to each well, and the plate is incubated at room temperature for 10 minutes in the dark.

Finally, the luminescence is measured using the Envision luminometer in luminescent reading mode. The luminescence readings provide a quantitative measure of histone acetylation at Lys23, which is used to assess the inhibition of KAT6A activity. The IC50 values for each compound are determined by plotting the luminescence data against the compound concentrations and fitting the data to a four-parameter logistic model to calculate the concentration at which 50% inhibition of KAT6A activity occurs.

### Comparative compounds

The compounds of the invention have been compared to a well known inhibitor of KAT6A activity: PF07248144.

### Assay Procedure

The prepared enzyme solution was added to a 384-well plate distributed of 4µL compounds 3x or DMSO and 4 µL KAT6A enzyme 3x, then vortexed and incubated 15 minutes at 30°C.

4 µL Mix antibodies + AcetylCoA + H4 peptide 3x or 4 µL (Mix antibodies + H4 peptide) 3x (condition w/o AcetylCoA) is added, then the plate is vertically centrifugated, vortexed and incubated 1h at 30°C with a film.

10 µL mix AlphaLISA beads 2.2 x in an assay buffer with DTT is added, and the plate is vortexed and incubated 5h at 23 °C protected from light before reading results with Envison.

Results on the efficacy of the compounds of the invention on KAT6A inhibition

The results from the inhibition of the enzymatic activity of Human KAT6A by the comparative compound PF07248144 and the compounds of the invention are reported in the following table 4.

Inhibitory effects were assessed using both enzymatic and cellular assays, with results expressed in micromolar (µM). The data provides a comparative analysis of the inhibition efficacy across these two assay formats.

**Table 4: IC₅₀ Values for the Inhibition of the enzymatic activity of Human KAT6A by the Compounds and control compound PF07248144 using both an enzymatic assay and a cellular assay. Values are reported in µM. (r: racemic, e1: enantiomer 1; e2: enantiomer 2).**

| **Compound ID** | **KAT6A Mean enzymatic Assay (µM)** | **Cellular assay ZR75-1 H3K23ac (µM)** |
|---|---|---|
| PF07248144 (Comparative compound) | 0.004 | 0.001 |
| 1 | 0.411 | N/A |
| 2-r (racemic) | 0.11 | 1.17 |
| 2-e1 (enantiomer 1) | 0.256 | N/A |
| 2-e2 (enantiomer 2) | 0.064 | 0.821 |
| 3-r | 0.08 | 0.51 |
| 4-r | 0.56 | 1.8 |
| 5-r | 0.014 | 0.017 |
| 6-r | 0.018 | 0.014 |
| 7-r | 0.1156 | 0.14 |
| 7-e1 | 0.065 | 0.19 |
| 7-e2 | 4.180 | N/A |

As shown by the results in Table 4, the compounds according to the invention exhibit activity against KAT6A at very low concentrations in the enzymatic assay and in cells.

### Radiometric evaluation for KAT6A, KAT6B, and KAT7

Histone acetyltransferase assays performed at Reaction Biology are radiometric activity assays using tritiated acetyl-Coenzyme A as a cofactor.

### Materials and reagents:

MYST3/KAT6A (Active Motif Cat# 81223) Recombinant human KAT6A protein that includes amino acids 488-778 (accession number NP_006757.2) was expressed in a baculovirus expression system and contains an N-terminal FLAG tag. The molecular weight of the protein is 35.5 kDa. This product is the catalytic domain of KAT6A protein.

MYST4/KAT6B (Sino Biological Cat# K315-31BG) Recombinant human KAT6B protein that includes amino acids 657-1069 (accession number NM_012330) was expressed in a baculovirus expression system and contains an N-terminal GST tag. The molecular weight of the protein is 76 kDa. This product is the catalytic domain of KAT6B protein.

MYST2/KAT7 (Sino Biological Cat# K315-31BG) Recombinant full length human KAT7 protein (accession number NM_007067) was expressed in a baculovirus expression system S9 insect cells and contains an N-terminal GST tag. The molecular weight of the protein is 110 kDa.

### Substrates: H3, or H4 (all full length)

MYST3/KAT6A: 10 nM + 5 µM Histone H3
MYST4/KAT6B: 10 nM + 2.5 µM Histone H4
MYST2/KAT7: 25 nM + 2.5 µM Histone H3

### [³H]-acetyl-CoA concentrations:

KAT6A: 0.5 µM
KAT6B: 0.5 µM
KAT7: 0.5 µM

### Assay buffer:

50 mM Tris-HCl, pH 8.0, 100 mM NaCl, 0.1 mM EDTA, 0.1 mM DTT, 10% glycerol.

### Liquid scintillation counter or microplate scintillation reader.

### Procedure

The enzymes are diluted in an appropriate assay buffer.

The tritiated acetyl-CoA ([³H]-acetyl-CoA) are prepared at a final concentration of 0.5 µM for KAT6A/B and KAT7.

Test compounds are prepared by serial dilution to create 10-dose IC50 curves, starting from 10 µM. Each reaction well in the plate receives histone substrate, enzyme, and the test compound or control. The reaction is initiated by adding the acetyl-CoA.

For reaction conditions, the assay is incubated at 30°C to 37°C for 30-60 minutes. Upon completion, the reaction is stopped by adding a stop solution, such as 2% SDS or acetic acid.

Filtration and detection involve transferring the reaction mixture to GF/B filter plates for radioisotope-based filtration. The unincorporated acetyl-CoA is removed by washing the plates three to five times with a cold buffer, leaving only the acetylated histone bound to the filter. The filter plates are then air-dried, and scintillation fluid is added to each well for detection. A scintillation counter measures the incorporation of the radioactive acetyl group into the histone substrate, with counts per minute (cpm) serving as the readout.

Data analysis starts by calculating the percentage inhibition of acetylation for each test compound relative to a vehicle control (DMSO). The IC50 is determined by plotting the percentage inhibition against the compound concentration on a logarithmic scale. A four-parameter logistic curve is fitted to the data to calculate the concentration of the compound required to inhibit 50% of the enzymatic activity

**Table 5: IC₅₀ Values for the inhibition of Human KAT6A, KAT6B and KAT7 by the compounds of the invention and control compound PF07248144 in radiometric assay.**

| **Compound ID** | **KAT6A (µM) Radiometric** | **KAT6B (µM) Radiometric** | **KAT7 (µM) Radiometric** | **KAT6A/KA T6B ratio** |
|---|---|---|---|---|
| PF07248144 | 0.0005 | 0.05 | 3.1 | 98 |
| 2-e2 | 0.075 | 10 | > 100 | **133** |
| 5-r | 0.007 | 0.987 | 38 | **141** |
| 6-r | 0.009 | 1.011 | 89.350 | 107 |

As shown by the results in Table 5, the compounds according to the invention exhibit activity against KAT6A at very low concentrations as measured by radiometric assay.

In addition, they exhibit a high specificity for KAT6A compared to other enzymes of the same family such as KAT6B and KAT7. For example, compounds 215, 251 and 252 have micromolar affinities for KAT6B, with 10 and 1 µM, respectively; whereas comparative compound PF07248144 binds at 50 nM. Compound 215 does not bind KAT7 at 100 µM and compounds 252 and 251 bind KAT7 at 90 and 38 µM, respectively; whereas the comparative compound PF07248144 binds KAT7 at 3 µM.

Such specificity for KAT6A compared to KAT6B and KAT7 is an asset for the molecules according to the present invention and suggests a therapeutic potential superior to the molecules of the state of the technique.

Such specificity for KAT6A compared to KAT6B, combined with the specificity for KAT6A over KAT7 confirm the strong therapeutic potential of the compounds according to the invention.

### REFERENCES

Bergamasco, M.I., Ranathunga, N., Abeysekera, W., et al. The histone acetyltransferase KAT6B is required for hematopoietic stem cell development and function. Stem Cell Reports. 2024 Apr 9;19(4):469-485. doi: 10.1016/j.stemcr.2024.02.005. Epub 2024 Mar 21. PMID: 38518784; PMCID: PMC11096436.

Coenen, Eva A et al. Pediatric acute myeloid leukemia with t(8;16)(p11;p13), a distinct clinical and biological entity: a collaborative study by the International-Berlin-Frankfurt-Munster AML-study group. Blood vol. 122,15 (2013): 2704-13

Hu, Z. et al. Genomic characterization of genes encoding histone acetylation modulator proteins identifies therapeutic targets for cancer treatment. Nat Commun 10, 733 (2019). Lv, D., et al. "Histone Acetyltransferase KAT6A Upregulates PI3K/AKT Signaling through TRIM24 Binding." Cancer Research, vol. 77 (22) (2017): 6190-6201 Mukohara T. et al. Inhibition of lysine acetyltransferase KAT6 in ER+HER2- metastatic breast cancer: a phase 1 trial. Nat Med 30, 2242-2250 (2024).

Mukohara, T., Park, Y.H., Sommerhalder, D. et al. Inhibition of lysine acetyltransferase KAT6 in ER+HER2- metastatic breast cancer: a phase 1 trial. Nat Med 30, 2242-2250 (2024).

Sharma S. et al. Discovery of a highly potent, selective, orally bioavailable inhibitor of KAT6A/B histone acetyltransferases with efficacy against KAT6A-high ER+ breast cancer. Cell Chemical Biology, Volume 30, Issue 10, 2023, 1191-1210.e20

Stahl, P. H., & Wermuth, C. G. (Eds.). (2011). Handbook of Pharmaceutical Salts: Properties, Selection, and Use.

Turner-Ivey B. et al. KAT6A, a Chromatin Modifier from the 8p11-p12 Amplicon is a Candidate Oncogene in Luminal Breast Cancer. Neoplasia, 16 (2014), pp. 644-655 Weber L.M. et al. The Histone Acetyltransferase KAT6A is Recruited to Unmethylated CpG Islands via a DNA Binding Winged Helix Domain. Nucleic Acids Research, vol. 51, no. 2, 25 Jan. 2023, pp. 574-594

## Claims

1. A compound of general formula (I) 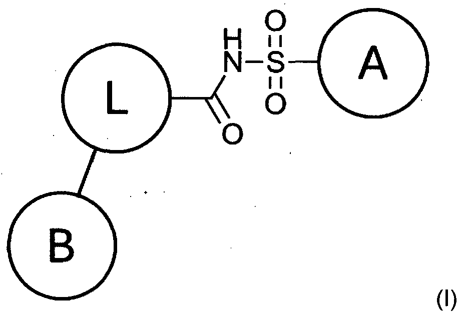 or a pharmaceutically acceptable salt thereof, wherein:
**A represents** an aryl group or a heteroaryl group, such as a benzo-fused alicyclic group, preferably a benzo-fused heterocyclic group;
wherein A is optionally substituted by one or more members selected from the group consisting of:
- alkoxy groups, such as a C₁-C₄ alkoxy group;
- alkyl groups, such as a C₁-C₄ alkyl group, linear or branched, optionally substituted by 1 to 3 fluorine atoms or a hydroxy group;
- halogens;
- unsubstituted or substituted amino groups, such as dialkylamino groups, such as a dimethylamino group (-N(CH₃)₂);
- cycloalkyl groups optionally substituted by one or more alkyl groups and/or halogens, such as a C₃-C₅ cycloalkyl group optionally substituted by 1 to 2 fluorine atoms or a hydroxy group;
- cycloalkyloxy groups, optionally substituted by one or more alkyl groups and/or halogens; and
- heterocyclyl groups, preferably a 4- to 6-member heterocycloalkyl group, such as azetidine, pyrrolidine, piperidine or piperazine, optionally substituted by one or more alkyl groups, hydroxy groups, alkoxy groups, dialkylamino groups, oxo groups and/or halogens; and
**L represents** a fused multicyclic ring system comprising at least two rings;
wherein:
- the fused multicyclic ring system is, as a whole, aromatic or partially saturated;
- the rings of the fused multicyclic ring system optionally includes one or more heteroatoms; preferably a partially saturated ring of the fused multicyclic ring system can include a heteroatom selected from nitrogen, oxygen, and sulfur; preferably a partially saturated ring or an aromatic ring of the fused multicyclic ring system can include a nitrogen;
- a carbon atom of one ring within the system is connected to group A via a N-acylsulfonamide function and a different ring is connected to group B; and
**B represents** a five-or six-membered aromatic heterocyclic ring preferably selected from pyrazole, thiazole, and pyridine, said ring being optionally substituted with one or more substituents; preferably, said ring is substituted with one or more substituents selected from alkyl groups such as methyl, halogenoalkyl groups such as fluoromethyl or difluoromethyl, and/or cycloalkyl groups.

2. The compound according to claim 1, wherein the compound is of formula (II): 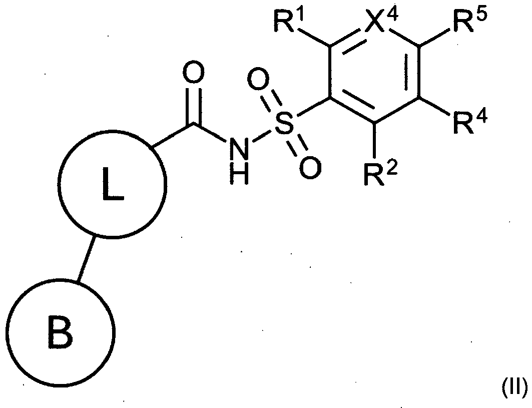 or a pharmaceutically acceptable salt thereof, wherein:
X⁴ is selected from -C-R³ or nitrogen;
R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂); Alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens.

3. The compound according to claim 1, wherein the compound is of formula (III): 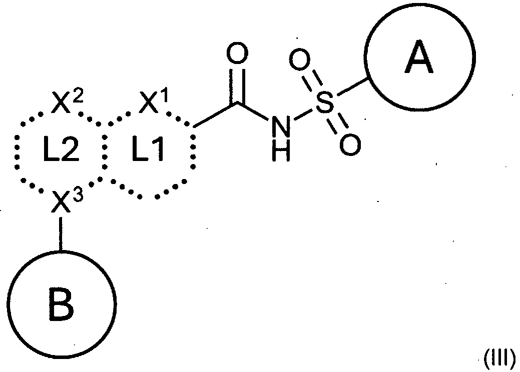 or a pharmaceutically acceptable salt thereof, wherein:
L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
X¹ represents a nitrogen atom or a carbon atom, each further bonded to a sufficient number of hydrogen atoms to satisfy its valence;
X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group,
optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and
X³ represents a nitrogen atom or a carbon atom further bonded to a sufficient number of hydrogen atoms to satisfy its valence.

4. The compound according to claim 1, wherein the compound is of formula (IVa): 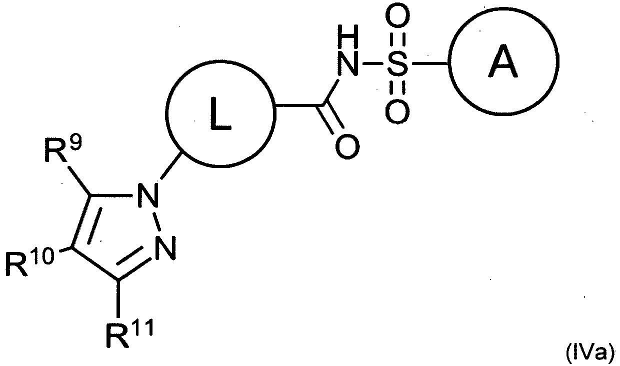 or a pharmaceutically acceptable salt thereof, wherein R⁹, R¹⁰ and R¹¹ each independently represent H; or an alkyl group, such as a linear or branched C₁-C₄ alkyl group, optionally substituted by 1 to 3 fluorine atoms; or a cycloalkyl group.

5. The compound according to claim 1, wherein the compound is of formula (IVb): 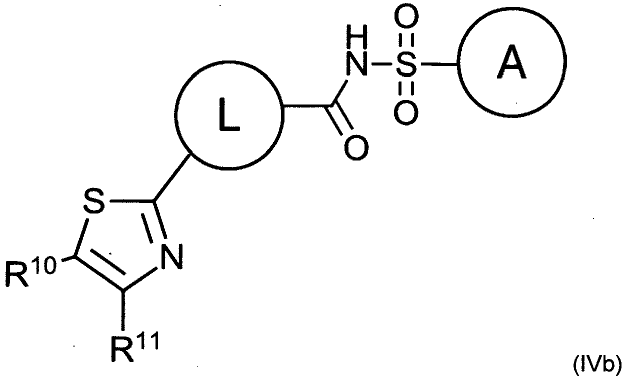 or a pharmaceutically acceptable salt thereof, wherein R¹⁰ and R¹¹ each independently represent H; or an alkyl group, such as a linear or branched C₁-C₄ alkyl group, optionally substituted by 1 to 3 fluorine atoms; or a cycloalkyl group.

6. The compound according to any one of claim 1 to 4, wherein the compound is of formula (Va): 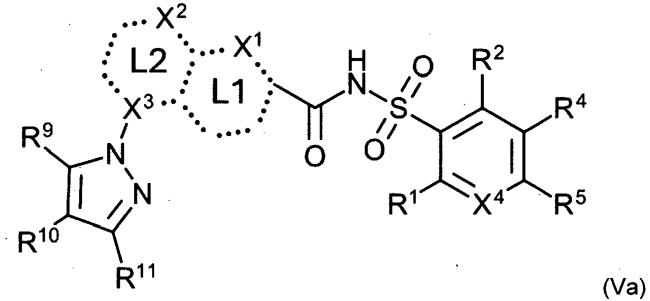 or a pharmaceutically acceptable salt thereof, wherein:
X⁴ is selected from -C-R³ or nitrogen;
R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂); Alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens;
L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
X¹ represents a nitrogen atom or a carbon atom, each further bonded to a sufficient number of hydrogen atoms to satisfy its valence;
X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and
X³ represents a carbon atom further bonded to a sufficient number of hydrogen atoms to satisfy its valence; and
R⁹, R¹⁰ and R¹¹ each independently represent H; or an alkyl group, such as a linear or branched C₁-C₄ alkyl group, optionally substituted by 1 to 3 fluorine atoms; or a cycloalkyl group.

7. The compound according to any one of claims 1 to 3 or 5, wherein the compound is of formula (Vb): 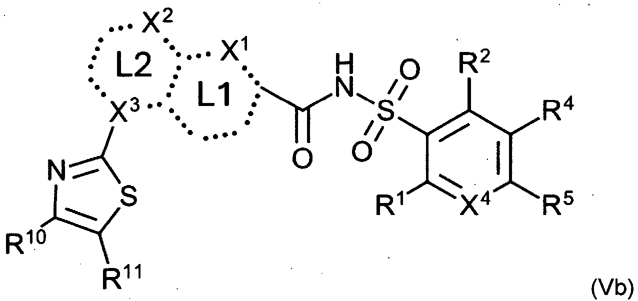 or a pharmaceutically acceptable salt thereof, wherein:
R¹, R², R⁴, R⁵, and, when present, R³, each independently represent H; an alkyl group, such as a linear or branched C₁-C₄ alkyl group, optionally substituted by 1 to 3 fluorine atoms or a hydroxy group; a cycloalkyl group, optionally substituted by one or more alkyl groups and/or halogens and/or hydroxy; a 4- to 6-member heterocyclyl group, such as azetidine, pyrrolidine, piperidine or piperazine, optionally substituted by one or more alkyl groups, hydroxy groups, alkoxy groups, dialkylamino groups, oxo groups and/or halogens; a halogen; an alkoxy group, such as a C₁-C₄ alkoxy group; a cycloalkyloxy group, optionally substituted by one or more alkyl groups and/or halogens; or a substituted or unsubstituted amino group, such as dialkylamino groups, such as a dimethylamino group (-N(CH₃)₂); Alternatively, any one pair selected from R¹ and R³, R² and R⁴, R⁴ and R⁵, or R³ and R⁵, taken together, form a cycloalkyl group, a 5- or 6-member heterocycloalkyl group or a heteroaromatic group, optionally substituted by one or more alkyl groups and/or halogens, while the remaining R¹, R², R³, R⁴, and R⁵, each independently, represent H; an alkyl group, optionally substituted by 1 to 3 fluorine atoms or a hydroxy group; a cycloalkyl group, optionally substituted by one or more alkyl groups and/or halogens and/or hydroxy groups; a halogen; an alkoxy group, such as a C₁-C₄ alkoxy group; a cycloalkyloxy group, optionally substituted by one or more alkyl groups and/or halogens;
L1 and L2 each independently represent a 6-membered ring, wherein at least one of L1 and L2 is aromatic or heteroaromatic; wherein the rings are completed by the required number of hydrogen atoms or valences to satisfy valency rules, unless explicitly substituted;
X¹ represents a nitrogen atom or a carbon atom, each further bonded to a sufficient number of hydrogen atoms to satisfy its valence;
X² represents an oxygen or a sulfur atom, -N-R⁸ wherein R⁸ being a C₁-C₅ alkyl or cycloalkyl; -(CR⁶)-; or -(CR⁶R⁷)-, wherein R⁶ and R⁷ each independently represent a hydrogen, an alkyl group, optionally substituted by a halogen such as fluorine; a halogen such as fluorine or a cyclic group, such as a 3- to 6-cycloalkyl group, optionally substituted by one or more alkyl groups and/or halogens; an alkynyl group, such as a C₂-C₄ alkynyl group; an alkoxy group, such as a C₁-C₄ alkoxy group; alternatively, when X² represents -(CR⁶R⁷)-, R⁶ and R⁷ taken together form a cyclic group, such as a 3- to 5-cycloalkyl group, optionally substituted by one or more alkyl groups and/or halogens; and
X³ represents a nitrogen atom or a carbon atom further bonded to a sufficient number of hydrogen atoms to satisfy its valence;
R¹⁰ and R¹¹ each independently represent H; or an alkyl group, such as a linear or branched C₁-C₄ alkyl group, optionally substituted by 1 to 3 fluorine atoms; or a cycloalkyl group.

8. The compound according to anyone of claims 1 to 7, wherein the **L group** comprises an aromatic bicyclic system or wherein L1 and L2 together consist in an aromatic bicyclic system, the rings of the aromatic bicyclic system optionally includes one or more nitrogen atoms.

9. The compound according to anyone of claims 3, 6, or 7, wherein L1 is an aromatic ring or heteroaromatic ring; preferably, L1 and L2 are both independently an aromatic ring or a heteroaromatic ring.

10. A compound of general formula (I) or a pharmaceutically acceptable salt thereof according to any of claims 1 to 9, wherein the compound has a structure selected from: 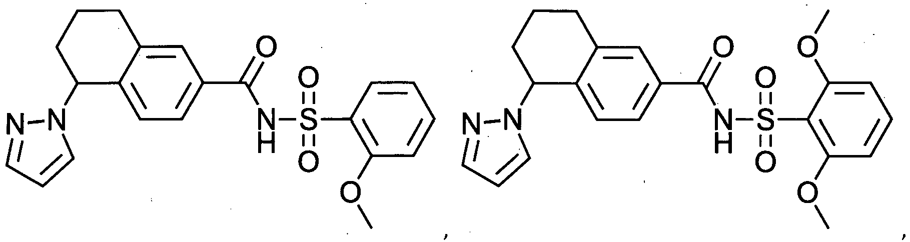 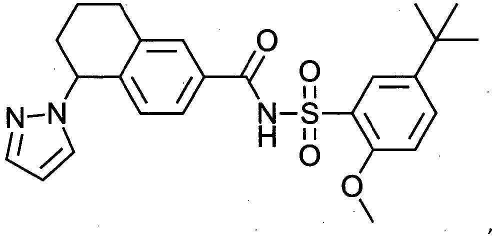 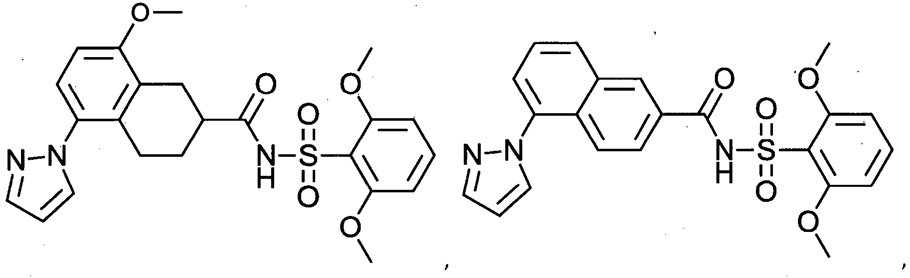 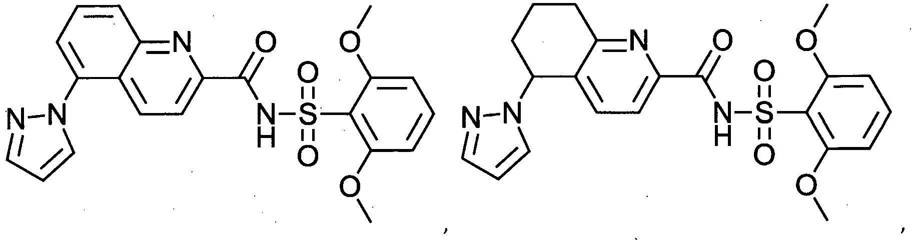 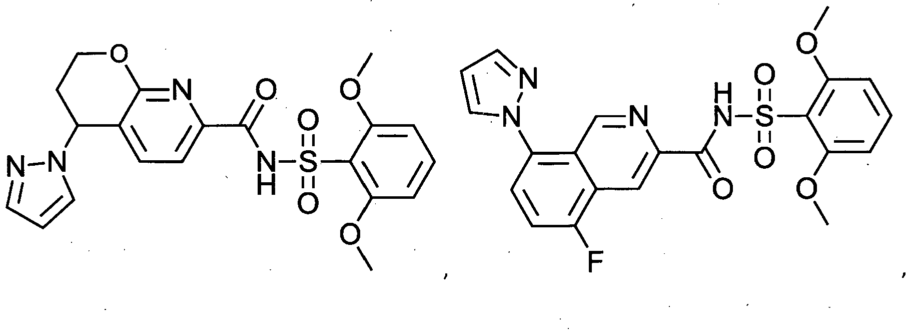 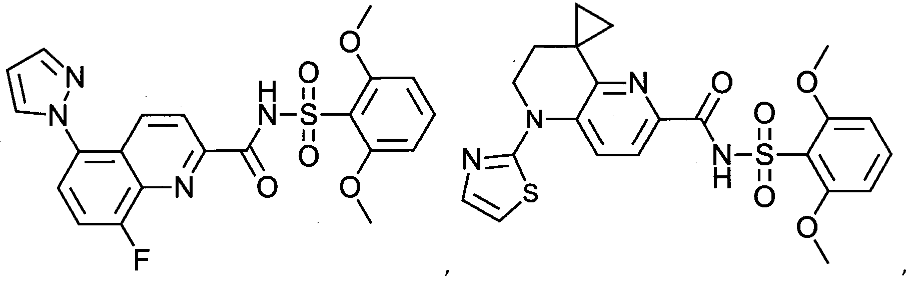 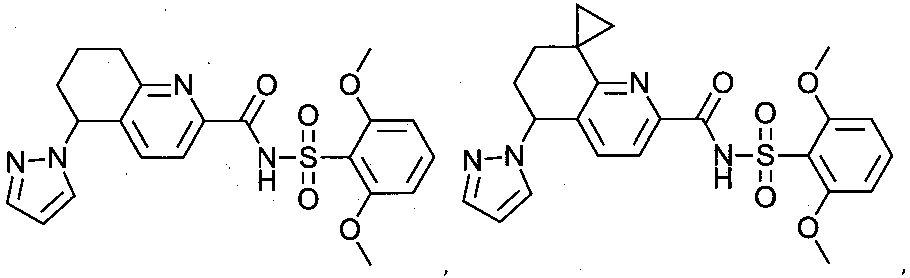 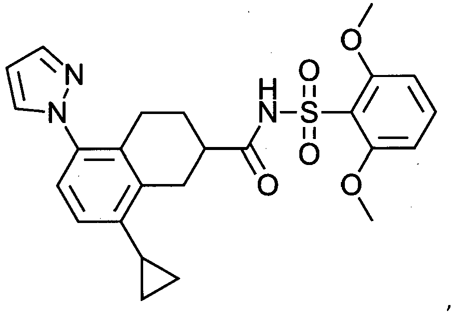 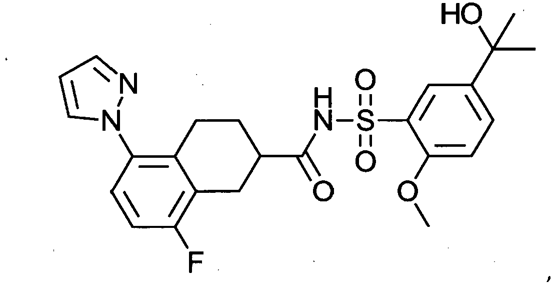 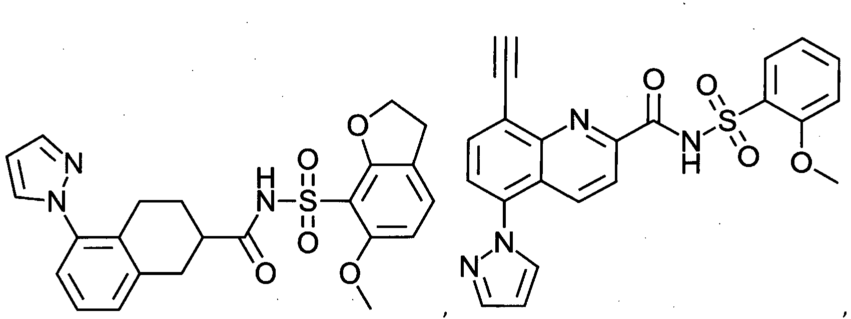 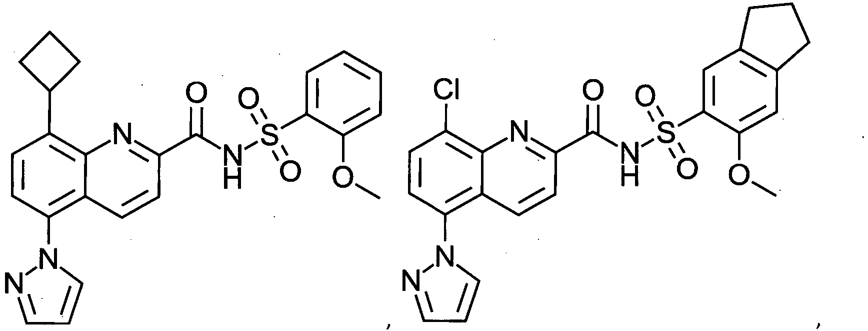 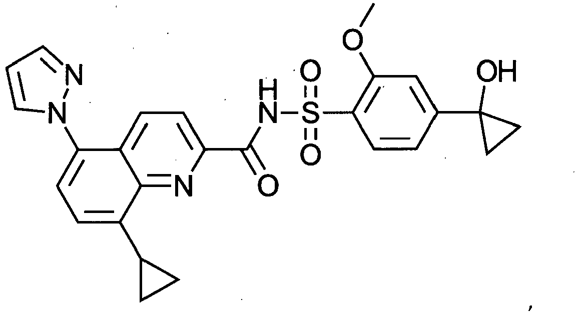 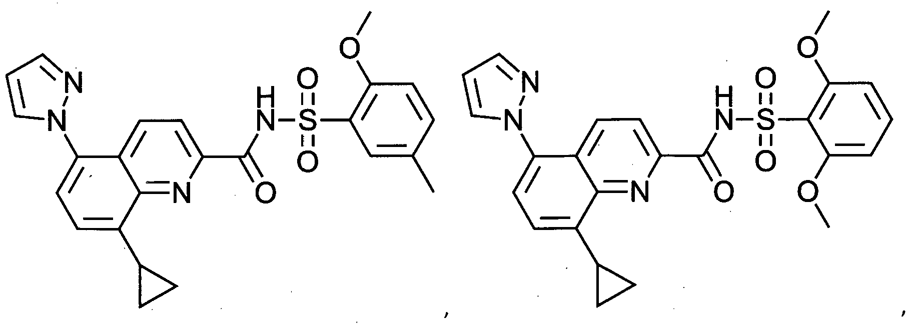 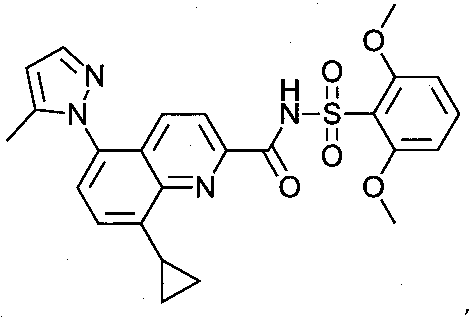 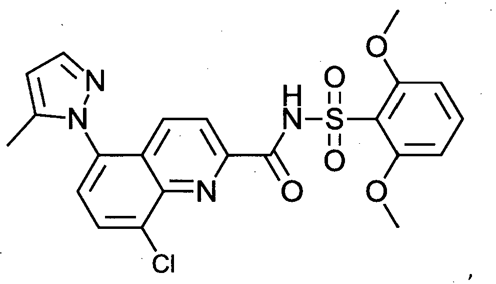 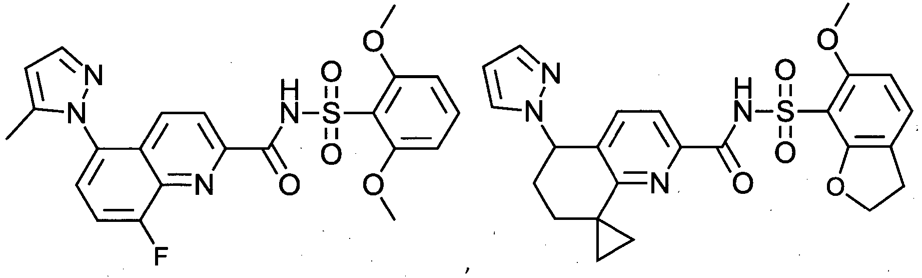 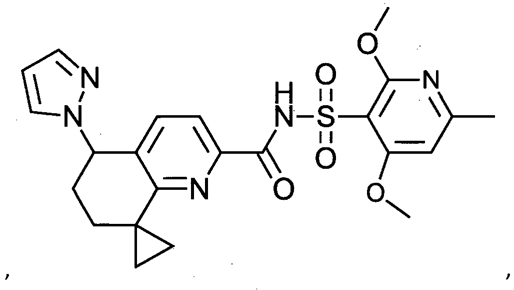 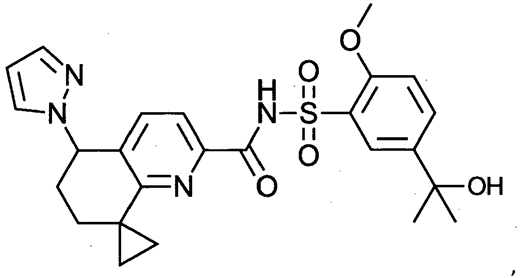 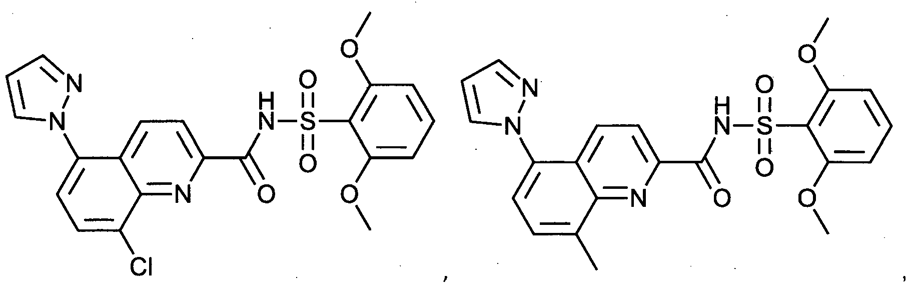 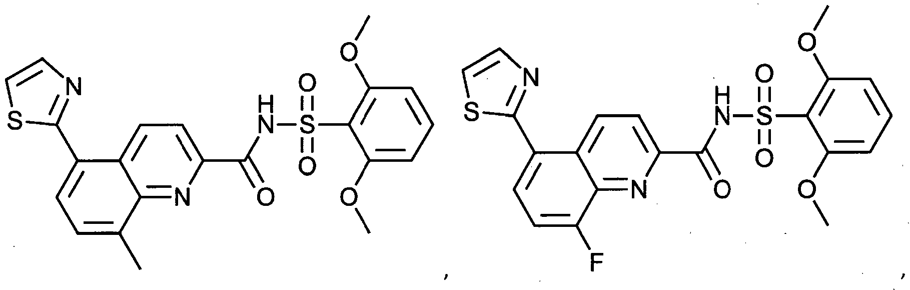 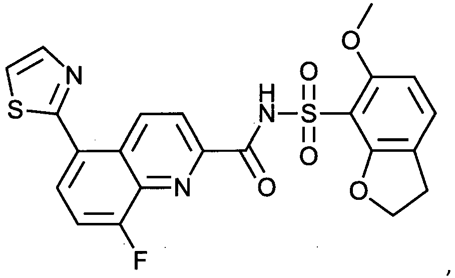 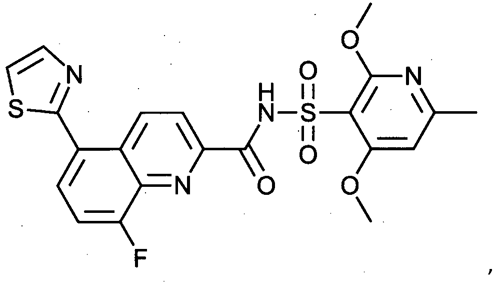 or

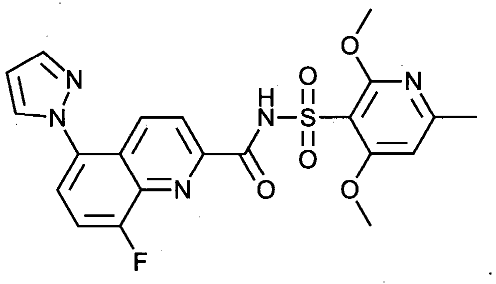

11. A compound of general formula (I) or a pharmaceutically acceptable salt thereof according to any of claims 1 to 10, wherein the compound has a structure selected from: 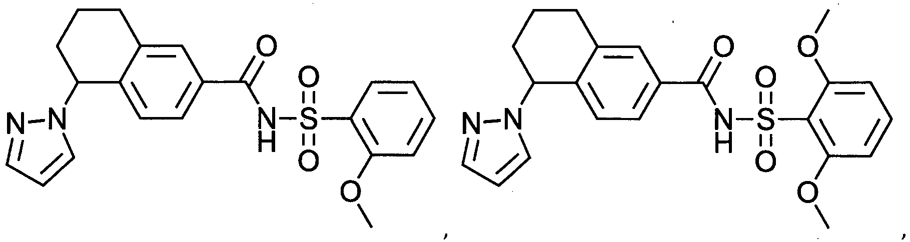 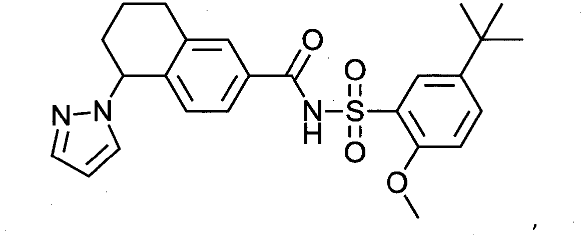 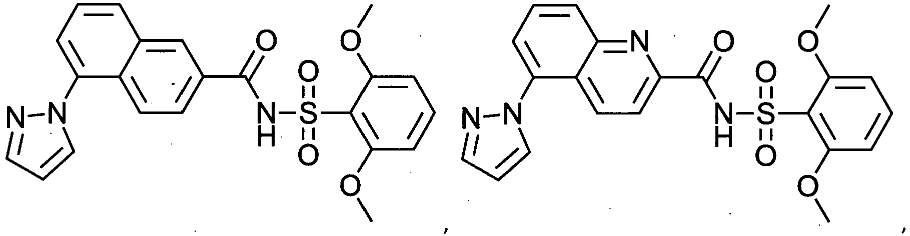 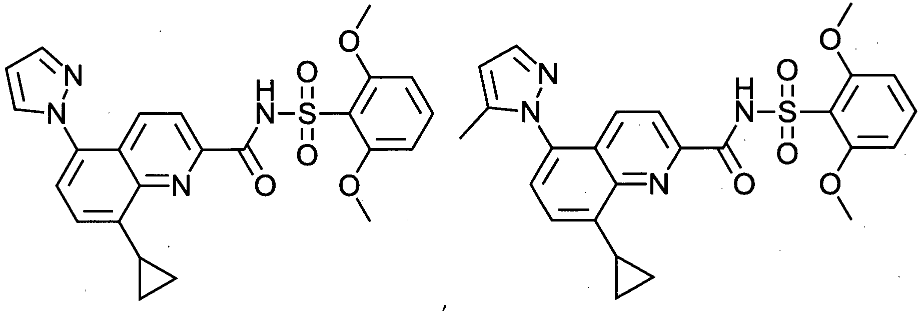 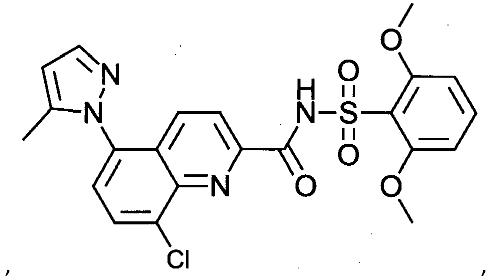 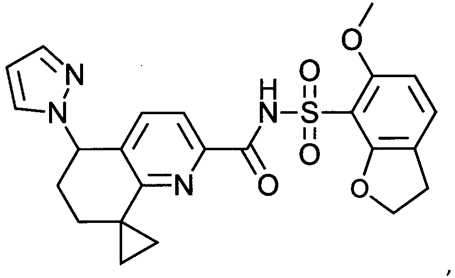 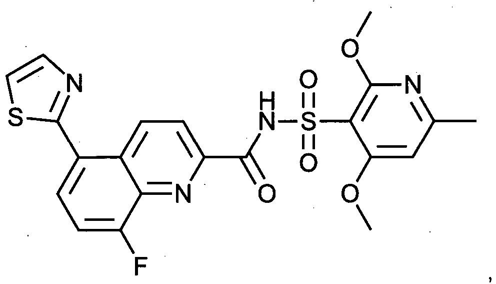 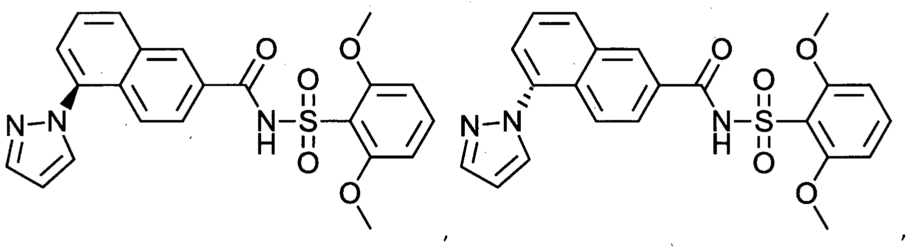 or

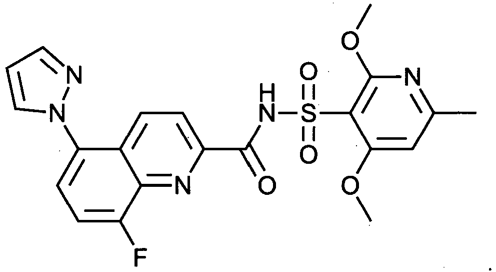

12. A compound according to any of claims 1 to 11, including all tautomeric forms, stereoisomeric forms, pharmaceutically acceptable salts thereof, racemic mixtures, substantially pure stereoisomeric forms, and optically pure stereoisomeric forms.

13. A pharmaceutical composition comprising a compound according to any one of claims 1 to 12, or a pharmaceutically acceptable salt thereof, and one or more pharmaceutically acceptable excipients.

14. The compound or a pharmaceutically acceptable salt thereof according to any one of claims 1 to 12, or the pharmaceutical composition according to claim 13, for use in the treatment or prophylaxis of a disease, preferably wherein the disease is a hyperproliferative disorder.

15. The compound, the pharmaceutically acceptable salt or the pharmaceutical composition for use according to claim 14, wherein it is for use in combination with a selective estrogen receptor degrader (SERD) such as fulvestrant or oral alternatives like camizestrant, immune checkpoint inhibitors targeting PD-1, PD-L1, or CTLA-4 and/or a cyclin-dependent kinase (CDK) inhibitor, including CDK2, CDK4, CDK6 or dual CDK2/4 or CDK4/6 inhibitors, such as palbociclib, ribociclib, or abemaciclib.
